# EUROPEAN PATENT APPLICATION

(11) **EP 4 032 976 A1**
(43) Date of publication of application: **27.07.2022**
(21) Application number: 19945890.2
(22) Date of filing: 17.09.2019
(51) Int. Cl.: C12N 9/02, C12N 15/53, C12N 15/63, A01H 1/00

(54) **MUTANT HYDROXYPHENYLPYRUVATE DIOXYGENASE POLYPEPTIDE, ENCODING GENE THEREOF AND USE THEREOF**

(71) Applicant: Beijing Dabeinong Biotechnology Co., Ltd., Beijing 100193 (CN)
(72) Inventor: XIAO, Xiang, Beijing 100193 (CN); TAO, Qing, Beijing 100193 (CN); YU, Caihong, Beijing 100193 (CN); SONG, Qingfang, Beijing 100193 (CN); BAO, Xiaoming, Beijing 100193 (CN)
(74) Representative: Nederlandsch Octrooibureau
(86) International application number: PCT/CN2019/106168
(87) International publication number: WO 2021/051265

(57) **Abstract**

Provided is a mutant hydroxyphenylpyruvate dioxygenase (HPPD) polypeptide, an encoding gene thereof and a use thereof. The mutant HPPD polypeptide retains the activity of catalyzing the conversion of hydroxyphenylpyruvate acid into homogentisic acid or homogentisate, and the sensitivity to HPPD inhibitor herbicides is lower than that of original unmutated HPPD. On position 372 corresponding to the amino acid sequence represented by SEQ ID NO: 1, the mutant HPPD polypeptide comprises the following mutations: F372A, F372G, F372V, F372P, F372S, F372T, F372C, F372M, F372Q, F372D or F372 deletion. The described mutant can provide plants with high tolerance to HPPD inhibitor herbicides, and can be used to cultivate plants that are tolerant to HPPD inhibitor herbicides.

## Description

### FIELD OF THE INVENTION

The present invention relates to a mutant hydroxyphenylpyruvate dioxygenase polypeptide, a coding gene and use thereof, and in particular, to a mutant hydroxyphenylpyruvate dioxygenase polypeptide which is tolerant to HPPD-inhibitor herbicides, a coding gene and use thereof.

### BACKGROUND

The hydroxyphenylpyruvate dioxygenases (abbreviated as HPPDs) are enzymes which, in the presence of iron ion (Fe²⁺) and oxygen, catalyze the reaction in which 4-hydroxyphenylpyruvic acid (abbreviated as HPP), a tyrosine degradation product, is transformed into homogentisic acid / homogentisate (abbreviated as HG), the precursor in plants of tocopherol and plastoquinone (abbreviated as PQ). Tocopherol acts as a membrane-associated antioxidant. PQ not only acts as an electron carrier between PSII and the cytochrome b6/f complex, but also an essential cofactor for the phytoene desaturase involved in the biosynthesis of carotenoids.

Herbicides that act by inhibiting HPPD mainly include three chemical families: triketones, isoxazoles, and pyrazolinates. Inhibition of HPPD blocks the biosynthesis of PQ from tyrosine in plants, thereby resulting in the depletion of PQ and deficiency in carotenoids. HPPD-inhibiting herbicides are plant phloem-mobile bleachers which cause the light-exposed new meristems and leaves to emerge white. Carotenoids are essential for photo-protection. In the absence of carotenoids, the synthesis and function of chlorophyll will be disrupted by UV-radiation and reactive oxygen intermediates, thereby leading to plant growth suppression or even death.

Methods for providing plants that are tolerant to HPPD-inhibitor herbicides have included: 1) overexpressing the HPPD enzyme so as to produce quantities of HPPD enzyme in the plant that are sufficient in relation to HPPD-inhibitor herbicides so as to have enough of the functional enzyme available despite the presence of its inhibitor; and 2) mutating the target HPPD enzyme into a functional HPPD that is less sensitive to herbicides or their active metabolites but retains the capability of transforming into HG. With respect to mutant HPPDs, while a given mutant HPPD enzyme may provide a useful level of tolerance to some HPPD-inhibitor herbicides, the same mutant HPPD may be quite inadequate to provide commercial levels of tolerance to a different, more desirable HPPD-inhibitor herbicide. For example, HPPD-inhibitor herbicides may differ in terms of the spectrum of weeds they control, their manufacturing costs, and their environmental benefits. Accordingly, new methods and/or compositions for conferring HPPD-inhibitor herbicide tolerance upon various crops and crop varieties are needed.

### SUMMARY OF THE INVENTION

The object of the present invention is to provide a new mutant hydroxyphenylpyruvate dioxygenase polypeptide, a coding gene and use thereof. The mutant hydroxyphenylpyruvate dioxygenase polypeptide not only has HPPD enzymatic activity, but also enables the plants transformed with genes encoding the mutant hydroxyphenylpyruvate dioxygenase polypeptide to have good tolerance to HPPD-inhibitor herbicides.

To achieve the above object, the present invention provides a mutant hydroxyphenylpyruvate dioxygenase polypeptide which retains the activity of catalyzing the reaction of transforming 4-hydroxyphenylpyruvic acid into homogentisic acid or homogentisate and is less sensitive to an HPPD-inhibitor herbicide than the native unmutated HPPD, comprising the following mutation at a position corresponding to position 372 of the amino acid sequence as set forth in SEQ ID NO:1: F372A, F372G, F372V, F372P, F372S, F372T, F372C, F372M, F372Q, F372D, or F372 deletion;

Preferably, the mutant hydroxyphenylpyruvate dioxygenase polypeptide comprises the following mutation at a position corresponding to position 372 of the amino acid sequence as set forth in SEQ ID NO:1: F372A, F372G or F372V.

More preferably, the mutant hydroxyphenylpyruvate dioxygenase polypeptide comprises the following mutation at a position corresponding to position 372 of the amino acid sequence as set forth in SEQ ID NO:1: F372A.

Further, the mutant hydroxyphenylpyruvate dioxygenase polypeptide is derived from HPPDs in plants or microorganisms.

Preferably, the plant comprises monocotyledonous plants and dicotyledonous plants; more preferably, the plant is oats (*Avena sativa*)*,* wheat (*Triticum aestivum*)*,* barley (*Hordeum vulgare*), millet (*Setaria italica*)*,* corn (*Zea mays*), sorghum (*Sorghum bicolor*), *Brachypodium distachyon,* rice (*Oryza sativa*), tobacco (*Nicotiana tabacum*), sunflower (*Helianthus annuus*), alfalfa (*Medicago sativa*), soybean (*Glycine max*), *cicer arietinum,* peanut (*Arachis hypogaea*)*,* sugar beet (*Beta vulgaris*), cucumber (*Cucumis sativus*), cotton (*Gossypium hirsutum*), oilseed rape (*Brassica napus*), potato (*Solanum tuberosum*), tomato (*Solanum lycopersicum*) or *Arabidopsis thaliana;*

Preferably, the microorganism is Cyanophyta, Pseudomonas fluorescens, or bacteria from the genus Sphingobium or Burkholderia.

Still further, when the native unmutated HPPD has an amino acid sequence as set forth in SEQ ID NO: 1, the polypeptide further comprises the following mutation at position 372 of the amino acid sequence as set forth in SEQ ID NO:1: F372L, F372I, F372W, F372N, F372E or F372K.

On the basis of the above technical solution, the mutant hydroxyphenylpyruvate dioxygenase polypeptide comprises a second mutation;

Preferably, the second mutation at a position corresponding to position 413 of the amino acid sequence as set forth in SEQ ID NO:1 comprises the following mutation: G413W, G413H, G413M, G413F or G413C; more preferably, the second mutation at a position corresponding to position 413 of the amino acid sequence as set forth in SEQ ID NO:1 is G413W mutation;

Optionally, when the native unmutated HPPD has an amino acid sequence as set forth in SEQ ID NO: 1, the second mutation at position 110 of the amino acid sequence as set forth in SEQ ID NO:1 is deletion mutation.

To achieve the above object, the present invention further provides a polynucleotide encoding the mutant hydroxyphenylpyruvate dioxygenase polypeptide.

To achieve the above object, the present invention further provides an expression cassette or a recombinant vector, comprising the polynucleotide under the regulation of effectively-linked regulatory sequences.

To achieve the above object, the present invention further provides a method for expanding the scope of herbicides to which the plants are tolerant, comprising expressing the mutant hydroxyphenylpyruvate dioxygenase polypeptide together with at least one herbicide-tolerant protein other than the mutant hydroxyphenylpyruvate dioxygenase polypeptide.

Further, the herbicide-tolerant protein is 5-enolpyruvylshikimate-3-phosphate synthase (EPSPS), glyphosate oxidoreductase, glyphosate-N-acetyltransferase, glyphosate decarboxylase, glufosinate acetyltransferase, alpha-ketoglutarate-dependent dioxygenase, dicamba monooxygenase, acetolactate synthase, cytochrome-like protein and/or protoporphyrinogen oxidase.

To achieve the above object, the present invention further provides a method for selecting transformed plant cells, comprising transforming a plurality of plant cells with the polynucleotide, and cultivating the cells under a concentration of the HPPD-inhibitor herbicide that allows the growth of the transformed cells expressing the polynucleotide, while killing the untransformed cells or inhibiting the growth of the untransformed cells.

Preferably, the plant comprises monocotyledonous plants and dicotyledonous plants; more preferably, the plant is oats, wheat, barley, millet, corn, sorghum, *Brachypodium distachyon,* rice, tobacco, sunflower, alfalfa, soybean, *Cicer arietinum,* peanuts, sugar beet, cucumber, cotton, oilseed rape, potato, tomato or *Arabidopsis thaliana;*

Preferably, the HPPD-inhibitor herbicide comprises an HPPD-inhibitor herbicide from the class of pyrazolinates, triketones and/or isoxazoles; more preferably, the HPPD-inhibitor herbicide of pyrazolinates is topramezone, the HPPD-inhibitor herbicide of triketones is mesotrione, and the HPPD-inhibitor herbicide of isoxazoles is isoxaflutole; particular preferably, the method for selecting transformed soybean plant cells comprises transforming a plurality of soybean plant cells with the polynucleotide, and cultivating the cells at a concentration of the HPPD-inhibitor herbicide that allows the growth of the transformed cells that express the polynucleotide, and kills the untransformed cells or inhibits the growth of untransformed cells, wherein the HPPD-inhibitor herbicide is topramezone, mesotrione, or isoxaflutole.

To achieve the above object, the present invention further provides a method for controlling weeds, comprising applying an effective dose of the HPPD-inhibitor herbicide to a field planting with a target plant, wherein the target plant contains the polynucleotide;

Preferably, the target plant comprises monocotyledonous plants and dicotyledonous plants; more preferably, the target plant is oats, wheat, barley, millet, corn, sorghum, *Brachypodium distachyon,* rice, tobacco, sunflower, alfalfa, soybean, *Cicer arietinum,* peanut, sugar beet, cucumber, cotton, oilseed rape, potato, tomato or *Arabidopsis thaliana;* further preferably, the target plant is glyphosate-tolerant plant, and the weeds are glyphosate-resistant weeds;

Preferably, the HPPD-inhibitor herbicide comprises an HPPD-inhibitor herbicide from the class of pyrazolinates, triketones and/or isoxazoles; more preferably, the HPPD-inhibitor herbicide of pyrazolinates is topramezone, the HPPD-inhibitor herbicide of triketones is mesotrione, and the HPPD-inhibitor herbicide of isoxazoles is isoxaflutole; particular preferably, the method for controlling weeds comprises applying an effective dose of the HPPD-inhibitor herbicide to a field in which a soybean plant is grown, wherein the soybean plant comprises the polynucleotide; wherein the HPPD-inhibitor herbicide is topramezone, mesotrione, or isoxaflutole.

To achieve the above object, the present invention further provides a method for protecting a plant from damages caused by an HPPD-inhibitor herbicide or for conferring tolerance to HPPD-inhibitor herbicide upon a plant, comprising introducing the polynucleotide or the expression cassette or the recombinant vector into the plant, resulting in an amount of the mutant hydroxyphenylpyruvate dioxygenase polypeptide that is sufficient to protect the plant into which the polynucleotide or the expression cassette or the recombinant vector has been introduced from damages caused by the HPPD-inhibitor herbicide.

Preferably, the plant comprises monocotyledonous plants and dicotyledonous plants; more preferably, the plant is oats, wheat, barley, millet, corn, sorghum, *Brachypodium distachyon,* rice, tobacco, sunflower, alfalfa, soybean, *Cicer arietinum,* peanut, sugar beet, cucumber, cotton, oilseed rape, potato, tomato or *Arabidopsis thaliana;*

Preferably, the HPPD-inhibitor herbicide comprises an HPPD-inhibitor herbicide from the class of pyrazolinates, triketones and/or isoxazoles; more preferably, the HPPD-inhibitor herbicide of pyrazolinates is topramezone, the HPPD-inhibitor herbicide of triketones is mesotrione, and the HPPD-inhibitor herbicide of isoxazoles is isoxaflutole; particular preferably, the method for protecting a soybean plant from damages caused by an HPPD-inhibitor herbicide or for conferring tolerance to the HPPD-inhibitor herbicide upon a soybean plant comprises introducing the polynucleotide or the expression cassette or the recombinant vector into the soybean plant, resulting in an amount of the mutant hydroxyphenylpyruvate dioxygenase polypeptides that is sufficient to protect the soybean plant into which the polynucleotide or the expression cassette or the recombinant vector has been introduced from the damage of the HPPD-inhibitor herbicide; wherein the HPPD-inhibitor herbicide is topramezone, mesotrione, or isoxaflutole.

To achieve the above object, the present invention further provides a method for generating a plant which is tolerant to an HPPD-inhibitor herbicide, comprising introducing the polynucleotide into the genome of the plant;

Preferably, the introducing method comprises genetic transformation, genome editing or gene mutation methods.

Preferably, the plant comprises monocotyledonous plants and dicotyledonous plants; more preferably, the plant is oats, wheat, barley, millet, corn, sorghum, *Brachypodium distachyon,* rice, tobacco, sunflower, alfalfa, soybean, *Cicer arietinum,* peanut, sugar beet, cucumber, cotton, oilseed rape, potato, tomato or *Arabidopsis thaliana;*

Preferably, the HPPD-inhibitor herbicide comprises an HPPD-inhibitor herbicide from the class of pyrazolinates, triketones and/or isoxazoles; more preferably, the HPPD-inhibitor herbicide of pyrazolinates is topramezone, the HPPD-inhibitor herbicide of triketones is mesotrione, and the HPPD-inhibitor herbicide of isoxazoles is isoxaflutole; particular preferably, the method for generating soybean plants that are tolerant to the HPPD-inhibitor herbicide comprises introducing the polynucleotide into the genome of the soybean plants; wherein the HPPD-inhibitor herbicide is topramezone, mesotrione, or isoxaflutole.

To achieve the above object, the present invention further provides a method for cultivating a plant which is tolerant to an HPPD-inhibitor herbicide, comprising:
planting at least one plant propagule, wherein the plant propagule comprises in its genome the polynucleotide;
allowing the plant propagule to grow into a plant; and
applying an effective dose of the HPPD-inhibitor herbicide to a plant growing environment comprising at least the plant, and harvesting the plant having a reduced plant damage and/or increased plant yield compared to other plants without the polynucleotide;

Preferably, the plant comprises monocotyledonous plants and dicotyledonous plants; more preferably, the plant is oats, wheat, barley, millet, corn, sorghum, *Brachypodium distachyon,* rice, tobacco, sunflower, alfalfa, soybean, *Cicer arietinum,* peanut, sugar beet, cucumber, cotton, oilseed rape, potato, tomato or *Arabidopsis thaliana;*

Preferably, the HPPD-inhibitor herbicide comprises an HPPD-inhibitor herbicide from the class of pyrazolinates, triketones and/or isoxazoles; more preferably, the HPPD-inhibitor herbicide of pyrazolinates is topramezone, the HPPD-inhibitor herbicide of triketones is mesotrione, and the HPPD-inhibitor herbicide of isoxazoles is isoxaflutole; particular preferably, the method for cultivating a soybean plant which is tolerant to the HPPD-inhibitor herbicide comprises planting at least one soybean plant seed, wherein the soybean plant seed comprises in its genome the polynucleotide; allowing the soybean plant seed to grow into a soybean plant; applying an effective dose of the HPPD-inhibitor herbicide to a plant growing environment comprising at least the soybean plant, and harvesting the soybean plant with reduced plant damage and/or increased plant yield compared to other soybean plants which do not comprise the polynucleotide; wherein the HPPD-inhibitor herbicide is topramezone, mesotrione, or isoxaflutole.

The present invention also provides a method for obtaining a processed agricultural product, comprising treating the harvested product obtained by the method from the plant which is tolerant to the HPPD-inhibitor herbicide to obtain the processed agricultural product.

To achieve the above object, the present invention further provides a planting system for controlling the growth of weeds, comprising an HPPD-inhibitor herbicide and a plant growing environment in which at least one target plant is present, wherein the target plant contains the polynucleotide;

Preferably, the target plant comprises monocotyledonous plants and dicotyledonous plants; more preferably, the target plant is oats, wheat, barley, millet, corn, sorghum, *Brachypodium distachyon,* rice, tobacco, sunflower, alfalfa, soybean, *Cicer arietinum,* peanut, sugar beet, cucumber, cotton, oilseed rape, potato, tomato or *Arabidopsis thaliana;* further preferably, the target plant is a glyphosate-tolerant plant, and the weeds are glyphosate-resistant weeds.

Preferably, the HPPD-inhibitor herbicide comprises an HPPD-inhibitor herbicide from the class of pyrazolinates, triketones and/or isoxazoles; more preferably, the HPPD-inhibitor herbicide of pyrazolinates is topramezone, the HPPD-inhibitor herbicide of triketones is mesotrione, and the HPPD-inhibitor herbicide of isoxazoles is isoxaflutole; particular preferably, the planting system for controlling the growth of weeds comprises an HPPD-inhibitor herbicide and a plant growing environment in which at least one soybean plant is present, wherein the soybean plant contains the polynucleotide, and the HPPD-inhibitor herbicide is topramezone, mesotrione, or isoxaflutole.

To achieve the above object, the present invention further provides use of the mutant hydroxyphenylpyruvate dioxygenase polypeptide for conferring tolerance to an HPPD-inhibitor herbicide upon a plant;

Preferably, the plant comprises monocotyledonous plants and dicotyledonous plants; more preferably, the plant is oats, wheat, barley, millet, corn, sorghum, *Brachypodium distachyon,* rice, tobacco, sunflower, alfalfa, soybean, *Cicer arietinum,* peanut, sugar beet, cucumber, cotton, oilseed rape, potato, tomato or *Arabidopsis thaliana;*

Preferably, the HPPD-inhibitor herbicide comprises an HPPD-inhibitor herbicide from the class of pyrazolinates, triketones and/or isoxazoles; more preferably, the HPPD-inhibitor herbicide of pyrazolinates is topramezone, the HPPD-inhibitor herbicide of triketones is mesotrione, and the HPPD-inhibitor herbicide of isoxazoles is isoxaflutole; particular preferably, use of the mutant hydroxyphenylpyruvate dioxygenase polypeptide for conferring tolerance to an HPPD-inhibitor herbicide upon a soybean plant, wherein the HPPD-inhibitor herbicide is topramezone, mesotrione, or isoxaflutole.

The article "a" and "an" as used herein refers to one or more than one (i.e., to at least one). For example, "an element" means one or more elements (components). Furthermore, the term "comprise" or variants thereof such as "comprises" or "comprising" should be understood to imply the inclusion of a stated element, integer or step, or group of elements, integers or steps, but not the exclusion of any other element, integer or step, or group of elements, integers or steps.

Within the context of the present invention, the terms "hydroxy phenyl pyruvate dioxygenase (HPPD)", "4-hydroxy phenyl pyruvate dioxygenase (4-HPPD)" and "p-hydroxy phenyl pyruvate dioxygenase (p-HPPD)" are synonymous.

The term "HPPD-inhibitor herbicide" refers to herbicides that act either directly or indirectly to inhibit HPPD, where the herbicides are bleachers. Most commercially available HPPD-inhibitor herbicides belong to one of the three chemical families as listed below: (1) Triketones, e.g. sulcotrione (i.e. 2-[2-chloro-4-(methylsulfonyl)benzoyl]-1,3-cyclohexane- -dione), mesotrione (i.e. 2-[4-(methylsulfonyl)-2-nitrobenzoyl]-1,3-cyclohexanedione); tembotrione (i.e. 2-[2-chloro-4-(methylsulfonyl)-3-[(2,2,2-trifluoroethoxy)methyl]benzoyl]- -1,3-cyclohexanedione); (2) Isoxazoles, e.g. isoxaflutole (i.e. (5-cyclopropyl-4-isoxazolyl)[2- -(methylsulfonyl)-4-(trifluoromethyl)phenyl]methanone); (3) Pyrazolinates, e.g. topramezone (i.e., [3-(4,5-dihydro-3-isoxazolyl)-2-methyl-4-(methylsulfonyl)phenyl] (5-hydroxy-1-methyl- -pyrazol-4-yl)methanone), pyrasulfotole (i.e., (5-hydroxy-1,3-dimethylpyrazol-4-yl)(2-methyl--sulfonyl-4-(trifluoromethylphenyl)methanone).

As used herein, topramezone (also known as BAS-670H) refers to [3-(4,5-dihydro-3-isoxazolyl) -2-methyl-4-(methylsulfonyl)phenyl] (5-hydroxy-1-methyl-pyrazol-4-yl)methanone as a white crystalline solid. It is a systemic conduction-type HPPD-inhibitor herbicide of pyrazolinates for the post-emergence treatment of stems and leaves in a typical dosage form of 30% suspension concentrate. Commercial formulations of topramezone (such as Topramezone SC) can be used for the control of gramineous and broad-leaf weeds, at 5.6-6.7 g per acre. The weeds that can be effectively controlled include but are not limited to, Digitaria sanguinalis (Calathodes oxycarpa), Barnyard grass, Eleusine indica Gaertn, Eriochloa villosa, Setaria viridis (Giant foxtail), Chenopodium album, Polygonaceae, Abutilon avicennae, Abutilon theophrasti, Pigweeds, Portulaca oleracea, Xanthium strumarium, and Solanum nigrum. Topramezone SC combined with Atrazine can result in a significantly enhanced effect. Apart from the excellent efficacy on the aforementioned weeds, topramezone can also have good controlling effects on malignant broad-leaf weeds, such as Cephalanoplos segetum Kitam (Cirsium segestum), Sonchus arvensis, Acalypha australis, and Commelina communis (Asiatic dayflower), and in particular it can effectively control Setaria viridis, Digitaria sanguinalis, Eleusine indica Gaertn, and Eriochloa villosa which are difficult to be controlled by mesotrione.

As used herein, the "effective dose" of topramezone means being used at an amount ranging from 25 to 100 g ai/ha, including from 30 to 95 g ai/ha, from 40 to 90 g ai/ha, from 50 to 85 g ai/ha or from 60 to 80 g ai/ha.

As used herein, the "mesotrione" refers to 2-[4-(methylsulfonyl)-2-nitrobenzoyl]-1,3- - cyclohexanedione as a brown or light yellow solid. It is a selective systemic conduction-type HPPD-inhibitor herbicide of triketones that provides both pre- and post-emergence weed control in plants. It can be absorbed by plants through the leaves and roots, and be translocated downward from top to bottom parts, resulting in chlorosis (yellowing) symptom of the meristems followed by necrosis (dead tissue) 3 to 5 days after herbicide application, and further death of the entire plants. Mesotrione is useful for pre- and post-emergence control of annual broad-leaf weeds and gramineous weeds in plants; wherein the annual broad-leaf weeds that can be controlled mainly comprise Xanthium strumarium, Abutilon theophrasti, Chenopodium album, amaranth, Polygonaceae, Solanum nigrum, and Ambrosia trifida; and the gramineous weeds that can be controlled mainly comprise young barnyard grass, Digitaria sanguinalis, Setaria viridis, and Brachiaria decumbens.

As used herein, the "effective dose" of mesotrione means being used at an amount ranging from 70 to 420 g ai/ha, including from 75 to 150 g ai/ha, from 105 to 210 g ai/ha, from 150 to 225 g ai/ha or from 210 to 315 g ai/ha.

As used herein, isoxaflutole refers to 5-cyclopropyl-4-isoxazolyl)[2-(methylsulfonyl)-4- - (trifluoromethyl)phenyl]methanone as a white to pale-yellow solid. It is a selective systemic pre-emergence HPPD-inhibitor herbicide of organic heterocyclic isoxazoles and predominantly works by absorption and translocation via the young weed roots. Isoxaflutole is mainly useful for controlling various annual broad-leaf weeds, such as Abutilon theophrasti, Chenopodium album, Kochia scoparia, Salsola arbuscula, Solanum nigrum, Amaranthus retroflexus, Polygonum bungeanum, Bidens pilosa, Portulaca oleracea, Chickweed, Elsholtzia, Xanthium strumarium, Acalypha australis, Amethystea caerulea, Polygonum Lapathifolium, and Veronica polita, in fields of dryland crops, and also has good controlling efficacy on some annually gramineous weeds, such as Digitaria sanguinalis, Barnyard grass, Eleusine indica Gaertn, Leptochloa chinensis, Setaria faberi, and Setaria viridis.

As used herein, the "effective dose" of isoxaflutole means being used at an amount ranging from 67 to 280 g ai/ha, including from 70 to 134 g ai/ha, from 70 to 140 g ai/ha, from 134 to 201 g ai/ha or from 140 to 210 g ai/ha.

As used herein, the term "resistance" is inheritable and allows a plant to grow and propagate under the circumstance where an effective treatment with an ordinary herbicide is performed on a given plant. As recognized by a person skilled in the art, even if there is certain degree of damage (such as small necrosis, dissolution, chlorosis or other damage) to the given plant treated with the herbicide, at least the yield is not significantly compromised and thus the plant can still be considered as "resistant". In other words, the given plant has increased ability to resist various degrees of damage induced by the herbicide, and in general, damage to a wild-type plant with the same genotype can be caused at the same dose of the herbicide. The term "tolerant" or "tolerance" in the present invention is more extensive than the term "resistance" and includes "resistance".

As used herein, the term "confer" refers to providing a characteristic or trait, such as herbicide tolerance and/or other desirable traits to a plant.

As used herein, the term "heterologous" means from another source. In the context of DNA, "heterologous" refers to any foreign "non-self' DNA including that from another plant of the same species. For example, in the present invention a soybean HPPD gene that can be expressed in a soybean plant by means of transgenic methods would still be considered as "heterologous" DNA.

As used herein, the term "nucleic acid" includes a deoxyribonucleotide or ribonucleotide polymer in either single- or double-stranded forms, and unless otherwise specified, encompasses known analogues (e.g., peptide nucleic acids) having the essential properties of natural nucleotides in that they hybridize to single-stranded nucleic acids in a manner similar to naturally occurring nucleotides.

As used herein, the term "encoding" or "encoded" when used in the context of a specified nucleic acid means that the nucleic acid comprises the requisite information to direct translation of the nucleotide sequence into a specified protein. The information by which a protein is encoded is specified by the use of codons. A nucleic acid encoding a protein may comprise non-translated sequences (e.g., introns) within translated regions of the nucleic acid or may lack such intervening non-translated sequences (e.g., as in cDNA).

The proteins of the invention may be altered in various ways including amino acid substitutions, deletions, truncations, and insertions. For example, amino acid sequence variants and fragments of the mutant HPPD proteins can be prepared by mutations in the DNA. Methods for the induction of polynucleotide mutations are well-known in the art. See, for example, Kunkel (1985) Proc. Natl. Acad. Sci. USA 82:488-492; Kunkel et al. (1987) Methods in Enzymol. 154:367-382; U.S. Pat. No. 4,873,192; Walker and Gaastra, eds. (1983) Techniques in Molecular Biology (MacMillan Publishing Company, New York) and the references cited therein. Guidance as to appropriate amino acid substitutions that often do not affect biological activity of the protein of interest may be found in the model of Dayhoff et al. (1978) Atlas of Protein Sequence and Structure (Natl. Biomed. Res. Found., Washington, D.C.). Conservative substitutions, such as exchanging one amino acid with another having similar properties, may be optimal.

As described herein, the mutant HPPD polypeptides or variants and fragments thereof possess HPPD enzymatic activity and confer tolerance to certain classes of HPPD-inhibitor herbicides upon plants. The mutant HPPD polypeptides have amino acid changes at one or more positions relative to the starting wild-type sequence from which they are derived, and exhibit enhanced tolerance to one or more HPPD-inhibitor herbicides. HPPD enzymes that exhibit enhanced tolerance to at least one HPPD-inhibitor herbicide may do so by virtue of exhibiting, relative to the like unmutated starting enzyme.

DNA sequences encoding such mutant HPPD polypeptides are used in the provision of plants, plant cells and seeds of the present invention that offer enhanced tolerance to one or more HPPD-inhibitor herbicides compared with like plants likewise expressing the unmutated starting enzyme.

Plant HPPD genes encoding such mutant HPPD polypeptides are useful for generating plants tolerant to HPPD-inhibitor herbicides. Plant HPPD genes so modified are particularly suitable for expression in plants to confer herbicide tolerance upon plants.

Many HPPD sequences are known in the art and can be used to generate mutant HPPD sequences by making substitutions, deletions, and/or additions in the corresponding amino acids. The position 372 of the present invention is calculated using the position of the amino acid at position 372 of the Avena native HPPD amino acid sequence set forth in SEQ ID NO:1 as the standard. The mutant HPPD polypeptide according to the present invention comprises a mutation occurring at an amino acid (phenylalanine) corresponding to position 372 of SEQ ID NO:1, with the mutated forms being F372A, F372G, F372V, F372P, F372S, F372T, F372C, F372M, F372Q, F372D, or F372 deletion; preferably F372A, F372G, and F372V; and more preferably, F372A. Thus, a known or suspected HPPD sequence can be aligned with the amino acid sequence as set forth in SEQ ID NO:1 using standard sequence alignment tools, and the substitutions or deletions in the corresponding amino acids with respect to the amino acid sequence as set forth in SEQ ID NO:1 as described herein can be made in the known or suspected HPPD sequence.

The present invention comprise a mutant HPPD polypeptide which is derived from HPPDs in plants or microorganisms, has HPPD enzymatic activity, and comprises at least one mutation at position 372 of the amino acid sequence as set forth in SEQ ID NO:1, optionally further in combination with a mutation at other known positions (the corresponding positions present in the HPPD polypeptide), for example, in combination with one or more mutations at the following corresponding positions: P215, G298, G332, F333, G334, G336, and N337 in the amino acid sequence of the HPPD from Pseudomonas fluorescens, and/or V217, A326, L358, and G408 in the amino acid sequence of the HPPD from Avena; preferably, the G336 comprises a mutated form of: G336W, G336H, G336M, G336F or G336C; the V217 mutation comprises a mutated form of V217I; the A326 mutation comprises a mutated form of A326R; the L358 mutation comprises a mutated form of L358M; and the G408 mutation comprises a mutated form of G408A. In the various embodiments described above, the mutated form at position 372 may be F372A, F372G, F372V, F372P, F372S, F372T, F372C, F372M, F372Q, F372D, or F372 deletion; preferably, the mutated form is F372A, F372G and F372V; and more preferably, the mutated form is F372A.

Further, the present invention comprise a mutant HPPD polypeptide which is derived from HPPDs (by substitutions, deletions and/or additions) in plants or microorganisms of different species or different ecotypes within the same species. In addition to the HPPDs from the ecotypes within the species as listed in the examples of the present invention, exemplary HPPDs from different ecotypes within the same species further include, but are not limited to, HPPDs from the ecotypes within the following species: HPPDs from the different ecotypes of cotton with the Accession Number: A0A0D2PWQ6, A0A2P5SI66, A0A0D2LWN1 or A0A0D2N7F6; HPPDs from *Brachypodium distachyon* with the Accession Number: I1IJR8; HPPDs from different ecotypes of barley with the Accession Number: BAJ86732.1, BAJ95714.1 or F2E412; HPPDs from different ecotypes of millets with the Accession Number: XP_012704274.1 or RCV05326.1; HPPDs from different ecotypes of rice with the Accession Number: A3A3J1, B8AIH6 or A0A0E0G1W2; HPPDs from different ecotypes of soybean with the Accession Number: A5Z1N7, I1M6Z4, A0A088MGH9 or I1M6Z5; HPPDs from different ecotypes of oilseed rape with the Accession Number: VDC64417.1, CDY10210.1, AFB74208.1, XP_013695640.1, XP_013695641.1, RID40406.1, RID48932.1, XP_009118533.1, XP_009119049.1, XP_013723237.1, AFB74218.1 or AFB74207.1; HPPDs from sunflower with the Accession Number: A0A251VJ25; HPPDs from different ecotypes of alfalfa with the Accession Number: XP_003617391.2, AAX59006.1, XP_003617384.1, XP_013466115.1, AET00342.2 or A0A396HWH5; HPPDs from sugar beet with the Accession Number: LOC104902719; HPPDs from different ecotypes of tobacco with the Accession Number: XP_009770088.1 or XP_009587203.1; HPPDs from potato with the Accession Number: LOC102595018; HPPDs from different ecotypes of tomato with the Accession Number: XP_004240171.1, NP_001310368.1, XP_015082678.1 or ADZ24700.1; and HPPDs from peanut with the Accession Number: XP_025608715.1 (these Accession Numbers are available at GenBank Database or UniProt Knowledgebase Database).

In one embodiment, the present invention comprise a mutant HPPD polypeptide which is derived from the amino acid sequence of the HPPD from Avena as set forth in SEQ ID NO:1 or has at least about 80%, 85%, 90%, 91%, 92%, 93%, 94%, 95%, 96%, 97%, 98%, 99% or more sequence identity to SEQ ID NO:1, possesses HPPD enzymatic activity, and comprises at least one mutation at position 372 of the amino acid sequence as set forth in SEQ ID NO:1, optionally further in combination with other known mutations. In different embodiments, the polypeptide may comprise at a position corresponding to position 372 of the amino acid sequence as set forth in SEQ ID NO:1 the following mutations: F372A, F372G, F372V, F372P, F372S, F372T, F372L, F372I, F372W, F372C, F372N, F372E, F372M, F372Q, F372D, F372K or F372 deletion; preferably, the mutated form is F372A, F372G and F372V; and more preferably, the mutated form is F372A.

In another embodiment, the present invention comprises a mutant HPPD polypeptide which is derived from the amino acid sequence of the HPPD from Pseudomonas fluorescens as set forth in SEQ ID NO:27 or has at least about 80%, 85%, 90%, 91%, 92%, 93%, 94%, 95%, 96%, 97%, 98%, 99% or more sequence identity to SEQ ID NO:27, possesses HPPD enzymatic activity, and comprises at least one mutation at a position corresponding to position 372 of the amino acid sequence as set forth in SEQ ID NO:1, optionally further in combination with other known mutations. In different embodiments, the polypeptide may comprise at a position corresponding to position 372 of the amino acid sequence as set forth in SEQ ID NO:1 the following mutations: F372A, F372G, F372V, F372P, F372S, F372T, F372C, F372M, F372Q, F372D, or F372 deletion; preferably, the mutated form is F372A, F372G and F372V; and more preferably, the mutated form is F372A.

The term "position 372" or "372 position" not only in a narrow sense refers to the amino acid (phenylalanine) at position 372 of the amino acid sequence as set forth in SEQ ID NO:1, but also in a broad sense comprises a position corresponding to the amino acid at position 372 of the amino acid sequence as set forth in SEQ ID NO:1 obtained in a known or suspected HPPD amino acid sequence which can be aligned with the amino acid sequence as set forth in SEQ ID NO:1 using standard sequence alignment tools (such as CLUSTAL software), which might not be the position 372 of the amino acid sequence of that HPPD.

The term "corresponding to" refers to a position corresponding to an amino acid at a particular position of the amino acid sequence as set forth in SEQ ID NO:1 obtained by aligning an HPPD amino acid sequence which is derived from a different species or different ecotype within the same species, with the amino acid sequence as set forth in SEQ ID NO:1 using standard sequence alignment tools, such as a position corresponding to the amino acid at position 372 of the amino acid sequence as set forth in SEQ ID NO:1.

The terms "polypeptide", "peptide", and "protein" are used interchangeably herein and refer to a polymer of amino acid residues. The terms apply to amino acid polymers in which one or more amino acid residues are an artificial chemical analogue of a corresponding naturally occurring amino acid, as well as to naturally occurring amino acid polymers. Polypeptides of the invention can be produced either from a nucleic acid disclosed herein, or by means of standard molecular biology techniques. For example, a truncated protein of the invention can be produced by expression of a recombinant nucleic acid of the invention in an appropriate host cell, or alternatively by a combination of *ex vivo* procedures, such as protease digestion and purification.

Accordingly, the present invention also provides nucleic acid molecules comprising polynucleotide sequences that encode mutant HPPD polypeptides that have enzymatic activity of HPPD and that confer tolerance in plants to certain classes of herbicides that inhibit HPPD, and variants and fragments thereof. In general, the invention includes any polynucleotide sequence that encodes any of the mutant HPPD polypeptides described herein, as well as any polynucleotide sequence that encodes HPPD polypeptides having one or more conservative amino acid substitutions relative to the mutant HHPD polypeptides described herein. Conservative substitutions providing functionally similar amino acids are well-known in the art. The following five groups each contain amino acids that are conservative substitutions for one another: Aliphatic: Glycine (G), Alanine (A), Valine (V), Leucine (L), Isoleucine (I); Aromatic: Phenylalanine (F), Tyrosine (Y), Tryptophan (W); Sulfur-containing: Methionine (M), Cysteine (C); Basic: Arginine (I), Lysine (K), Histidine (H); Acidic: Aspartic acid (D), Glutamic acid (E), Asparagine (N), Glutamine (Q).

In one embodiment, the present invention provides a polynucleotide sequence encoding an amino acid sequence derived from HPPDs in plants or microorganisms, where the polypeptide has HPPD enzymatic activity and comprises at least one mutation at a position corresponding to the amino acid at position 372 of SEQ ID NO:1.

Accordingly, sequences which have tolerance activity to HPPD-inhibitor herbicides and hybridize to genes encoding the mutant HPPD polypeptides of the invention are included within the present invention. Exemplary sequences comprise at least about 75%, 80%, 85%, 90%, 91%, 92%, 93%, 94%, 95%, 96%, 97%, 98%, 99% or more sequence identity to SEQ ID NO:5, SEQ ID NO:21, SEQ ID NO:26, SEQ ID NO:31, SEQ ID NO:37, SEQ ID NO:43, SEQ ID NO:49, SEQ ID NO:55, SEQ ID NO:61, SEQ ID NO:67, SEQ ID NO:73, SEQ ID NO:79, SEQ ID NO:85, SEQ ID NO:91, SEQ ID NO:97, SEQ ID NO:103, SEQ ID NO:109, SEQ ID NO:115, SEQ ID NO:121, SEQ ID NO:127, SEQ ID NO:133, SEQ ID NO:139, SEQ ID NO:145, SEQ ID NO:151, SEQ ID NO:157, SEQ ID NO:163, SEQ ID NO:169, and SEQ ID NO:175 of the invention.

Any conventional nucleic acid hybridization or amplification method can be used to identify the presence of the mutant HPPD gene of the present invention. A nucleic acid molecule or a fragment thereof is capable of specifically hybridizing to other nucleic acid molecules under certain circumstances. In the present invention, if two nucleic acid molecules can form an antiparallel double-stranded nucleic acid structure, then it can be considered that these two nucleic acid molecules can be specifically hybridized to each other. If two nucleic acid molecules exhibit a complete complementarity, then one nucleic acid molecule of the two is said to be the "complement" of the other nucleic acid molecule. In the present invention, when each nucleotide of a nucleic acid molecule is complementary to the corresponding nucleotide of another nucleic acid molecule, then these two nucleic acid molecules are said to exhibit a "complete complementarity". If two nucleic acid molecules can be hybridized to each other with a sufficient stability to allow them to anneal and bind with each other at least under conventional "low stringency" conditions, then these two nucleic acid molecules are said to be "minimally complementary". Similarly, if two nucleic acid molecules can be hybridized to each other with a sufficient stability to allow them to anneal and bind with each other under conventional "high stringency" conditions, then these two nucleic acid molecules are said to be "complementary". Deviation from a complete complementarity is permissible, as long as this deviation does not completely prevent two molecules from forming a double-stranded structure. In order to enable a nucleic acid molecule to act as a primer or probe, it is only guaranteed that the molecule has a sufficient complementarity in its sequence to allow a stable double-stranded structure to be formed under the conditions of particular solvent and salt concentration.

In the present invention, a substantially homologous sequence is a nucleic acid molecule that can be specifically hybridized to the complementary strand of a matched nucleic acid molecule under high stringency conditions. Suitable stringent conditions that promote DNA hybridization are well-known to a person skilled in the art; for example, the suitable stringent conditions can be achieved by treating with 6.0× sodium chloride/sodium citrate (SSC) under conditions of approximately 45 °C, and then washing with 2.0×SSC under conditions of 50 °C. For example, the salt concentration in the washing step can be selected from the low stringency condition of about 2.0×SSC and 50 °C to the high stringency condition of about 0.2×SSC and 50 °C. In addition, the temperature condition in the washing step can rise from the low stringency condition of room temperature (about 22 °C) to the high stringency condition of about 65 °C. The temperature condition and the salt concentration can both vary, and it is also possible that one of the two remains unchanged, while the other varies. Preferably, the stringent conditions in the present invention can be achieved by specifically hybridizing to the mutant HPPD gene of the present invention in a 6×SSC, 0.5% SDS solution at 65 °C, and then washing the membrane each once with 2×SSC, 0.1% SDS and 1×SSC, 0.1% SDS.

As used herein, the term "hybridizing" or "hybridizing specifically" refers to the binding, duplexing, or hybridizing of a molecule only to a particular nucleotide sequence under stringent conditions when that sequence is present in a complex mixture (e.g., total cellular) DNA or RNA.

Because of the degeneracy of the genetic codon, a variety of different DNA sequences may encode the same amino acid sequence. It is within the skill of a person skilled in the art to produce these alternative DNA sequences encoding the same or substantially the same protein. These different DNA sequences are included in the scope of the present invention. The "substantially the same" sequence refers to a sequence with an amino acid substitution, deletion, addition or insertion that does not substantively affect the herbicide tolerance activity, and includes a fragment retaining the herbicide tolerance activity.

The term "functional activity" or "activity" in the present invention means that the protein/enzyme used in the present invention (alone or in combination with other proteins) has the ability to degrade an herbicide or diminish the herbicide activity. A plant producing the protein of the present invention preferably produces an "effective amount" of the protein, so that when treating the plant with an herbicide, the protein expression level is sufficient to confer the plant a complete or partial tolerance to the herbicide (unless otherwise specified, in a general amount). The herbicide can be used in an amount which would usually kill a target plant or in a normal field amount and concentration. Preferably, the plant cell and plant of the present invention are protected from growth suppression or damage caused by treatment with the herbicide. The transformed plant and plant cell of the present invention are preferably tolerant to HPPD-inhibitor herbicides, that is, the transformed plant and plant cell can grow in the presence of an effective dose of HPPD-inhibitor herbicides.

The gene and protein in the present invention not only comprise a specific exemplary sequence, but also comprise a portion and/or a fragment (including an internal deletion and/or terminal deletion compared to the full-length protein), a variant, a mutant, a variant protein, a substitute (a protein having substituted amino acids), a chimera and a fusion protein which retain the HPPD-inhibitor herbicide tolerance activity characteristic of the specific exemplary protein.

The term "variant" in the present invention is intended to mean substantially similar sequences. For polynucleotides, a variant comprises a deletion and/or addition of one or more nucleotides at one or more internal sites within the reference polynucleotide and/or a substitution of one or more nucleotides at one or more sites in the mutant HPPD polynucleotide. As used herein, the term "reference polynucleotide or polypeptide" comprises a mutant HPPD nucleotide sequence or amino acid sequence, respectively. As used herein, the term "native polynucleotide or polypeptide" comprises a naturally occurring nucleotide sequence or amino acid sequence, respectively. For polynucleotides, conservative variants include those sequences that, because of the degeneracy of the genetic code, encode the amino acid sequence of one of the mutant HPPD polypeptides of the invention. Naturally occurring allelic variants such as these can be identified with the use of well-known molecular biology techniques, for example, with polymerase chain reaction (PCR) and hybridization techniques as outlined below. Variant polynucleotides also include synthetically derived polynucleotide, such as those generated, for example, by using site-directed mutagenesis but which still encode a mutant HPPD protein of the invention. Generally, variants of a particular polynucleotide of the invention will have at least about 75%, 80%, 85%, 90%, 91%, 92%, 93%, 94%, 95%, 96%, 97%, 98%, 99% or more sequence identity to that particular polynucleotide as determined by sequence alignment programs and parameters.

"Variant protein" in the present invention is intended to mean a protein derived from the reference protein by deletion or addition of one or more amino acids at one or more internal sites in the mutant HPPD protein and/or substitution of one or more amino acids at one or more sites in the mutant HPPD protein. Variant proteins encompassed by the present invention are biologically active, that is they continue to possess the desired biological activity of the mutant HPPD protein, that is, HPPD enzymatic activity and/or herbicide tolerance as described herein. Such variants may result from, for example, genetic polymorphism or from human manipulation. Biologically active variants of a mutant HPPD protein of the invention will have at least about 85%, 90%, 91%, 92%, 93%, 94%, 95%, 96%, 97%, 98%, 99% or more sequence identity across the entirety of the amino acid sequence for the mutant HPPD protein as determined by sequence alignment programs and parameters. A biologically active variant of a protein of the invention may differ from that protein by as few as 1-15 amino acid residues, as few as 1-10, such as 6-10, as few as 5, as few as 4, 3, 2, or even 1, amino acid residue.

Methods of alignment of sequences are well-known in the art and can be accomplished using mathematical algorithms such as the algorithm of Myers and Miller (1988) CABIOS 4:11-17; the local alignment algorithm of Smith et al. (1981) Adv. Appl. Math. 2:482; the global alignment algorithm of Needleman and Wunsch (1970) J. Mol. Biol. 48:443-453; and the algorithm of Karlin and Altschul (1990) Proc. Natl. Acad. Sci. USA 872264, modified as in Karlin and Altschul (1993) Proc. Natl. Acad. Sci. USA 90:5873-5877. Computer implementations of these mathematical algorithms can be utilized for comparison of sequences to determine sequence identity. Such implementations include, but are not limited to: CLUSTAL in the PC/Gene program (available from Intelligenetics, Mountain View, California); the ALIGN program (Version 2.0) and GAP, BESTFIT, BLAST, FASTA, and TFASTA in the GCG Wisconsin Genetics Software Package, Version 10 (available from Accelrys Inc., 9685 Scranton Road, San Diego, California, USA).

In certain examples, the amino acids encoding mutant HPPD polypeptides or variants thereof that retain HPPD enzymatic activity can be stacked with any combination of polynucleotide sequences of interest in order to create plants with a desired trait. The term "trait" refers to the phenotype derived from a particular sequence or groups of sequences. For example, the amino acids/polynucleotides encoding a mutant HPPD polypeptide or variant thereof that retains HPPD enzymatic activity may be stacked with any other polynucleotides encoding polypeptides that confer a desirable trait, including but not limited to resistance to diseases, insects, and herbicides, tolerance to heat and drought, reduced time to crop maturity, improved industrial processing, such as for the conversion of starch or biomass to fermentable sugars, and improved agronomic quality, such as high oil content and high protein content.

It is well-known for a person skilled in the art that the benefits of a combination of two or more modes of action in improving the spectrum of weeds controlled and/or the control of naturally more tolerant species or resistant weed species, can also be extended to chemicals for which herbicide tolerance was enabled in crops through artificial methods (either transgenically or non-transgenically) beyond HPPD tolerant crops. In fact, the traits encoding the following resistances can be superposed alone or in multiple combinations to provide the ability to effectively control or prevent weed shifts to herbicides: glyphosate resistance (such as EPSPS, GOX, and GAT from resistant plants or bacteria), glufosinate resistance (such as PAT and Bar), herbicide resistance to acetolactate synthase (ALS) inhibitors (such as imidazolinones, sulfonyl urea, triazole pyrimidines, sulfonated aniline, pyrimidinyl thiobenzoic acids and other chemicals resistant genes, e.g., AHAS, Csrl, and SurA), phenoxyauxin herbicide resistance (such as aryloxyalkanoate dioxygenase-12 (AAD-12)), dicamba herbicide resistance (such as dicamba monooxygenase (DMO)), bromoxynil resistance (such as Bxn), phytoene desaturase (PDS) inhibitor resistance, herbicide resistance to photosystem II inhibitors (such as psbA), herbicide resistance to photosystem I inhibitors, herbicide resistance to protoporphyrinogen oxidase IX (PPO) inhibitors (such as PPO-1), phenylurea herbicide resistance (such as CYP76B1), and dichloromethoxybenzoic acid degrading enzymes.

Glyphosate is widely used, as it controls a very broad spectrum of broad-leaf and gramineous weed species. However, repeat use of glyphosate in glyphosate-tolerant crop and non-crop applications has (and will continue to) selected for weed shifts to naturally more tolerant species or glyphosate resistant biotypes. Most herbicide resistance management programs suggest using an effective dose of tank-mixed herbicide partners as a means for delaying the emergence of resistant weeds, wherein the herbicide partners provide control for the same species, but have different modes of action. Superposing the gene encoding the mutant HPPD polypeptide of the present invention with a glyphosate tolerance trait (and/or other herbicide tolerance traits) can achieve the control of glyphosate resistant weed species (broad-leaf weed species controlled by one or more HPPD-inhibitor herbicides) in glyphosate tolerant crops by enabling the selective use of glyphosate and HPPD-inhibitor herbicides (such as topramezone, mesotrione, or isoxaflutole) in the same crop. The applications of these herbicides can be performed simultaneously in a tank mixture containing two or more herbicides with different modes of action, or can be performed alone in a single herbicide composition in sequential applications (e.g., before planting, or before or after emergence) (with the interval time of applications ranging from 2 hours to 3 months); or alternatively, the applications of these herbicides can be performed by using a combination of any number of herbicides representative of each applicable compound category at any time (from 7 months after planting a crop to the time when the crop is harvested (or the pre-harvest interval for a single herbicide, wherein the shortest is taken)).

The flexibility in controlling broad-leaf weeds is very important, in terms of the application time, application amount of single herbicide, and abilities to control the stubborn or resistant weeds. The application range of glyphosate superposed with a glyphosate resistant gene/mutant HPPD gene in crops can be from 250 to 2500 g ae/ha. The application range of HPPD-inhibitor herbicides (one or more) can be from 25 to 500 g ai/ha. The optimal combination of time for these applications depends on the specific conditions, species and environments.

An herbicide formulation (e.g., an ester, acid or salt-formulation, or soluble concentrate, emulsifiable concentrate or soluble liquid) and a tank mix additive (e.g., an adjuvant or compatilizer) can significantly affect the weed control of a given herbicide or a combination of one or more herbicides. Any chemical combination of any of the foregoing herbicides is within the scope of the present invention.

In addition, the gene encoding the mutant HPPD polypeptide of the present invention alone or being stacked with other characteristics of herbicide resistant crops can be stacked with one or more other input traits (for example, insect resistance, fungal resistance or stress tolerance, etc.) or output traits (for example, increased yield, improved oil amount, increased fiber quality, etc.). Therefore, the present invention can be used to provide complete agricultural solutions for improving the qualities of crops with the abilities for flexibly and economically controlling any number of agriculture pests.

These stacked combinations can be created by any method including, but not limited to, cross-breeding plants by any conventional or TopCross methodology, or genetic transformation. If the sequences are stacked by genetically transforming the plants, the polynucleotide sequences of interest can be combined at any time and in any order. For example, a transgenic plant comprising one or more desired traits can be used as the target to introduce further traits by subsequent transformation. The traits can be introduced simultaneously in a co-transformation protocol with the polynucleotides of interest provided by any combination of transformation cassettes. For example, if two sequences will be introduced, the two sequences can be contained in separate transformation cassettes (trans) or contained on the same transformation cassette (cis). Expression of the sequences can be driven by the same promoter or by different promoters. In certain cases, it may be desirable to introduce a transformation cassette that will suppress the expression of the polynucleotide of interest. This may be combined with any combination of other suppression cassettes or overexpression cassettes to generate the desired combination of traits in the plant. It is further recognized that polynucleotide sequences can be stacked at a desired genomic location using a site-specific recombination system.

The gene encoding the mutant HPPD polypeptide according to the present invention has higher tolerance to HPPD-inhibitor herbicides, which is an important basis for herbicide tolerant crops and selectable marker trait opportunities.

The term "expression cassette" as used herein means a nucleic acid molecule capable of directing expression of a particular nucleotide sequence in an appropriate host cell, comprising a promoter effectively linked to the nucleotide sequence of interest (i.e., a polynucleotide encoding a mutant HPPD polypeptide or variant thereof that retains HPPD enzymatic activity, alone or in combination with one or more additional nucleic acid molecules encoding polypeptides that confer desirable traits) which is effectively linked to termination signals. The coding region usually codes for a protein of interest but may also code for a functional RNA of interest, for example antisense RNA or a non-translated RNA, in the sense or antisense direction. The expression cassette comprising the nucleotide sequence of interest may be chimeric, meaning that at least one of its components is heterologous with respect to at least one of its other components. The expression cassette may also be one that is naturally occurring but has been obtained in a recombinant form useful for heterologous expression. Typically, however, the expression cassette is heterologous with respect to the host, i.e., the particular DNA sequence of the expression cassette does not occur naturally in the host cell and must have been introduced into new host cell by a transformation event. The expression of the nucleotide sequence in the expression cassette may be under the control of a constitutive promoter or of an inducible promoter that initiates transcription only when the host cell is exposed to some particular external stimulus. Additionally, the promoter can also be specific to a particular tissue or organ or stage of development.

The present invention encompasses the transformation of plants with expression cassettes capable of expressing a polynucleotide of interest (i.e., a polynucleotide encoding a mutant HPPD polypeptide or variant thereof that retains HPPD enzymatic activity, alone or in combination with one or more additional nucleic acid molecules encoding polypeptides that confer desirable traits). The expression cassette will include in the 5'-3' direction of transcription, a transcriptional and translational initiation region (i.e., a promoter) and a polynucleotide open reading frame. The expression cassette may optionally comprise a transcriptional and translational termination region (i.e., termination region) functional in plants. In some embodiments, the expression cassette comprises a selectable marker gene to allow for selection for stable transformants. Expression constructs of the invention may also comprise a leader sequence and/or a sequence allowing for inducible expression of the polynucleotide of interest.

The regulatory sequences of the expression construct are effectively linked to the polynucleotide of interest. The regulatory sequence in the present invention includes, but is not limited to, a promoter, a transit peptide, a terminator, an enhancer, a leader sequence, an intron and other regulatory sequences operably linked to the herbicide-tolerant gene encoding the mutant HPPD polypeptide.

The promoter is a plant expressible promoter. The "plant expressible promoter" refers to a promoter that ensures the expression of the coding sequence linked thereto in a plant cell. The plant expressible promoter can be a constitutive promoter. Examples of the promoters directing the constitutive expression in plants include, but are not limited to, a 35S promoter derived from a cauliflower mosaic virus, maize Ubi promoters, rice GOS2 gene promoters, and the like. Alternatively, the plant expressible promoter can be a tissue specific promoter, i.e. the promoter directs the expression of an coding sequence in several tissues, such as green tissues, at a level higher than in other tissues of the plant (which can be measured through conventional RNA trials), such as a PEP carboxylase promoter. Alternatively, the plant expressible promoter can be a wound-inducible promoter. The wound-inducible promoter or a promoter directing a wound-induced expression pattern means that when a plant suffers from a wound caused by a mechanical factor or the gnawing of insects, the expression of the coding sequence under the regulation of the promoter is significantly improved compared to normal growth conditions. Examples of the wound-inducible promoters include, but are not limited to, promoters of potato and tomato protease inhibitor genes (pin I and pin II) and a maize protease inhibitor gene (MPI).

The transit peptide (also known as secretion signal sequence or targeting sequence) directs a transgenic product to a specific organelle or cell compartment. For a receptor protein, the transit peptide may be heterologous, for example, targeting the chloroplast using a sequence encoding the chloroplast transit peptide, or targeting the endoplasmic reticulum using a 'KDEL' retention sequence, or targeting the vacuole using CTPP of a barley phytolectin gene.

The leader sequence includes, but is not limited to, a small RNA virus leader sequence, such as an EMCV leader sequence (a 5' non-coding region of encephlomyocarditis virus); a potato virus Y group leader sequence, such as a MDMV (Maize Dwarf Mosaic Virus) leader sequence; human immunoglobulin heavy chain binding protein (BiP); an untranslated leader sequence of the coat protein mRNA of alfalfa mosaic virus (AMV RNA4); and a tobacco mosaic virus (TMV) leader sequence.

The enhancer includes, but is not limited to, a cauliflower mosaic virus (CaMV) enhancer, figwort mosaic virus (FMV) enhancer, carnation etched ring virus (CERV) enhancer, cassava vein mosaic virus (CsVMV) enhancer, mirabilis mosaic virus (MMV) enhancer, cestrum yellow leaf curling virus (CmYLCV) enhancer, cotton leaf curl Multan virus (CLCuMV) enhancer, commelina yellow mottle virus (CoYMV) enhancer and peanut chlorotic streak caulimovirus (PCLSV) enhancer.

For use in a monocotyledonous plant, the intron includes, but is not limited to, a maize hsp70 intron, maize ubiquitin intron, Adh intron 1, sucrose synthase intron or rice Act1 intron. For use in a dicotyledonous plant, the intron includes, but is not limited to, a CAT-1 intron, pKANNIBAL intron, PIV2 intron and "super ubiquitin" intron.

The terminator can be a suitable polyadenylation signal sequence that functions in a plant, including, but not limited to, a polyadenylation signal sequence derived from the Agrobacterium tumefaciens nopaline synthetase (NOS) gene, a polyadenylation signal sequence derived from the protease inhibitor II (pinII) gene, a polyadenylation signal sequence derived from the pea ssRUBISCO E9 gene and a polyadenylation signal sequence derived from the α-tubulin gene.

The "effectively linking" in the present invention indicates the binding of nucleic acid sequences that enables one of the sequences to provide a function required for the sequence linked thereto. The "effectively linking" in the present invention can link a promoter to a sequence of interest, so that the transcription of the sequence of interest is controlled and regulated by the promoter. When a sequence of interest encodes a protein and the expression of the protein is desired, "effectively linking" means that: a promoter is linked to the sequence in such a manner that the resulting transcript is efficiently translated. If the linking of a promoter to a coding sequence is a transcript fusion and expression of the encoded protein is to be achieved, such linking is created that the first translation initiation codon in the resulting transcript is the initiation codon in the coding sequence. Alternatively, if the linking of a promoter to a coding sequence is a translation fusion and expression of the encoded protein is to be achieved, such a linking is created that the first translation initiation codon contained in the 5' untranslated sequence is linked to the promoter in such a manner that the relationship of the resulting translation product with the translation open reading frame encoding the desired protein is an in-frame. Nucleic acid sequences that can be "effectively linked" include, but are not limited to: sequences providing gene expression functions (i.e., gene expression elements, such as promoters, 5' untranslated regions, introns, protein coding regions, 3' untranslated regions, polyadenylation sites and/or transcription terminators), sequences providing DNA transfer and/or integration functions (i.e., T-DNA boundary sequences, site-specific recombinase recognition sites and integrase recognition sites), sequences providing selective functions (i.e., antibiotic resistance markers and biosynthesis genes), sequences providing marker scoring functions, sequences assisting in sequence manipulation in vitro or in vivo (i.e., polylinker sequences and site-specific recombination sequences), and sequences providing replication functions (i.e., the bacterial origins of replication, autonomously replicating sequences and centromeric sequences).

The genome of a plant, plant tissue or plant cell in the present invention refers to any genetic material within the plant, plant tissue or plant cell, and includes nuclear, plastid and mitochondrial genomes.

As used herein, the term "plant part" or "plant tissue" includes plant cells, plant protoplasts, plant cell tissue cultures from which plants can be regenerated, plant calli, plant clumps, and plant cells that are intact in plants or parts of plants such as embryos, pollen, ovules, seeds, leaves, flowers, branches, fruit, kernels, ears, cobs, husks, stalks, roots, root tips, anthers, and the like.

The mutant HPPD polypeptide of the present invention can be applied to various types of plants. The dicotyledonous plant includes, but is not limited to, alfalfa, beans, cauliflowers, cabbages, carrots, celery, cotton, cucumbers, eggplants, lettuces, melon, peas, peppers, zucchinis, radishes, oilseed rape, spinach, soybeans, pumpkins, tomatoes, *Arabidopsis thaliana,* peanuts or watermelons; preferably, the dicotyledonous plant refers to cucumbers, soybeans, *Arabidopsis thaliana,* tobacco, cotton or oilseed rape. The monocotyledonous plant includes, but is not limited to, maize, rice, sorghum, wheat, barley, rye, millet, sugar cane, oats or turfgrass. Preferably, the monocotyledonous plant refers to maize, rice, sorghum, wheat, barley, millet, sugar cane or oats.

As used herein, the term "plant transformation" means that once an herbicide resistant or tolerant mutant HPPD polynucleotide, alone or in combination with one or more additional nucleic acid molecules encoding polypeptides that confer desirable traits, has been cloned into an expression system, it is transformed into a plant cell. The receptors and target expression cassettes of the present invention can be introduced into the plant cell in a number of art-recognized ways. The term "introducing" in the context of a polynucleotide, for example, a nucleotide construct of interest, is intended to mean presenting to the plant the polynucleotide in such a manner that the polynucleotide gains access to the interior of a cell of the plant. Where more than one polynucleotide is to be introduced, these polynucleotides can be assembled as part of a single nucleotide construct, or as separate nucleotide constructs, and can be located on the same or different transformation vectors. Accordingly, these polynucleotides can be introduced into the host cell of interest in a single transformation event, in separate transformation events, or, for example, in plants, as part of a breeding protocol. The methods of the invention do not depend on a particular method for introducing one or more polynucleotides into a plant, only that the polynucleotide(s) gains access to the interior of at least one cell of the plant. Methods for introducing one or more polynucleotides into plants are known in the art including, but not limited to, transient transformation methods, stable transformation methods, and virus-mediated methods or genome-editing techniques.

The term "stable transformation" means that an exogenous gene is introduced into the genome of the plant and stably integrates into the plant or its genome of any successive generations, so that the exogenous gene is stably inherited.

The term "transient transformation" means that a nucleic acid molecule or protein is introduced into the plant cell to execute the function, but does not integrate into the genome of the plant, so that an exogenous gene cannot be stably inherited.

The term "genome-editing technique" refers to the techniques used to modify the genome that are capable of precisely manipulating the genomic sequences to realize operations such as site-directed gene mutations, insertions and deletions. At present, the genome-editing techniques mainly include homing endonucleases (HEs), Zinc-finger nucleases (ZFNs), transcription activator-like effector nucleases (TALENs), and Clustered regulatory interspaced short palindromic repeat (CRISPR) technologies.

Numerous transformation vectors available for plant transformation are known to those of ordinary skills in the art, and the genes pertinent to this invention can be used in conjunction with any such vectors. The selection of vector will depend upon the preferred transformation technique and the target species for transformation. For certain target species, different antibiotic or herbicide selection markers may be preferred. Selection markers used routinely in transformation include the nptII gene (which was published by Bevan et al., Nature 304:184-187 (1983)), which confers resistance to kanamycin and related antibiotics or herbicides; the pat and bar genes, which confer resistance to the herbicide glufosinate (also called phosphinothricin; see White et al., Nucl. Acids Res 18: 1062 (1990), Spencer et al. Theon. Appl. Genet. 79: 625-631 (1990) and U.S. Pat. Nos. 5,561,236 and 5,276,268); the hph gene, which confers resistance to the antibiotic hygromycin (Blochinger & Diggelmann, Mol. Cell. Biol. 4: 2929-2931); the dhfr gene, which confers resistance to methatrexate (Bourouis et al., EMBO J. 2(7): 1099-1104 (1983)); the EPSPS gene, which confers resistance to glyphosate (U.S. Pat. Nos. 4,940,935 and 5,188,642); the glyphosate N-acetyltransferase (GAT) gene, which also confers resistance to glyphosate (Castle et al. (2004) Science, 304:1151-1154; U.S. Patent App. Pub. Nos. 20070004912, 20050246798, and 20050060767); and the mannose-6-phosphate isomerase gene, which provides the ability to metabolize mannose (U.S. Pat. Nos. 5,767,378 and 5,994,629).

Methods for regeneration of plants are also well-known in the art. For example, Ti plasmid vectors have been utilized for the delivery of foreign DNA, as well as direct DNA uptake, liposomes, electroporation, microinjection, and microprojectiles.

The planting system in the present invention refers to a combination of a plant and any herbicide tolerance thereof and/or an herbicide treatment available in different plant developmental stages, thus producing a high-yielding and/or damage-reduced plant.

In the present invention, weeds refer to plants competing with the cultivated target plants in the plant growth environment.

The term "control" and/or "prevention" in the present invention refers to at least a direct application of (e.g., by spraying) an effective dose of an HPPD-inhibitor herbicide to the plant growth environment, so as to minimize weed development and/or stop weed growth. At the same time, the cultivated target plants should be morphologically normal and can be cultivated under conventional methods for product consumption and/or generation; and preferably, compared to non-transgenic wild-type plants, the cultivated plants have reduced plant damage and/or an increased plant yield. The reduced plant damage includes, but is not limited to, an improved stem resistance and/or an increased grain weight, etc. The "control" and/or "prevention" effect of the mutant HPPD polypeptide on weeds can exist independently, and will not be diminished and/or lost due to the presence of other substances that can "control" and/or "prevent" the weeds. Specifically, if any tissue of a transgenic plant (containing the gene encoding the mutant HPPD polypeptide) has and/or produces the mutant HPPD polypeptide and/or another substance that can control weeds simultaneously and/or separately, then the presence of the another substance will neither affect the "control" and/or "prevention" effect of the mutant HPPD polypeptide on the weeds, nor result in the "control" and/or "prevention" effect being completely and/or partially achieved by the another substance, regardless of the mutant HPPD polypeptide.

The "plant propagule" in the present invention includes, but is not limited to, plant sexual propagules and plant vegetative propagules. The plant sexual propagules include, but are not limited to, plant seeds; and the plant vegetative propagules refer to vegetative organs or a specific tissue of a plant which can generate a new plant under *ex vivo* conditions. The vegetative organs or the specific tissue include, but are not limited to, roots, stems and leaves, for example: plants with roots as the vegetative propagules including strawberries, sweet potatoes and the like; plants with stems as the vegetative propagules including sugar cane, potatoes (tubers) and the like; and plants with leaves as the vegetative propagules including aloe, begonias and the like.

The present invention may confer a new herbicide resistance trait to a plant, and no adverse effects on the phenotypes (including yields) are observed. The plant in the present invention can tolerate, e.g., 2×, 3×, 4× or 5× the general application level of at least one herbicide tested. The improvement of these levels of tolerance is within the scope of the present invention. For example, foreseeable optimization and further development can be performed on various techniques known in the art, in order to increase the expression of a given gene.

The present invention provides a mutant HPPD polypeptide, a coding gene and use thereof, having the following advantages:
1. The present invention first discloses that various forms of mutations (preferably, mutations from phenylalanine to alanine, glycine or valine) occurring at position 372 of the HPPD polypeptide derived from different species or different ecotypes within the same species can confer higher tolerance to HPPD-inhibitor herbicides upon the plants, and in particular the tolerance to four-fold field concentration of topramezone, and thus has a very broad application prospect in plants.
2. The mutation at position 372 of the HPPD polypeptide of the present invention in combination with those at other positions can also confer tolerance to topramezone upon the plants.

The technical solution of the present invention is further illustrated in details through the drawings and examples below.

### BRIEF DESCRIPTION OF THE DRAWINGS

FIG. 1 is a schematic structural diagram of a recombinant expression vector DBN11375 containing the AsHPPDm-F372A-02 nucleotide sequence for *Arabidopsis thaliana* according to the present invention;
FIG. 2 is a photo showing the tolerance of the *Arabidopsis thaliana* T₁ plants into which an *Avena sativa* HPPD gene (unmutated and mutated) was introduced to topramezone according to the present invention (A: wild-type *Arabidopsis thaliana* plants; B: *Arabidopsis thaliana* T₁ plants into which an AsHPPD-02 nucleotide was introduced; C: *Arabidopsis thaliana* T₁ plants into which an AsHPPDm-F372A-02 nucleotide sequence was introduced);
FIG. 3 is a phylogenetic tree of HPPDs from different species according to the present invention;
FIG. 4 is a series of photos showing the tolerance of *Arabidopsis thaliana* T₁ plants into which HPPD genes (unmutated and mutated) from different sources were introduced to topramezone according to the present invention (A: wild-type *Arabidopsis thaliana* plants; B: *Arabidopsis thaliana* T₁plants into which unmutated HPPD genes (optimized according to codon usage bias) were introduced; C: *Arabidopsis thaliana* T₁ plants into which mutated HPPD genes (F372A) were introduced);
FIG. 5 is a schematic structural diagram of a control recombinant expression vector DBN11375NN according to the present invention;
FIG. 6 is a schematic structural diagram of a recombinant expression vector DBN11950 containing the AsHPPDm-F372A-02 nucleotide sequence for *Oryza sativa* according to the present invention;

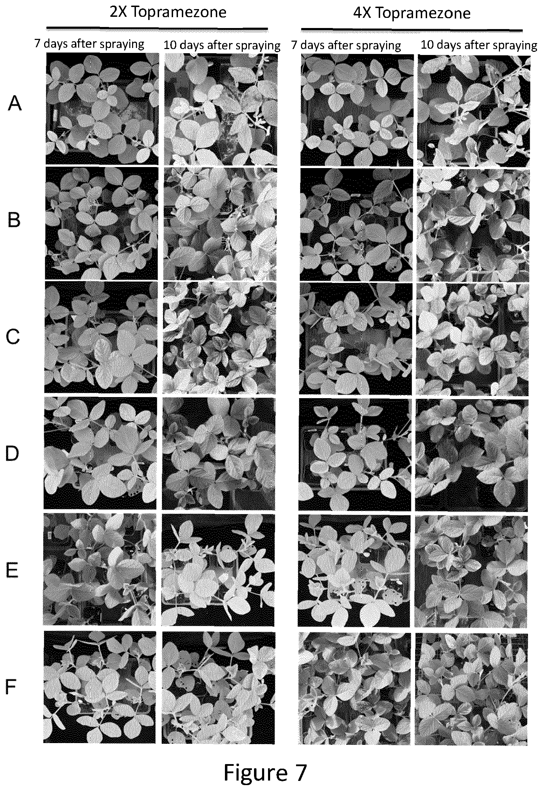

FIG. 7 is a series of photos showing the tolerance of T₁ soybean plants into which a mutated HPPD gene was introduced to topramezone according to the present invention (A: soybean T₁ plants into which a control vector DBN11375NN was introduced; B: soybean T₁ plants into which an AsHPPDm-F372A-02 nucleotide sequence was introduced; C: soybean T₁ plants into which a ZmHPPDm-F372A-02 nucleotide sequence was introduced; D: soybean T₁ plants into which a PfHPPDm-F372A-02 nucleotide sequence was introduced; E: soybean T₁ plants into which an AsHPPDm-F372A-A110 nucleotide sequence was introduced; F: soybean T₁ plants into which a PfHPPDm-F372A-G413W nucleotide sequence was introduced).

### DETAILED DESCRIPTION OF THE INVENTION

The embodiments of the mutant hydroxyphenylpyruvate dioxygenase polypeptide, the coding gene and use thereof according to the present invention will be further illustrated in specific examples.

### Example 1: Selection of position 372 of AsHPPD for mutation and verification of the mutation effect

### 1. Acquisition of AsHPPD and AsHPPDm-F372A genes

The amino acid sequence (440 amino acids) of the *Avena sativa* native HPPD (AsHPPD) is set forth as SEQ ID NO: 1 in the SEQUENCE LISTING; the AsHPPD-01 nucleotide sequence (1323 nucleotides) encoding the AsHPPD is set forth as SEQ ID NO: 2 in the SEQUENCE LISTING, and the AsHPPD-02 nucleotide sequence encoding the AsHPPD, which was obtained based on the *Arabidopsis thaliana*/soybean/rice common codon usage bias, is set forth as SEQ ID NO: 3 in the SEQUENCE LISTING.

Position 372 of the AsHPPD amino acid sequence was mutated from the original phenylalanine (F) to alanine (A), to obtain a mutant AsHPPD (AsHPPDm-F372A) amino acid sequence as set forth in SEQ ID NO: 4 in the SEQUENCE LISTING. The mutant AsHPPD-01 (AsHPPDm-F372A-01) nucleotide sequence is set forth as SEQ ID NO: 5 in the SEQUENCE LISTING. The AsHPPDm-F372A-02 nucleotide sequence encoding the AsHPPDm-F372A, which was obtained based on the *Arabidopsis thaliana*/soybean/rice common codon usage bias, is set forth as SEQ ID NO: 6 in the SEQUENCE LISTING.

### 2. Synthesis of the aforementioned nucleotide sequences

The 5' and 3' ends of the synthesized AsHPPD-02 nucleotide sequence (SEQ ID NO: 3) and AsHPPDm-F372A-02 nucleotide sequence (SEQ ID NO: 6) were respectively linked to a universal adapter primer 1:
Universal adapter primer 1 for the 5' end: 5'-agtttttctgattaacagactagt-3', as set forth in SEQ ID NO: 399 in the SEQUENCE LISTING; and
Universal adapter primer 1 for the 3' end: 5'-caaatgtttgaacgatcggcgcgcc-3', as set forth in SEQ ID NO: 400 in the SEQUENCE LISTING.

### 3. Construction of recombinant expression vectors containing Avena sativa HPPD genes (F372A) for Arabidopsis thaliana

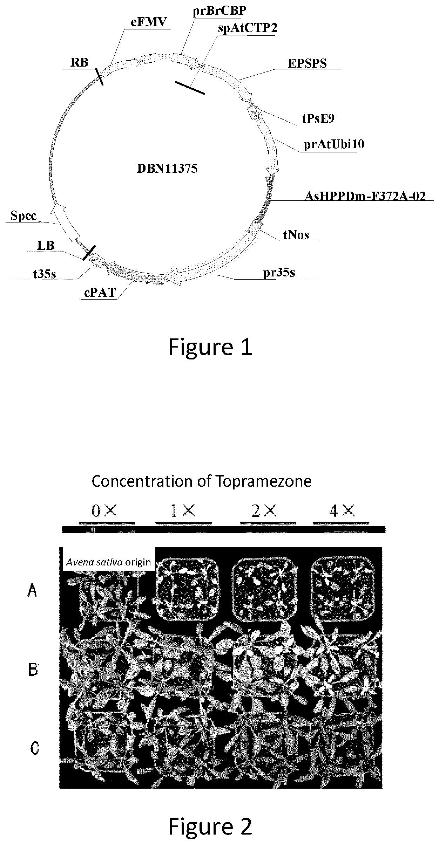

A plant expression vector DBNBC-01 was subjected to double digestion using restriction enzymes *Spe* I and *Asc* I to linearize the plant expression vector. The digestion product was purified to obtain the linearized DBNBC-01 expression vector backbone (vector backbone: pCAMBIA2301 (which is available from CAMBIA)) which then underwent a recombination reaction with the AsHPPDm-F372A-02 nucleotide sequence linked to the universal adapter primer 1, according to the procedure of Takara In-Fusion products seamless connection kit (Clontech, CA, USA, CAT: 121416) instructions, to construct a recombinant expression vector DBN11375 with the schematic structure as shown in FIG. 1 (Spec: spectinomycin gene; RB: right border; eFMV: 34S enhancer of Figwort mosaic virus (SEQ ID NO: 7); prBrCBP: promoter of oilseed rape eukaryotic elongation factor gene 1α (Tsf1) (SEQ ID NO: 8); spAtCTP2: *Arabidopsis thaliana* chloroplast transit peptide (SEQ ID NO: 9); EPSPS: 5-enolpyruvylshikimate-3-phosphate synthase gene (SEQ ID NO: 10); tPsE9: terminator of a pea RbcS gene (SEQ ID NO: 11); prAtUbi10: promoter of an *Arabidopsis thaliana* Ubiquitin 10 gene (SEQ ID NO: 12); AsHPPDm-F372A-02: AsHPPDm-F372A-02 nucleotide sequence (SEQ ID NO: 6); tNos: terminator of a nopaline synthase gene (SEQ ID NO: 13); pr35S: the cauliflower mosaic virus 35S promoter (SEQ ID NO: 14); PAT: phosphinothricin acetyltransferase gene (SEQ ID NO: 15); t35S: cauliflower mosaic virus 35S terminator (SEQ ID NO: 16); LB: left border).

*Escherichia coli* T₁ competent cells were transformed with the recombinant expression vector DBN11375 by using a heat shock method under the following heat shock conditions: 50 µL of *Escherichia coli* T₁ competent cells and 10 µL of plasmid DNA (recombinant expression vector DBN11375) were water-bathed at 42°C for 30 seconds, shake cultured at 37°C for 1 hour (using a shaker at a rotation speed of 100 rpm for shaking), and then cultured under the condition of a temperature of 37°C on the LB solid plate containing 50 mg/L of spectinomycin for 12 hours; white bacterial colonies were picked out, and cultured under the condition of a temperature of 37°C overnight in an LB liquid culture medium (10 g/L of tryptone, 5 g/L of yeast extract, 10 g/L of NaCl, and 50 mg/L of spectinomycin; adjusted to a pH of 7.5 with NaOH). The plasmids in the cells were extracted through an alkaline method: the bacteria solution was centrifuged at a rotation speed of 12,000 rpm for 1 min, the supernatant was removed, and the precipitated thalli were suspended with 100 µL of ice pre-cooled solution I (25 mM Tris-HCl, 10 mM EDTA (ethylenediaminetetraacetic acid), and 50 mM glucose, with a pH of 8.0); 200 µL of newly prepared solution II (0.2M NaOH, 1% SDS (sodium dodecyl sulfate)) was added, mixed by inverting the tube 4 times, and placed on ice for 3-5 min; 150 µL of ice-cold solution III (3 M potassium acetate, 5 M acetic acid) was added, mixed uniformly immediately and placed on ice for 5-10 min; the mixture was centrifuged under the conditions of a temperature of 4°C and a rotation speed of 12,000 rpm for 5 min, 2-fold volumes of anhydrous ethanol was added to the supernatant, mixed uniformly and placed at room temperature for 5 min; the mixture was centrifuged under the conditions of a temperature of 4°C and a rotation speed of 12,000 rpm for 5 min, the supernatant was discarded, and the precipitate was washed with ethanol at a concentration of 70% (V/V) and then was air dried; 30 µL of TE (10 mM Tris-HCl, and 1 mM EDTA, with a pH of 8.0) containing RNase (20 µg/mL) was added to dissolve the precipitate; the obtained product was water bathed at a temperature of 37°C for 30 min to digest the RNA; and stored at a temperature of -20°C for use. The extracted plasmids were identified by sequencing. The results showed that the nucleotide sequence between the *Spe*I and *Asc*I sites in the recombinant expression vector DBN11375 was the one as set forth in SEQ ID NO: 6 in the SEQUENCE LISTING, i.e., the AsHPPDm-F372A-02 nucleotide sequence.

### According to the method for constructing the recombinant expression vector DBN11375 as described above, the AsHPPD-02 nucleotide sequence linked to the universal adapter primer 1 was subjected to a recombination reaction with the linearized DBNBC-01 expression vector backbone to constructe a recombinant expression vector DBN11375N. Sequencing verified that the nucleotide sequence in the recombinant expression vector DBN11375N comprises the one as set forth in SEQ ID NO: 3 in the SEQUENCE LISTING; i.e., the AsHPPD-02 nucleotide sequence was inserted correctly.

### 4. Transformation of Agrobacterium with the recombinant expression vectors for Arabidopsis thaliana

The recombinant expression vectors DBN11375 and DBN11375N which had been constructed correctly were respectively transformed into *Agrobacterium* GV3101 using a liquid nitrogen method, under the following transformation conditions: 100 µL of *Agrobacterium* GV3101 and 3 µL of plasmid DNA (recombinant expression vector) were placed in liquid nitrogen for 10 minutes, and bathed in warm water at 37°C for 10 min; the transformed *Agrobacterium* GV3101 was inoculated into an LB tube, cultured under the conditions of a temperature of 28°C and a rotation speed of 200 rpm for 2 hours, and spread on the LB solid plate containing 50 mg/L of rifampicin and 50 mg/L of spectinomycin until positive single clones were grown, and single clones were picked out for culturing and the plasmids thereof were extracted. The extracted plasmids were identified by sequencing. The results showed that the structures of the recombinant expression vectors DBN11375 and DBN11375N were completely correct.

### 5. Acquisition of transgenic Arabidopsis thaliana plants

Seeds of wild-type *Arabidopsis thaliana* were suspended in a 0.1% (w/v) agarose solution. The suspended seeds were stored at 4°C for 2 days to fulfill the need for dormancy, in order to ensure synchronous seed germination. Vermiculite was mixed with horse manure soil, the mixture was sub-irrigated with water to wet, and the soil mixture was allowed to drain the water away for 24 hours. The pretreated seeds were sowed in the soil mixture and covered with a moisturizing cover for 7 days. The seeds were germinated and the plants were cultivated in a greenhouse under long sunlight conditions (16-hour light/8-hour dark) at a constant temperature (22°C) and a constant humidity (40-50%), with a light intensity of 120-150 µmol/m²s⁻¹. The plants were initially irrigated with Hoagland's nutrient solution and then with deionized water, thus keeping the soil moist, but not water penetrated.

*Arabidopsis thaliana* was transformed using the flower soaking method. One or more 15-30 mL pre-cultures of a LB culture solution (10 g/L of tryptone, 5 g/L of yeast extract, and 10 g/L of NaCl; adjusted to a pH of 7.5 with NaOH) containing spectinomycin (50 mg/L) and rifampicin (10 mg/L) were inoculated with the picked *Agrobacterium* colonies. The pre-cultures were incubated at a temperature of 28° C and a rotation speed of 220 rpm with shaking at a constant speed overnight. Each pre-culture was used to inoculate two 500 mL cultures of the LB culture solution containing spectinomycin (50 mg/L) and rifampicin (10 mg/L), and the cultures were incubated at 28°C with continuous shaking overnight. Centrifugation at a rotation speed of about 4,000 rpm was carried out at room temperature for 20 minutes to precipitate cells, and the resulting supernatant was discarded. The cell precipitate was gently re-suspended in 500 mL of an osmotic medium which contained 1/2×MS salt/B5 vitamin, 10% (w/v) sucrose, 0.044 µM of benzylaminopurine (10 µL/L (1 mg/mL stock solution in DMSO)) and 300 µL/L of Silvet L-77. About 1-month-old *Arabidopsis thaliana* plants were soaked in an osmotic culture medium which contained re-suspended cells for 15 seconds to ensure immersion of the latest inflorescence. Then, the *Arabidopsis thaliana* plants were reclined laterally and covered and they were kept wet in dark for 24 hours. The *Arabidopsis thaliana* plants were normally cultivated with a photoperiod of 16 hours of light/8 hours of darkness at 22°C. Seeds were harvested after about 4 weeks.

The newly harvested (AsHPPDm-F372A-02 nucleotide sequence and AsHPPD-02 nucleotide sequence) T₁ seeds were dried at room temperature for 7 days. The seeds were sowed in 26.5 cm ×51 cm germination disks, and 200 mg of T₁ seeds (about 10,000 seeds) were accepted per disk, wherein the seeds had been previously suspended in distilled water and stored at 4°C for 2 days to fulfill the need for dormancy, in order to ensure synchronous seed germination.

Vermiculite was mixed with horse manure soil, the mixture was sub-irrigated with water to wet, and water was drained by gravity. The pretreated seeds were sowed evenly in the soil mixture using a pipette, and covered with a moisturizing cover for 4-5 days. The cover was removed 1 day before the post-emergence spraying application of glufosinate (used to select the co-transformed PAT gene) for the selection of initial transformant.

The T₁ plants were sprayed with a 0.2% solution of a Liberty herbicide (200 g ai/L of glufosinate) by a DeVilbiss compressed air nozzle at a spray volume of 10 mL/disk (703 L/ha) 7 days after planting (DAP) and 11 DAP (the cotyledon stage and 2-4 leaf stage, respectively) to provide an effective amount of glufosinate of 280 g ai/ha per application. Surviving plants (actively growing plants) were identified 4-7 days after the final spraying, and transplanted to 7 cm×7 cm square pots prepared from horse manure soil and vermiculite (3-5 plants/disk). The transplanted plants were covered with a moisturizing cover for 3-4 days, and placed in a 22°C culture chamber or directly transferred into a greenhouse as described above. Then, the cover was removed, and at least 1 day before testing the ability of the mutant HPPD gene to provide topramezone herbicide tolerance, the plants were planted in a greenhouse (22±5°C, 50±30% RH, 14 hours of light: 10 hours of darkness, a minimum of 500 µE/m²s⁻¹ natural+supplemental light).

### 6. Detection of the herbicide tolerance of the transgenic Arabidopsis thaliana plants containing the AsHPPDm-F372A-02 nucleotide sequence.

T₁ transformants were initially selected from the untransformed seeds using a glufosinate selection scheme. About 20,000 T₁ seeds were screened, and 314 T₁ generation positive transformants (PAT gene) were identified, with a transformation efficiency of about 1.6%.

The *Arabidopsis thaliana* T₁ plants into which the AsHPPDm-F372A-02 nucleotide sequence was introduced, *Arabidopsis thaliana* T₁ plants into which the AsHPPD-02 nucleotide sequence was introduced and wild-type *Arabidopsis thaliana* plants (18 days after sowing) were sprayed with topramezone at four different concentrations, i.e., 25 g ai/ha (one-fold field concentration, 1×), 50 g ai/ha (two-fold field concentration, 2×), 100 g ai/ha (four-fold field concentration, 4×) and 0 g ai/ha (water, 0×) to determine the tolerance of *Arabidopsis thaliana* to the herbicide. The degree of damage caused by the herbicide was measured for each plant according to the proportion of bleached leaf area (the proportion of bleached leaf area=bleached leaf area/total leaf area×100%) 7 days after spraying (7 DAT): the case where there is basically no bleached phenotype is defined as grade 0, the case where the proportion of bleached leaf area is less than 50% is defined as grade 1,the case where the proportion of bleached leaf area is more than 50% is defined as grade 2, and the case where the proportion of bleached leaf area is 100% grade is defined as grade 3.

According to the formula X=[Σ(N×S)/(T×M)]×100, the performance of resistance of the transformation event of each recombinant expression vector was scored (X-the score for pesticide damage, N-the number of plants with the same grade of damage, S- the pesticide damage grade, T-the total number of plants, M-the maximum grade of pesticide damage) and the resistance is evaluated based on the scores: highly resistant plants (scores 0-15), moderately resistant plants (scores 16-33), poorly resistant plants (scores 34-67) and non-resistant plants (scores 68-100). The experimental results are shown in TABLE 1 and FIG. 2.

**TABLE 1 Topramezone tolerance of transgenic Arabidopsis thaliana T₁ plants**

| *Arabidopsis thaliana* genotypes | Concentration (g ai/ha) | Classification and statistics of the grade of pesticide damage | | | | Scores | Resistance evaluation |
|---|---|---|---|---|---|---|---|
| | | Grade 0 | Grade 1 | Grade 2 | Grade 3 | | |
| Wild type | 0 | 16 | 0 | 0 | 0 | 0 | |
| | 25 | 0 | 0 | 0 | 16 | 100 | non-resistant |
| | 50 | 0 | 0 | 0 | 16 | 100 | non-resistant |
| | 100 | 0 | 0 | 0 | 16 | 100 | non-resistant |
| AsHPPD-02 | 0 | 16 | 0 | 0 | 0 | 0 | |
| | 25 | 0 | 4 | 12 | 0 | 58 | poorly resistant |
| | 50 | 0 | 0 | 6 | 10 | 88 | non-resistant |
| | 100 | 0 | 0 | 4 | 12 | 92 | non-resistant |
| AsHPPDm-F372A-02 | 0 | 16 | 0 | 0 | 0 | 0 | |
| | 25 | 16 | 0 | 0 | 0 | 0 | highly resistant |
| | 50 | 16 | 0 | 0 | 0 | 0 | highly resistant |
| | 100 | 16 | 0 | 0 | 0 | 0 | highly resistant |

The results of TABLE 1 and FIG. 2 show that the *Arabidopsis thaliana* T₁ plants into which the AsHPPDm-F372A-02 nucleotide sequence was introduced had a good tolerance to (highly resistant to) topramezone, while the wild-type *Arabidopsis thaliana* plants and the *Arabidopsis thaliana* T₁ plants into which the AsHPPD-02 nucleotide was introduced had no or relatively low tolerance to topramezone at various concentrations. Thus, it is inferred that mutation of position 372 of the HPPD amino acid sequence (F372A) can confer tolerance to topramezone upon the plants.

### Example 2: Mutation of position 372 of the HPPD amino acid sequences from different species (F372A) and verification of the mutation effect

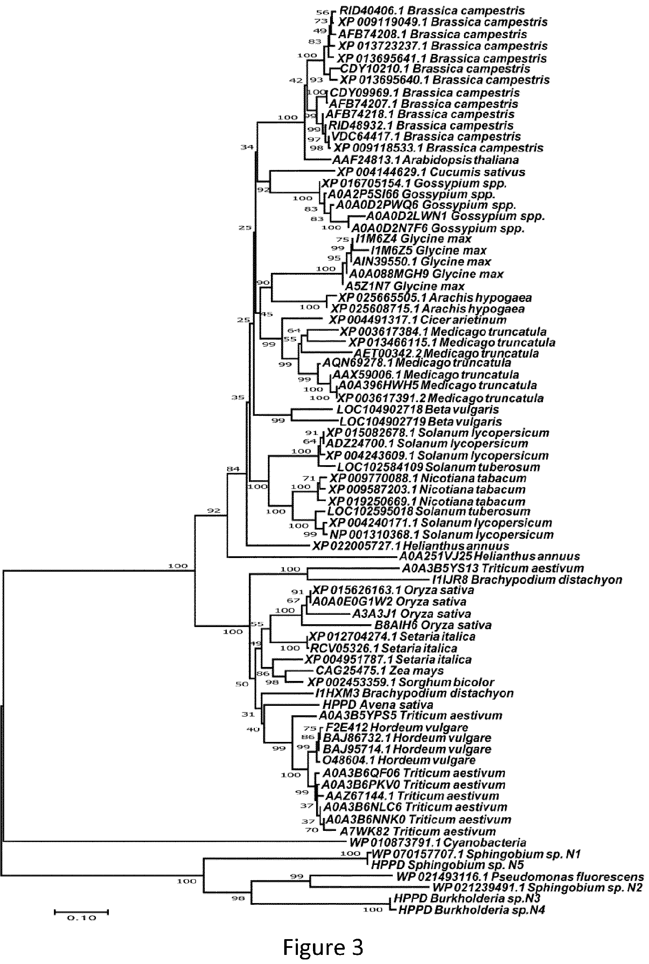

In order to further verify the effect of mutation of position 372 of the HPPD amino acid sequence, a phylogenetic tree of HPPDs from different species (as shown in FIG. 3) was analyzed. HPPDs from representative species on different branches were selected and position 372 of the amino acid sequence was mutated (F372A) so as to verify the mutation effect.

### 1. Acquisition of HPPDs from different species and mutant HPPDs (F372A)

The amino acid sequence (444 amino acids) of the native *Zea mays* HPPD (ZmHPPD) is set forth as SEQ ID NO: 17 in the SEQUENCE LISTING; the ZmHPPD-01 nucleotide sequence (1335 nucleotides) encoding the ZmHPPD is set forth as SEQ ID NO: 18 in the SEQUENCE LISTING, and the ZmHPPD-02 nucleotide sequence encoding the ZmHPPD, which was obtained based on the *Arabidopsis thaliana*/soybean/rice common codon usage bias, is set forth as SEQ ID NO: 19 in the SEQUENCE LISTING. Position 372 of the ZmHPPD amino acid sequence was mutated from the original phenylalanine to alanine to obtain a mutant ZmHPPD (ZmHPPDm-F372A) amino acid sequence as set forth in SEQ ID NO: 20 in the SEQUENCE LISTING. The mutant ZmHPPD-01 (ZmHPPDm-F372A-01) nucleotide sequence is set forth as SEQ ID NO: 21 in the SEQUENCE LISTING. The ZmHPPDm-F372A-02 nucleotide sequence encoding the ZmHPPDm-F372A, which was obtained based on the *Arabidopsis thaliana*/soybean/rice common codon usage bias, is set forth as SEQ ID NO: 22 in the SEQUENCE LISTING.

The amino acid sequence (445 amino acids) of the native *Arabidopsis thaliana* HPPD (AtHPPD) is set forth as SEQ ID NO: 23 in the SEQUENCE LISTING; the AtHPPD nucleotide sequence (1338 nucleotides) encoding the AtHPPD is set forth as SEQ ID NO: 24 in the SEQUENCE LISTING. Position 372 of the AtHPPD amino acid sequence was mutated from the original phenylalanine to alanine to obtain a mutant AtHPPD (AtHPPDm-F372A) amino acid sequence as set forth in SEQ ID NO: 25 in the SEQUENCE LISTING. The AtHPPDm-F372A nucleotide sequence encoding the AtHPPDm-F372A is set forth as SEQ ID NO: 26 in the SEQUENCE LISTING.

The amino acid sequence (358 amino acids) of the native *Pseudomonas fluorescens* HPPD (PfHPPD) is set forth as SEQ ID NO: 27 in the SEQUENCE LISTING; the PfHPPD-01 nucleotide sequence (1077 nucleotides) encoding the PfHPPD is set forth as SEQ ID NO: 28 in the SEQUENCE LISTING, and the PfHPPD-02 nucleotide sequence encoding the PfHPPD, which was obtained based on the *Arabidopsis thaliana*/soybean/rice common codon usage bias, is set forth as SEQ ID NO: 29 in the SEQUENCE LISTING. Position 372 of the PfHPPD amino acid sequence was mutated from the original phenylalanine to alanine to obtain a mutant PfHPPD (PfHPPDm-F372A) amino acid sequence as set forth in SEQ ID NO: 30 in the SEQUENCE LISTING. The mutant PfHPPD-01 (PfHPPDm-F372A-01) nucleotide sequence is set forth as SEQ ID NO: 31 in the SEQUENCE LISTING. The PfHPPDm-F372A-02 nucleotide sequence encoding the PfHPPDm-F372A, which was obtained based on the *Arabidopsis thaliana*/soybean/rice common codon usage bias, is set forth as SEQ ID NO: 32 in the SEQUENCE LISTING.

The amino acid sequence (436 amino acids) of the native *Gossypium hirsutum* HPPD (GsHPPD) is set forth as SEQ ID NO: 33 in the SEQUENCE LISTING; the GsHPPD-01 nucleotide sequence (1311 nucleotides) encoding the GsHPPD is set forth as SEQ ID NO: 34 in the SEQUENCE LISTING, and the GsHPPD-02 nucleotide sequence encoding the GsHPPD, which was obtained based on the *Arabidopsis thaliana* codon usage bias, is set forth as SEQ ID NO: 35 in the SEQUENCE LISTING. Position 372 of the GsHPPD amino acid sequence was mutated from the original phenylalanine to alanine to obtain a mutant GsHPPD (GsHPPDm-F372A) amino acid sequence as set forth in SEQ ID NO: 36 in the SEQUENCE LISTING. The mutant GsHPPD-01 (GsHPPDm-F372A-01) nucleotide sequence is set forth as SEQ ID NO: 37 in the SEQUENCE LISTING. The GsHPPDm-F372A-02 nucleotide sequence encoding the GsHPPDm-F372A, which was obtained based on the *Arabidopsis thaliana* codon usage bias, is set forth as SEQ ID NO: 38 in the SEQUENCE LISTING.

The amino acid sequence (436 amino acids) of the native *Triticum aestivum* HPPD (TaHPPD) is set forth as SEQ ID NO: 39 in the SEQUENCE LISTING; the TaHPPD-01 nucleotide sequence (1311 nucleotides) encoding the TaHPPD is set forth as SEQ ID NO: 40 in the SEQUENCE LISTING, and the TaHPPD-02 nucleotide sequence encoding the TaHPPD, which was obtained based on the *Arabidopsis thaliana* codon usage bias, is set forth as SEQ ID NO: 41 in the SEQUENCE LISTING. Position 372 of the TaHPPD amino acid sequence was mutated from the original phenylalanine to alanine to obtain a mutant TaHPPD (TaHPPDm-F372A) amino acid sequence as set forth in SEQ ID NO: 42 in the SEQUENCE LISTING. The mutant TaHPPD-01 (TaHPPDm-F372A-01) nucleotide sequence is set forth as SEQ ID NO: 43 in the SEQUENCE LISTING. The TaHPPDm-F372A-02 nucleotide sequence encoding the TaHPPDm-F372A, which was obtained based on the *Arabidopsis thaliana* codon usage bias, is set forth as SEQ ID NO: 44 in the SEQUENCE LISTING.

The amino acid sequence (436 amino acids) of the native *Brachypodium distachyon* HPPD (BdHPPD) is set forth as SEQ ID NO: 45 in the SEQUENCE LISTING; the BdHPPD-01 nucleotide sequence (1311 nucleotides) encoding the BdHPPD is set forth as SEQ ID NO: 46 in the SEQUENCE LISTING, and the BdHPPD-02 nucleotide sequence encoding the BdHPPD, which was obtained based on the *Arabidopsis thaliana* codon usage bias, is set forth as SEQ ID NO: 47 in the SEQUENCE LISTING. Position 372 of the BdHPPD amino acid sequence was mutated from the original phenylalanine to alanine to obtain a mutant BdHPPD (BdHPPDm-F372A) amino acid sequence as set forth in SEQ ID NO: 48 in the SEQUENCE LISTING. The mutant BdHPPD-01 (BdHPPDm-F372A-01) nucleotide sequence is set forth as SEQ ID NO: 49 in the SEQUENCE LISTING. The BdHPPDm-F372A-02 nucleotide sequence encoding the BdHPPDm-F372A, which was obtained based on the *Arabidopsis thaliana* codon usage bias, is set forth as SEQ ID NO: 50 in the SEQUENCE LISTING.

The amino acid sequence (434 amino acids) of the native *Hordeum vulgare* HPPD (HvHPPD) is set forth as SEQ ID NO: 51 in the SEQUENCE LISTING; the HvHPPD-01 nucleotide sequence (1305 nucleotides) encoding the HvHPPD is set forth as SEQ ID NO: 52 in the SEQUENCE LISTING, and the HvHPPD-02 nucleotide sequence encoding the HvHPPD, which was obtained based on the *Arabidopsis thaliana* codon usage bias, is set forth as SEQ ID NO: 53 in the SEQUENCE LISTING. Position 372 of the HvHPPD amino acid sequence was mutated from the original phenylalanine to alanine to obtain a mutant HvHPPD (HvHPPDm-F372A) amino acid sequence as set forth in SEQ ID NO: 54 in the SEQUENCE LISTING. The mutant HvHPPD-01 (HvHPPDm-F372A-01) nucleotide sequence is set forth as SEQ ID NO: 55 in the SEQUENCE LISTING. The HvHPPDm-F372A-02 nucleotide sequence encoding the HvHPPDm-F372A, which was obtained based on the *Arabidopsis thaliana* codon usage bias, is set forth as SEQ ID NO: 56 in the SEQUENCE LISTING.

The amino acid sequence (441 amino acids) of the native *Setaria italica* HPPD (SiHPPD) is set forth as SEQ ID NO: 57 in the SEQUENCE LISTING; the SiHPPD-01 nucleotide sequence (1326 nucleotides) encoding the SiHPPD is set forth as SEQ ID NO: 58 in the SEQUENCE LISTING, and the SiHPPD-02 nucleotide sequence encoding the SiHPPD, which was obtained based on the *Arabidopsis thaliana* codon usage bias, is set forth as SEQ ID NO: 59 in the SEQUENCE LISTING. Position 372 of the SiHPPD amino acid sequence was mutated from the original phenylalanine to alanine to obtain a mutant SiHPPD (SiHPPDm-F372A) amino acid sequence as set forth in SEQ ID NO: 60 in the SEQUENCE LISTING. The mutant SiHPPD-01 (SiHPPDm-F372A-01) nucleotide sequence is set forth as SEQ ID NO: 61 in the SEQUENCE LISTING. The SiHPPDm-F372A-02 nucleotide sequence encoding the SiHPPDm-F372A, which was obtained based on the *Arabidopsis thaliana* codon usage bias, is set forth as SEQ ID NO: 62 in the SEQUENCE LISTING.

The amino acid sequence (440 amino acids) of the native *Sorghum bicolor* HPPD (SbHPPD) is set forth as SEQ ID NO: 63 in the SEQUENCE LISTING; the SbHPPD-01 nucleotide sequence (1323 nucleotides) encoding the SbHPPD is set forth as SEQ ID NO: 64 in the SEQUENCE LISTING, and the SbHPPD-02 nucleotide sequence encoding the SbHPPD, which was obtained based on the *Arabidopsis thaliana* codon usage bias, is set forth as SEQ ID NO: 65 in the SEQUENCE LISTING. Position 372 of the SbHPPD amino acid sequence was mutated from the original phenylalanine to alanine to obtain a mutant SbHPPD (SbHPPDm-F372A) amino acid sequence as set forth in SEQ ID NO: 66 in the SEQUENCE LISTING. The mutant SbHPPD-01 (SbHPPDm-F372A-01) nucleotide sequence is set forth as SEQ ID NO: 67 in the SEQUENCE LISTING. The SbHPPDm-F372A-02 nucleotide sequence encoding the SbHPPDm-F372A, which was obtained based on the *Arabidopsis thaliana* codon usage bias, is set forth as SEQ ID NO: 68 in the SEQUENCE LISTING.

The amino acid sequence (446 amino acids) of the native *Oryza sativa* HPPD (OsHPPD) is set forth as SEQ ID NO: 69 in the SEQUENCE LISTING; the OsHPPD-01 nucleotide sequence (1341 nucleotides) encoding the OsHPPD is set forth as SEQ ID NO: 70 in the SEQUENCE LISTING, and the OsHPPD-02 nucleotide sequence encoding the OsHPPD, which was obtained based on the *Arabidopsis thaliana* codon usage bias, is set forth as SEQ ID NO: 71 in the SEQUENCE LISTING. Position 372 of the OsHPPD amino acid sequence was mutated from the original phenylalanine to alanine to obtain a mutant OsHPPD (OsHPPDm-F372A) amino acid sequence as set forth in SEQ ID NO: 72 in the SEQUENCE LISTING. The mutant OsHPPD-01 (OsHPPDm-F372A-01) nucleotide sequence is set forth as SEQ ID NO: 73 in the SEQUENCE LISTING. The OsHPPDm-F372A-02 nucleotide sequence encoding the OsHPPDm-F372A, which was obtained based on the *Arabidopsis thaliana* codon usage bias, is set forth as SEQ ID NO: 74 in the SEQUENCE LISTING.

The amino acid sequence (488 amino acids) of the native *Glycine max* HPPD (GmHPPD) is set forth as SEQ ID NO: 75 in the SEQUENCE LISTING; the GmHPPD-01 nucleotide sequence

(1467 nucleotides) encoding the GmHPPD is set forth as SEQ ID NO: 76 in the SEQUENCE LISTING, and the GmHPPD-02 nucleotide sequence encoding the GmHPPD, which was obtained based on the *Arabidopsis thaliana* codon usage bias, is set forth as SEQ ID NO: 77 in the SEQUENCE LISTING. Position 372 of the GmHPPD amino acid sequence was mutated from the original phenylalanine to alanine to obtain a mutant GmHPPD (GmHPPDm-F372A) amino acid sequence as set forth in SEQ ID NO: 78 in the SEQUENCE LISTING. The mutant GmHPPD-01 (GmHPPDm-F372A-01) nucleotide sequence is set forth as SEQ ID NO: 79 in the SEQUENCE LISTING. The GmHPPDm-F372A-02 nucleotide sequence encoding the GmHPPDm-F372A, which was obtained based on the *Arabidopsis thaliana* codon usage bias, is set forth as SEQ ID NO: 80 in the SEQUENCE LISTING.

The amino acid sequence (434 amino acids) of the native *Cicer arietinum* HPPD (CaHPPD) is set forth as SEQ ID NO: 81 in the SEQUENCE LISTING; the CaHPPD-01 nucleotide sequence (1305 nucleotides) encoding the CaHPPD is set forth as SEQ ID NO: 82 in the SEQUENCE LISTING, and the CaHPPD-02 nucleotide sequence encoding the CaHPPD, which was obtained based on the *Arabidopsis thaliana* codon usage bias, is set forth as SEQ ID NO: 83 in the SEQUENCE LISTING. Position 372 of the CaHPPD amino acid sequence was mutated from the original phenylalanine to alanine to obtain a mutant CaHPPD (CaHPPDm-F372A) amino acid sequence as set forth in SEQ ID NO: 84 in the SEQUENCE LISTING. The mutant CaHPPD-01 (CaHPPDm-F372A-01) nucleotide sequence is set forth as SEQ ID NO: 85 in the SEQUENCE LISTING. The CaHPPDm-F372A-02 nucleotide sequence encoding the CaHPPDm-F372A, which was obtained based on the *Arabidopsis thaliana* codon usage bias, is set forth as SEQ ID NO: 86 in the SEQUENCE LISTING.

The amino acid sequence (443 amino acids) of the native *Brassica napus* HPPD (BnHPPD) is set forth as SEQ ID NO: 87 in the SEQUENCE LISTING; the BnHPPD-01 nucleotide sequence (1332 nucleotides) encoding the BnHPPD is set forth as SEQ ID NO: 88 in the SEQUENCE LISTING, and the BnHPPD-02 nucleotide sequence encoding the BnHPPD, which was obtained based on the *Arabidopsis thaliana* codon usage bias, is set forth as SEQ ID NO: 89 in the SEQUENCE LISTING. Position 372 of the BnHPPD amino acid sequence was mutated from the original phenylalanine to alanine to obtain a mutant BnHPPD (BnHPPDm-F372A) amino acid sequence as set forth in SEQ ID NO: 90 in the SEQUENCE LISTING. The mutant BnHPPD-01 (BnHPPDm-F372A-01) nucleotide sequence is set forth as SEQ ID NO: 91 in the SEQUENCE LISTING. The BnHPPDm-F372A-02 nucleotide sequence encoding the BnHPPDm-F372A, which was obtained based on the *Arabidopsis thaliana* codon usage bias, is set forth as SEQ ID NO: 92 in the SEQUENCE LISTING.

The amino acid sequence (451 amino acids) of the native *Helianthus annuus* HPPD (HaHPPD) is set forth as SEQ ID NO: 93 in the SEQUENCE LISTING; the HaHPPD-01 nucleotide sequence (1356 nucleotides) encoding the HaHPPD is set forth as SEQ ID NO: 94 in the SEQUENCE LISTING, and the HaHPPD-02 nucleotide sequence encoding the HaHPPD, which was obtained based on the *Arabidopsis thaliana* codon usage bias, is set forth as SEQ ID NO: 95 in the SEQUENCE LISTING. Position 372 of the HaHPPD amino acid sequence was mutated from the original phenylalanine to alanine to obtain a mutant HaHPPD (HaHPPDm-F372A) amino acid sequence as set forth in SEQ ID NO: 96 in the SEQUENCE LISTING. The mutant HaHPPD-01 (HaHPPDm-F372A-01) nucleotide sequence is set forth as SEQ ID NO: 97 in the SEQUENCE LISTING. The HaHPPDm-F372A-02 nucleotide sequence encoding the HaHPPDm-F372A, which was obtained based on the *Arabidopsis thaliana* codon usage bias, is set forth as SEQ ID NO: 98 in the SEQUENCE LISTING.

The amino acid sequence (435 amino acids) of the native *Medicago sativa* HPPD (MsHPPD) is set forth as SEQ ID NO: 99 in the SEQUENCE LISTING; the MsHPPD-01 nucleotide sequence (1308 nucleotides) encoding the MsHPPD is set forth as SEQ ID NO: 100 in the SEQUENCE LISTING, and the MsHPPD-02 nucleotide sequence encoding the MsHPPD, which was obtained based on the *Arabidopsis thaliana* codon usage bias, is set forth as SEQ ID NO: 101 in the SEQUENCE LISTING. Position 372 of the MsHPPD amino acid sequence was mutated from the original phenylalanine to alanine to obtain a mutant MsHPPD (MsHPPDm-F372A) amino acid sequence as set forth in SEQ ID NO: 102 in the SEQUENCE LISTING. The mutant MsHPPD-01 (MsHPPDm-F372A-01) nucleotide sequence is set forth as SEQ ID NO: 103 in the SEQUENCE LISTING. The MsHPPDm-F372A-02 nucleotide sequence encoding the MsHPPDm-F372A, which was obtained based on the *Arabidopsis thaliana* codon usage bias, is set forth as SEQ ID NO: 104 in the SEQUENCE LISTING.

The amino acid sequence (434 amino acids) of the native *Beta vulgaris* HPPD (BvHPPD) is set forth as SEQ ID NO: 105 in the SEQUENCE LISTING; the BvHPPD-01 nucleotide sequence (1305 nucleotides) encoding the BvHPPD is set forth as SEQ ID NO: 106 in the SEQUENCE LISTING, and the BvHPPD-02 nucleotide sequence encoding the BvHPPD, which was obtained based on the *Arabidopsis thaliana* codon usage bias, is set forth as SEQ ID NO: 107 in the SEQUENCE LISTING. Position 372 of the BvHPPD amino acid sequence was mutated from the original phenylalanine to alanine to obtain a mutant BvHPPD (BvHPPDm-F372A) amino acid sequence as set forth in SEQ ID NO: 108 in the SEQUENCE LISTING. The mutant BvHPPD-01 (BvHPPDm-F372A-01) nucleotide sequence is set forth as SEQ ID NO: 109 in the SEQUENCE LISTING. The BvHPPDm-F372A-02 nucleotide sequence encoding the BvHPPDm-F372A, which was obtained based on the *Arabidopsis thaliana* codon usage bias, is set forth as SEQ ID NO: 110 in the SEQUENCE LISTING.

The amino acid sequence (447 amino acids) of the native *Nicotiana tabacum* HPPD (NtHPPD) is set forth as SEQ ID NO: 111 in the SEQUENCE LISTING; the NtHPPD-01 nucleotide sequence (1344 nucleotides) encoding the NtHPPD is set forth as SEQ ID NO: 112 in the SEQUENCE LISTING, and the NtHPPD-02 nucleotide sequence encoding the NtHPPD, which was obtained based on the *Arabidopsis thaliana* codon usage bias, is set forth as SEQ ID NO: 113 in the SEQUENCE LISTING. Position 372 of the NtHPPD amino acid sequence was mutated from the original phenylalanine to alanine to obtain a mutant NtHPPD (NtHPPDm-F372A) amino acid sequence as set forth in SEQ ID NO: 114 in the SEQUENCE LISTING. The mutant NtHPPD-01 (NtHPPDm-F372A-01) nucleotide sequence is set forth as SEQ ID NO: 115 in the SEQUENCE LISTING. The NtHPPDm-F372A-02 nucleotide sequence encoding the NtHPPDm-F372A, which was obtained based on the *Arabidopsis thaliana* codon usage bias, is set forth as SEQ ID NO: 116 in the SEQUENCE LISTING.

The amino acid sequence (455 amino acids) of the native *Cucumis sativus* HPPD (CsHPPD) is set forth as SEQ ID NO: 117 in the SEQUENCE LISTING; the CsHPPD-01 nucleotide sequence (1368 nucleotides) encoding the CsHPPD is set forth as SEQ ID NO: 118 in the SEQUENCE LISTING, and the CsHPPD-02 nucleotide sequence encoding the CsHPPD, which was obtained based on the *Arabidopsis thaliana* codon usage bias, is set forth as SEQ ID NO: 119 in the SEQUENCE LISTING. Position 372 of the CsHPPD amino acid sequence was mutated from the original phenylalanine to alanine to obtain a mutant CsHPPD (CsHPPDm-F372A) amino acid sequence as set forth in SEQ ID NO: 120 in the SEQUENCE LISTING. The mutant CsHPPD-01 (CsHPPDm-F372A-01) nucleotide sequence is set forth as SEQ ID NO: 121 in the SEQUENCE LISTING. The CsHPPDm-F372A-02 nucleotide sequence encoding the CsHPPDm-F372A, which was obtained based on the *Arabidopsis thaliana* codon usage bias, is set forth as SEQ ID NO: 122 in the SEQUENCE LISTING.

The amino acid sequence (447 amino acids) of the native *Solanum tuberosum* HPPD (StHPPD) is set forth as SEQ ID NO: 123 in the SEQUENCE LISTING; the StHPPD-01 nucleotide sequence (1343 nucleotides) encoding the StHPPD is set forth as SEQ ID NO: 124 in the SEQUENCE LISTING, and the StHPPD-02 nucleotide sequence encoding the StHPPD, which was obtained based on the *Arabidopsis thaliana* codon usage bias, is set forth as SEQ ID NO: 125 in the SEQUENCE LISTING. Position 372 of the StHPPD amino acid sequence was mutated from the original phenylalanine to alanine to obtain a mutant StHPPD (StHPPDm-F372A) amino acid sequence as set forth in SEQ ID NO: 126 in the SEQUENCE LISTING. The mutant StHPPD-01 (StHPPDm-F372A-01) nucleotide sequence is set forth as SEQ ID NO: 127 in the SEQUENCE LISTING. The StHPPDm-F372A-02 nucleotide sequence encoding the StHPPDm-F372A, which was obtained based on the *Arabidopsis thaliana* codon usage bias, is set forth as SEQ ID NO: 128 in the SEQUENCE LISTING.

The amino acid sequence (441 amino acids) of the native *Solanum lycopersicum* HPPD (SlHPPD) is set forth as SEQ ID NO: 129 in the SEQUENCE LISTING; the SlHPPD-01 nucleotide sequence (1326 nucleotides) encoding the SlHPPD is set forth as SEQ ID NO: 130 in the SEQUENCE LISTING, and the SlHPPD-02 nucleotide sequence encoding the SlHPPD, which was obtained based on the *Arabidopsis thaliana* codon usage bias, is set forth as SEQ ID NO: 131 in the SEQUENCE LISTING. Position 372 of the SlHPPD amino acid sequence was mutated from the original phenylalanine to alanine to obtain a mutant SlHPPD (SlHPPDm-F372A) amino acid sequence as set forth in SEQ ID NO: 132 in the SEQUENCE LISTING. The mutant SlHPPD-01 (SlHPPDm-F372A-01) nucleotide sequence is set forth as SEQ ID NO: 133 in the SEQUENCE LISTING. The SlHPPDm-F372A-02 nucleotide sequence encoding the SlHPPDm-F372A, which was obtained based on the *Arabidopsis thaliana* codon usage bias, is set forth as SEQ ID NO: 134 in the SEQUENCE LISTING.

The amino acid sequence (504 amino acids) of the native *Arachis hypogaea* HPPD (AhHPPD) is set forth as SEQ ID NO: 135 in the SEQUENCE LISTING; the AhHPPD-01 nucleotide sequence (1515 nucleotides) encoding the AhHPPD is set forth as SEQ ID NO: 136 in the SEQUENCE LISTING, and the AhHPPD-02 nucleotide sequence encoding the AhHPPD, which was obtained based on the *Arabidopsis thaliana* codon usage bias, is set forth as SEQ ID NO: 137 in the SEQUENCE LISTING. Position 372 of the AhHPPD amino acid sequence was mutated from the original phenylalanine to alanine to obtain a mutant AhHPPD (AhHPPDm-F372A) amino acid sequence as set forth in SEQ ID NO: 138 in the SEQUENCE LISTING. The mutant AhHPPD-01 (AhHPPDm-F372A-01) nucleotide sequence is set forth as SEQ ID NO: 139 in the SEQUENCE LISTING. The AhHPPDm-F372A-02 nucleotide sequence encoding the AhHPPDm-F372A, which was obtained based on the *Arabidopsis thaliana* codon usage bias, is set forth as SEQ ID NO: 140 in the SEQUENCE LISTING.

The amino acid sequence (339 amino acids) of the native *Cyanobacteria* HPPD (CyHPPD) is set forth as SEQ ID NO: 141 in the SEQUENCE LISTING; the CyHPPD-01 nucleotide sequence (1020 nucleotides) encoding the CyHPPD is set forth as SEQ ID NO: 142 in the SEQUENCE LISTING, and the CyHPPD-02 nucleotide sequence encoding the CyHPPD, which was obtained based on the *Arabidopsis thaliana* codon usage bias, is set forth as SEQ ID NO: 143 in the SEQUENCE LISTING. Position 372 of the CyHPPD amino acid sequence was mutated from the original phenylalanine to alanine to obtain a mutant CyHPPD (CyHPPDm-F372A) amino acid sequence as set forth in SEQ ID NO: 144 in the SEQUENCE LISTING. The mutant CyHPPD-01 (CyHPPDm-F372A-01) nucleotide sequence is set forth as SEQ ID NO: 145 in the SEQUENCE LISTING. The CyHPPDm-F372A-02 nucleotide sequence encoding the CyHPPDm-F372A, which was obtained based on the *Arabidopsis thaliana* codon usage bias, is set forth as SEQ ID NO: 146 in the SEQUENCE LISTING.

The amino acid sequence (350 amino acids) of the native *Sphingobium sp. N1* HPPD (N1HPPD) is set forth as SEQ ID NO: 147 in the SEQUENCE LISTING; the N1HPPD-01 nucleotide sequence (1053 nucleotides) encoding the N1HPPD is set forth as SEQ ID NO: 148 in the SEQUENCE LISTING, and the N1HPPD-02 nucleotide sequence encoding the N1HPPD, which was obtained based on the *Arabidopsis thaliana* codon usage bias, is set forth as SEQ ID NO: 149 in the SEQUENCE LISTING. Position 372 of the N1HPPD amino acid sequence was mutated from the original phenylalanine to alanine to obtain a mutant N1HPPD (N1HPPDm-F372A) amino acid sequence as set forth in SEQ ID NO: 150 in the SEQUENCE LISTING. The mutant N1HPPD-01 (N1HPPDm-F372A-01) nucleotide sequence is set forth as SEQ ID NO: 151 in the SEQUENCE LISTING. The N1HPPDm-F372A-02 nucleotide sequence encoding the N1HPPDm-F372A, which was obtained based on the *Arabidopsis thaliana* codon usage bias, is set forth as SEQ ID NO: 152 in the SEQUENCE LISTING.

The amino acid sequence (363 amino acids) of the native *Sphingobium sp. N2* HPPD (N2HPPD) is set forth as SEQ ID NO: 153 in the SEQUENCE LISTING; the N2HPPD-01 nucleotide sequence (1092 nucleotides) encoding the N2HPPD is set forth as SEQ ID NO: 154 in the SEQUENCE LISTING, and the N2HPPD-02 nucleotide sequence encoding the N2HPPD, which was obtained based on the *Arabidopsis thaliana* codon usage bias, is set forth as SEQ ID NO: 155 in the SEQUENCE LISTING. Position 372 of the N2HPPD amino acid sequence was mutated from the original phenylalanine to alanine to obtain a mutant N2HPPD (N2HPPDm-F372A) amino acid sequence as set forth in SEQ ID NO: 156 in the SEQUENCE LISTING. The mutant N2HPPD-01 (N2HPPDm-F372A-01) nucleotide sequence is set forth as SEQ ID NO: 157 in the SEQUENCE LISTING. The N2HPPDm-F372A-02 nucleotide sequence encoding the N2HPPDm-F372A, which was obtained based on the *Arabidopsis thaliana* codon usage bias, is set forth as SEQ ID NO: 158 in the SEQUENCE LISTING.

The amino acid sequence (365 amino acids) of the native *Burkholderia sp. N3* HPPD (N3HPPD) is set forth as SEQ ID NO: 159 in the SEQUENCE LISTING; the N3HPPD-01 nucleotide sequence (1098 nucleotides) encoding the N3HPPD is set forth as SEQ ID NO: 160 in the SEQUENCE LISTING, and the N3HPPD-02 nucleotide sequence encoding the N3HPPD, which was obtained based on the *Arabidopsis thaliana* codon usage bias, is set forth as SEQ ID NO: 161 in the SEQUENCE LISTING. Position 372 of the N3HPPD amino acid sequence was mutated from the original phenylalanine to alanine to obtain a mutant N3HPPD (N3HPPDm-F372A) amino acid sequence as set forth in SEQ ID NO: 162 in the SEQUENCE LISTING. The mutant N3HPPD-01 (N3HPPDm-F372A-01) nucleotide sequence is set forth as SEQ ID NO: 163 in the SEQUENCE LISTING. The N3HPPDm-F372A-02 nucleotide sequence encoding the N3HPPDm-F372A, which was obtained based on the *Arabidopsis thaliana* codon usage bias, is set forth as SEQ ID NO: 164 in the SEQUENCE LISTING.

The amino acid sequence (365 amino acids) of the native *Burkholderia sp. N4* HPPD (N4HPPD) is set forth as SEQ ID NO: 165 in the SEQUENCE LISTING; the N4HPPD-01 nucleotide sequence (1098 nucleotides) encoding the N4HPPD is set forth as SEQ ID NO: 166 in the SEQUENCE LISTING, and the N4HPPD-02 nucleotide sequence encoding the N4HPPD, which was obtained based on the *Arabidopsis thaliana* codon usage bias, is set forth as SEQ ID NO: 167 in the SEQUENCE LISTING. Position 372 of the N4HPPD amino acid sequence was mutated from the original phenylalanine to alanine to obtain a mutant N4HPPD (N4HPPDm-F372A) amino acid sequence as set forth in SEQ ID NO: 168 in the SEQUENCE LISTING. The mutant N4HPPD-01 (N4HPPDm-F372A-01) nucleotide sequence is set forth as SEQ ID NO: 169 in the SEQUENCE LISTING. The N4HPPDm-F372A-02 nucleotide sequence encoding the N4HPPDm-F372A, which was obtained based on the *Arabidopsis thaliana* codon usage bias, is set forth as SEQ ID NO: 170 in the SEQUENCE LISTING.

The amino acid sequence (350 amino acids) of the native *Sphingobium sp. N5* HPPD (N5HPPD) is set forth as SEQ ID NO: 171 in the SEQUENCE LISTING; the N5HPPD-01 nucleotide sequence (1053 nucleotides) encoding the N5HPPD is set forth as SEQ ID NO: 172 in the SEQUENCE LISTING, and the N5HPPD-02 nucleotide sequence encoding the N5HPPD, which was obtained based on the *Arabidopsis thaliana* codon usage bias, is set forth as SEQ ID NO: 173 in the SEQUENCE LISTING. Position 372 of the N5HPPD amino acid sequence was mutated from the original phenylalanine to alanine to obtain a mutant N5HPPD (N5HPPDm-F372A) amino acid sequence as set forth in SEQ ID NO: 174 in the SEQUENCE LISTING. The mutant N5HPPD-01 (N5HPPDm-F372A-01) nucleotide sequence is set forth as SEQ ID NO: 175 in the SEQUENCE LISTING. The N5HPPDm-F372A-02 nucleotide sequence encoding the N5HPPDm-F372A, which was obtained based on the *Arabidopsis thaliana* codon usage bias, is set forth as SEQ ID NO: 176 in the SEQUENCE LISTING.

### 2. Construction of recombinant expression vectors containing HPPDS from different species (F372A) for Arabidopsis thaliana

According to the method of constructing the recombinant expression vector DBN11375 containing the AsHPPDm-F372A-02 nucleotide sequence as described above in point 3 of Example 1, the ZmHPPDm-F372A-02 nucleotide sequence, AtHPPDm-F372A nucleotide sequence, PfHPPDm-F372A-02 nucleotide sequence, GsHPPDm-F372A-02 nucleotide sequence, TaHPPDm-F372A-02 nucleotide sequence, BdHPPDm-F372A-02 nucleotide sequence, HvHPPDm-F372A-02 nucleotide sequence, SiHPPDm-F372A-02 nucleotide sequence, SbHPPDm-F372A-02 nucleotide sequence, OsHPPDm-F372A-02 nucleotide sequence, GmHPPDm-F372A-02 nucleotide sequence, CaHPPDm-F372A-02 nucleotide sequence, BnHPPDm-F372A-02 nucleotide sequence, HaHPPDm-F372A-02 nucleotide sequence, MsHPPDm-F372A-02 nucleotide sequence, BvHPPDm-F372A-02 nucleotide sequence, NtHPPDm-F372A-02 nucleotide sequence, CsHPPDm-F372A-02 nucleotide sequence, StHPPDm-F372A-02 nucleotide sequence, SlHPPDm-F372A-0 nucleotide sequence, AhHPPDm-F372A-02 nucleotide sequence, CyHPPDm-F372A-02 nucleotide sequence, N1HPPDm-F372A-02 nucleotide sequence, N2HPPDm-F372A-02 nucleotide sequence, N3HPPDm-F372A-02 nucleotide sequence, N4HPPDm-F372A-02 nucleotide sequence, and N5HPPDm-F372A-02 nucleotide sequence which were linked to the universal adapter primer 1 was respectively subjected to a recombination reaction with the linearized DBNBC-01 expression vector backbone to obtain the recombinant expression vectors DBN11376 to DBN11402 in sequence. Sequencing verified that the aforementioned nucleotide sequences were inserted correctly in the recombinant expression vectors DBN11376 to DBN11402.

According to the method of constructing the recombinant expression vector DBN11375 containing the AsHPPDm-F372A-02 nucleotide sequence as described above in point 3 of Example 1, the ZmHPPD-02 nucleotide sequence, AtHPPD nucleotide sequence, PfHPPD-02 nucleotide sequence, GsHPPD-02 nucleotide sequence, TaHPPD-02 nucleotide sequence, BdHPPD-02 nucleotide sequence, HvHPPD-02 nucleotide sequence, SiHPPD-02 nucleotide sequence, SbHPPD-02 nucleotide sequence, OsHPPD-02 nucleotide sequence, GmHPPD-02 nucleotide sequence, CaHPPD-02 nucleotide sequence, BnHPPD-02 nucleotide sequence, HaHPPD-02 nucleotide sequence, MsHPPD-02 nucleotide sequence, BvHPPD-02 nucleotide sequence, NtHPPD-02 nucleotide sequence, CsHPPD-02 nucleotide sequence, StHPPD-02 nucleotide sequence, SlHPPD-02 nucleotide sequence, AhHPPD-02 nucleotide sequence, CyHPPD-02 nucleotide sequence, N1HPPD-02 nucleotide sequence, N2HPPD-02 nucleotide sequence, N3HPPD-02 nucleotide sequence, N4HPPD-02 nucleotide sequence and N5HPPD-02 nucleotide sequence which were linked to the universal adapter primer 1 was respectively subjected to a recombination reaction with the linearized DBNBC-01 expression vector backbone to obtain the recombinant expression vectors DBN11376N to DBN11402N in sequence. Sequencing verified that the aforementioned nucleotide sequences were inserted correctly in the recombinant expression vectors DBN11376N to DBN11402N.

### 3. Transformation of Agrobacterium with the recombinant expression vectors for Arabidopsis thaliana

According to the method of transforming *Agrobacterium* with the recombinant expression vectors for *Arabidopsis thaliana* as described above in point 4 of Example 1, the recombinant expression vectors DBN11376 to DBN11402 and DBN11376N to DBN11402N which had been constructed correctly were transformed into *Agrobacterium* GV3101 respectively using a liquid nitrogen method. The results were verified by sequencing, showing that the structures of the recombinant expression vectors DBN11376 to DBN11402 and DBN11376N to DBN11402N were completely correct.

### 4. Detection of the herbicide tolerance of the Arabidopsis thaliana plants into which the HPPDs (F372A) from different species were introduced

According to the method as described above in point 5 of Example 1, *Arabidopsis thaliana* inflorescences were immersed in the *Agrobacterium* solution as described in Example 3 so as to introduce the T-DNA in the recombinant expression vectors DBN11376 to DBN11402 and DBN11376N to DBN11402N constructed in Example 2 into the *Arabidopsis thaliana* chromosomes, thereby obtaining the corresponding transgenic *Arabidopsis thaliana* plants, i.e., *Arabidopsis thaliana* T₁ plants into which the ZmHPPDm-F372A-02 nucleotide sequence was introduced, *Arabidopsis thaliana* T₁ plants into which the AtHPPDm-F372A nucleotide sequence was introduced, *Arabidopsis thaliana* T₁ plants into which the PfHPPDm-F372A-02 nucleotide sequence was introduced, *Arabidopsis thaliana* T₁ plants into which the GsHPPDm-F372A-02 nucleotide sequence was introduced, *Arabidopsis thaliana* T₁ plants into which the TaHPPDm-F372A-02 nucleotide sequence was introduced, *Arabidopsis thaliana* T₁ plants into which the BdHPPDm-F372A-02 nucleotide sequence was introduced, *Arabidopsis thaliana* T₁ plants into which the HvHPPDm-F372A-02 nucleotide sequence was introduced, *Arabidopsis thaliana* T₁ plants into which the SiHPPDm-F372A-02 nucleotide sequence was introduced, *Arabidopsis thaliana* T₁ plants into which the SbHPPDm-F372A-02 nucleotide sequence was introduced, *Arabidopsis thaliana* T₁ plants into which the OsHPPDm-F372A-02 nucleotide sequence was introduced, *Arabidopsis thaliana* T₁ plants into which the GmHPPDm-F372A-02 nucleotide sequence was introduced, *Arabidopsis thaliana* T₁ plants into which the CaHPPDm-F372A-02 nucleotide sequence was introduced, *Arabidopsis thaliana* T₁ plants into which the BnHPPDm-F372A-02 nucleotide sequence was introduced, *Arabidopsis thaliana* T₁ plants into which the HaHPPDm-F372A-02 nucleotide sequence was introduced, *Arabidopsis thaliana* T₁ plants into which the MsHPPDm-F372A-02 nucleotide sequence was introduced, *Arabidopsis thaliana* T₁ plants into which the BvHPPDm-F372A-02 nucleotide sequence was introduced, *Arabidopsis thaliana* T₁ plants into which the NtHPPDm-F372A-02 nucleotide sequence was introduced, *Arabidopsis thaliana* T₁ plants into which the CsHPPDm-F372A-02 nucleotide sequence was introduced, *Arabidopsis thaliana* T₁ plants into which the StHPPDm-F372A-02 nucleotide sequence was introduced, *Arabidopsis thaliana* T₁ plants into which the SlHPPDm-F372A-02 nucleotide sequence was introduced, *Arabidopsis thaliana* T₁ plants into which the AhHPPDm-F372A-02 nucleotide sequence was introduced, *Arabidopsis thaliana* T₁ plants into which the CyHPPDm-F372A-02 nucleotide sequence was introduced, *Arabidopsis thaliana* T₁ plants into which the N1HPPDm-F372A-02 nucleotide sequence was introduced, *Arabidopsis thaliana* T₁ plants into which the N2HPPDm-F372A-02 nucleotide sequence was introduced, *Arabidopsis thaliana* T₁ plants into which the N3HPPDm-F372A-02 nucleotide sequence was introduced, *Arabidopsis thaliana* T₁ plants into which the N4HPPDm-F372A-02 nucleotide sequence was introduced, *Arabidopsis thaliana* T₁ plants into which the N5HPPDm-F372A-02 nucleotide sequence was introduced, *Arabidopsis thaliana* T₁ plants into which the ZmHPPD-02 nucleotide sequence was introduced, *Arabidopsis thaliana* T₁ plants into which the AtHPPD nucleotide sequence was introduced, *Arabidopsis thaliana* T₁ plants into which the PfHPPD-02 nucleotide sequence was introduced, *Arabidopsis thaliana* T₁ plants into which the GsHPPD-02 nucleotide sequence was introduced, *Arabidopsis thaliana* T₁ plants into which the TaHPPD-02 nucleotide sequence was introduced, *Arabidopsis thaliana* T₁ plants into which the BdHPPD-02 nucleotide sequence was introduced, *Arabidopsis thaliana* T₁ plants into which the HvHPPD-02 nucleotide sequence was introduced, *Arabidopsis thaliana* T₁ plants into which the SiHPPD-02 nucleotide sequence was introduced, *Arabidopsis thaliana* T₁ plants into which the SbHPPD-02 nucleotide sequence was introduced, *Arabidopsis thaliana* T₁ plants into which the OsHPPD-02 nucleotide sequence was introduced, *Arabidopsis thaliana* T₁ plants into which the GmHPPD-02 nucleotide sequence was introduced, *Arabidopsis thaliana* T₁ plants into which the CaHPPD-02 nucleotide sequence was introduced, *Arabidopsis thaliana* T₁ plants into which the BnHPPD-02 nucleotide sequence was introduced, *Arabidopsis thaliana* T₁ plants into which the HaHPPD-02 nucleotide sequence was introduced, *Arabidopsis thaliana* T₁ plants into which the MsHPPD-02 nucleotide sequence was introduced, *Arabidopsis thaliana* T₁ plants into which the BvHPPD-02 nucleotide sequence was introduced, *Arabidopsis thaliana* T₁ plants into which the NtHPPD-02 nucleotide sequence was introduced, *Arabidopsis thaliana* T₁ plants into which the CsHPPD-02 nucleotide sequence was introduced, *Arabidopsis thaliana* T₁ plants into which the StHPPD-02 nucleotide sequence was introduced, *Arabidopsis thaliana* T₁ plants into which the SlHPPD-02 nucleotide sequence was introduced, *Arabidopsis thaliana* T₁ plants into which the AhHPPD-02 nucleotide sequence was introduced, *Arabidopsis thaliana* T₁ plants into which the CyHPPD-02 nucleotide sequence was introduced, *Arabidopsis thaliana* T₁ plants into which the N1HPPD-02 nucleotide sequence was introduced, *Arabidopsis thaliana* T₁ plants into which the N2HPPD-02 nucleotide sequence was introduced, *Arabidopsis thaliana* T₁ plants into which the N3HPPD-02 nucleotide sequence was introduced, *Arabidopsis thaliana* T₁ plants into which the N4HPPD-02 nucleotide sequence was introduced, and *Arabidopsis thaliana* T₁ plants into which the N5HPPD-02 nucleotide sequence was introduced.

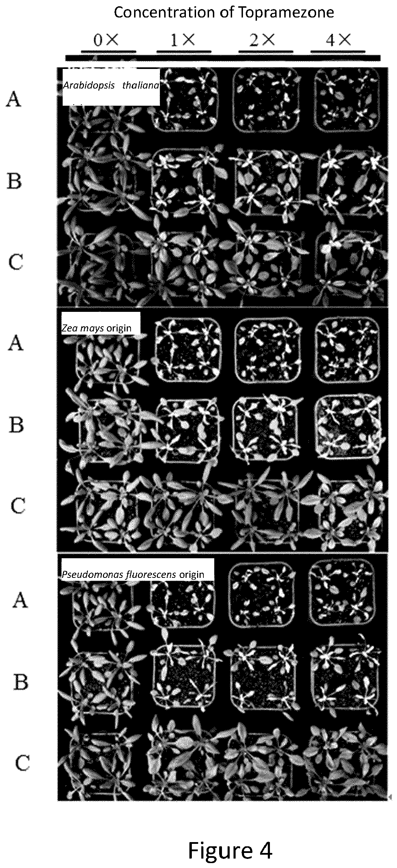

According to the method as described above in point 6 of Example 1, the aforementioned *Arabidopsis thaliana* T₁ plants and wild-type *Arabidopsis thaliana* plants (18 days after sowing) were sprayed with topramezone at three different concentrations respectively, i.e., 25 g ai/ha (one-fold field concentration, 1×), 100 g ai/ha (four-fold field concentration, 4×) and 0 g ai/ha (water, 0×) to detect the tolerance of *Arabidopsis thaliana* to the herbicide. The experimental results are shown in TABLE 2 and FIG. 4.

**TABLE 2 Topramezone tolerance of Arabidopsis thaliana T₁ plants into which HPPDs (F372A) from different species were introduced**

| Source of the gene | *Arabidopsis thaliana* genotypes | Concentration ( g ai/ha ) | Classification and statistics of pesticide damage grade | | | | Scores | Resistance evaluation |
|---|---|---|---|---|---|---|---|---|
| | | | Grade 0 | Grade 1 | Grade 2 | Grade 3 | | |
| | Wild type | 0 | 16 | 0 | 0 | 0 | 0 | |
| | *Arabidopsis* | 25 | 0 | 0 | 0 | 16 | 100 | non-resistant |
| | *thaliana* | 100 | 0 | 0 | 0 | 16 | 100 | non-resistant |
| *Zea mays* | ZmHPPD-02 | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | ZmHPPDm-F372A-02 | 0 | 16 | 0 | 0 | 0 | 0 | |
| | | 25 | 16 | 0 | 0 | 0 | 0 | highly resistant |
| | | 100 | 4 | 12 | 0 | 0 | 25 | moderately resistant |
| *Triticum aestivum* | TaHPPD-02 | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | TaHPPDm-F372A-02 | 0 | 16 | 0 | 0 | 0 | 0 | |
| | | 25 | 16 | 0 | 0 | 0 | 0 | highly resistant |
| | | 100 | 15 | 1 | 0 | 0 | 2 | highly resistant |
| *Brachypodium distachyon* | BdHPPD-02 | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | BdHPPDm-F372A-02 | 0 | 16 | 0 | 0 | 0 | 0 | |
| | | 25 | 16 | 0 | 0 | 0 | 0 | highly resistant |
| | | 100 | 6 | 10 | 0 | 0 | 21 | moderately resistant |
| *Hordeum vulgare* | HvHPPD-02 | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | HvHPPDm-F372A-02 | 0 | 16 | 0 | 0 | 0 | 0 | |
| | | 25 | 15 | 1 | 0 | 0 | 2 | highly resistant |
| | | 100 | 8 | 8 | 0 | 0 | 17 | moderately resistant |
| *Setaria italica* | SiHPPD-02 | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | SiHPPDm-F372A-02 | 0 | 16 | 0 | 0 | 0 | 0 | |
| | | 25 | 13 | 2 | 1 | 0 | 8 | highly resistant |
| | | 100 | 6 | 8 | 2 | 0 | 25 | moderately resistant |
| *Sorghum bicolor* | SbHPPD-02 | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | SbHPPDm-F372A-02 | 0 | 16 | 0 | 0 | 0 | 0 | |
| | | 25 | 16 | 0 | 0 | 0 | 0 | highly resistant |
| | | 100 | 4 | 4 | 8 | 0 | 42 | poorly resistant |
| *Oryza sativa* | OsHPPD-02 | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | OsHPPDm-F372A-02 | 0 | 16 | 0 | 0 | 0 | 0 | |
| | | 25 | 8 | 8 | 0 | 0 | 17 | moderately resistant |
| | | 100 | 0 | 0 | 0 | 16 | 100 | non-resistant |
| *Arabidopsis thaliana* | AtHPPD | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | AtHPPDm-F372A | 0 | 16 | 0 | 0 | 0 | 0 | |
| | | 25 | 4 | 4 | 8 | 0 | 42 | poorly resistant |
| | | 100 | 0 | 0 | 4 | 12 | 92 | non-resistant |
| *Gossypium hirsutum* | GsHPPD-02 | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | GsHPPDm-F372A-02 | 0 | 16 | 0 | 0 | 0 | 0 | |
| | | 25 | 16 | 0 | 0 | 0 | 0 | highly resistant |
| | | 100 | 0 | 0 | 0 | 16 | 100 | non-resistant |
| *Glycine max* | GmHPPD-02 | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | GmHPPDm-F372A-02 | 0 | 16 | 0 | 0 | 0 | 0 | |
| | | 25 | 0 | 12 | 4 | 0 | 42 | poorly resistant |
| | | 100 | 0 | 0 | 0 | 16 | 100 | non-resistant |
| *Cicer aretinum* | CaHPPD-02 | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | CaHPPD-F372A-02 | 0 | 16 | 0 | 0 | 0 | 0 | |
| | | 25 | 0 | 13 | 3 | 0 | 40 | poorly resistant |
| | | 100 | 0 | 0 | 0 | 16 | 100 | non-resistant |
| *Brassica napus* | BnHPPD-02 | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | BnHPPDm-F372A-02 | 0 | 16 | 0 | 0 | 0 | 0 | |
| | | 25 | 0 | 4 | 12 | 0 | 58 | poorly resistant |
| | | 100 | 0 | 0 | 0 | 16 | 100 | non-resistant |
| *Helianthus annuus* | HaHPPD-02 | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | HaHPPDm-F372A-02 | 0 | 16 | 0 | 0 | 0 | 0 | |
| | | 25 | 0 | 8 | 8 | 0 | 50 | poorly resistant |
| | | 100 | 0 | 2 | 12 | 2 | 67 | poorly resistant |
| *Medicago sativa* | MsHPPD-02 | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | MsHPPDm-F372A-02 | 0 | 16 | 0 | 0 | 0 | 0 | |
| | | 25 | 5 | 7 | 4 | 0 | 31 | moderately resistant |
| | | 100 | 0 | 4 | 9 | 3 | 65 | poorly resistant |
| *Beta vulgaris* | BvHPPD-02 | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | BvHPPDm-F372A-02 | 0 | 16 | 0 | 0 | 0 | 0 | |
| | | 25 | 0 | 6 | 10 | 0 | 54 | poorly resistant |
| | | 100 | 0 | 0 | 4 | 12 | 92 | non-resistant |
| *Nicotiana tabacum* | NtHPPD-02 | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | NtHPPDm-F372A-02 | 0 | 16 | 0 | 0 | 0 | 0 | |
| | | 25 | 0 | 6 | 10 | 0 | 54 | poorly resistant |
| | | 100 | 0 | 0 | 0 | 16 | 100 | non-resistant |
| *Cucumis sativus* | CsHPPD-02 | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | CsHPPDm-F372A-02 | 0 | 16 | 0 | 0 | 0 | 0 | |
| | | 25 | 0 | 0 | 16 | 0 | 67 | poorly resistant |
| | | 100 | 0 | 0 | 0 | 16 | 100 | non-resistant |
| *Solanum tuberosum* | StHPPD-02 | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | StHPPDm-F372A-02 | 0 | 16 | 0 | 0 | 0 | 0 | |
| | | 25 | 4 | 12 | 0 | 0 | 25 | moderately resistant |
| | | 100 | 0 | 1 | 4 | 11 | 88 | non-resistant |
| *Solanum lycopersicum* | SlHPPD-02 | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | SlHPPDm-F372A-02 | 0 | 16 | 0 | 0 | 0 | 0 | |
| | | 25 | 1 | 1 | 14 | 0 | 60 | poorly resistant |
| | | 100 | 0 | 1 | 4 | 11 | 88 | non-resistant |
| *Arachis hypogaea* | AhHPPD-02 | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | AhHPPDm-F372A-02 | 0 | 16 | 0 | 0 | 0 | 0 | |
| | | 25 | 2 | 12 | 2 | 0 | 33 | moderately resistant |
| | | 100 | 0 | 0 | 10 | 6 | 79 | non-resistant |
| *Pseudomonas fluorescens* | PfHPPD-02 | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | PfHPPDm-F372A-02 | 0 | 16 | 0 | 0 | 0 | 0 | |
| | | 25 | 16 | 0 | 0 | 0 | 0 | highly resistant |
| | | 100 | 12 | 4 | 0 | 0 | 8 | highly resistant |
| *Cyanobacteria* | CyHPPD-02 | 0 | 16 | 0 | 0 | 0 | 0 | |
| | | 25 | 0 | 0 | 0 | 0 | 16 | non-resistant |
| | | 100 | 0 | 0 | 0 | 0 | 16 | non-resistant |
| | CyHPPDm-F372A-02 | 0 | 16 | 0 | 0 | 0 | 0 | |
| | | 25 | 1 | 14 | 1 | 0 | 33 | moderately resistant |
| | | 100 | 0 | 1 | 15 | 0 | 65 | poorly resistant |
| N1 | N1HPPD-02 | 0 | 16 | 0 | 0 | 0 | 0 | |
| | | 25 | 2 | 12 | 1 | 1 | 35 | poorly resistant |
| | | 100 | 0 | 4 | 8 | 4 | 67 | poorly resistant |
| | N1HPPDm-F372A-02 | 0 | 16 | 0 | 0 | 0 | 0 | |
| | | 25 | 16 | 0 | 0 | 0 | 0 | highly resistant |
| | | 100 | 16 | 0 | 0 | 0 | 0 | highly resistant |
| N2 | N2HPPD-02 | 0 | 16 | 0 | 0 | 0 | 0 | |
| | | 25 | 0 | 1 | 15 | 0 | 65 | poorly resistant |
| | | 100 | 0 | 0 | 0 | 16 | 100 | non-resistant |
| | N2HPPDm-F372A-02 | 0 | 16 | 0 | 0 | 0 | 0 | |
| | | 25 | 16 | 0 | 0 | 0 | 0 | highly resistant |
| | | 100 | 16 | 0 | 0 | 0 | 0 | highly resistant |
| N3 | N3HPPD-02 | 0 | 16 | 0 | 0 | 0 | 0 | |
| | | 25 | 0 | 6 | 8 | 2 | 58 | poorly resistant |
| | | 100 | 0 | 0 | 0 | 16 | 100 | non-resistant |
| | N3HPPDm-F372A-02 | 0 | 16 | 0 | 0 | 0 | 0 | |
| | | 25 | 16 | 0 | 0 | 0 | 0 | highly resistant |
| | | 100 | 12 | 4 | 0 | 0 | 8 | highly resistant |
| N4 | N4HPPD-02 | 0 | 16 | 0 | 0 | 0 | 0 | |
| | | 25 | 5 | 6 | 5 | 0 | 33 | moderately resistant |
| | | 100 | 0 | 8 | 2 | 6 | 63 | poorly resistant |
| | N4HPPDm-F372A-02 | 0 | 16 | 0 | 0 | 0 | 0 | |
| | | 25 | 16 | 0 | 0 | 0 | 0 | highly resistant |
| | | 100 | 12 | 4 | 0 | 0 | 8 | highly resistant |
| N5 | N5HPPD-02 | 0 | 16 | 0 | 0 | 0 | 0 | |
| | | 25 | 6 | 6 | 4 | 0 | 21 | moderately resistant |
| | | 100 | 0 | 7 | 5 | 4 | 60 | poorly resistant |
| | N5HPPDm-F372A-02 | 0 | 16 | 0 | 0 | 0 | 0 | |
| | | 25 | 16 | 0 | 0 | 0 | 0 | highly resistant |
| | | 100 | 13 | 3 | 0 | 0 | 6 | highly resistant |

The results of TABLE 2 and FIG. 4 show that compared with the *Arabidopsis thaliana* plants into which unmutated HPPD genes were introduced, all the *Arabidopsis thaliana* plants into which HPPD genes with a mutation at position 372 from different species were introduced had different degrees of tolerance to topramezone, and in particular the *Arabidopsis thaliana* plants into which HPPD genes with a mutation at position 372 from microorganisms had relatively superior tolerance to topramezone, while the wild-type *Arabidopsis thaliana* plants had no tolerance to topramezone. Thus, it can be seen that position 372 is the key position of HPPDs. Mutation of position 372 (F372A) of amino acid sequences of HPPDs from different species can impart tolerance to topramezone upon plants.

**Example 3: Mutation of position 372 of amino acid sequences of HPPDs from different ecotypes within the same species (F372A) and verification of the mutation effect**

In order to verify the effect of mutation of position 372 of the amino acid sequence of HPPDs from different ecotypes within the same species, position 372 of HPPDs from different ecotypes of *Triticum aestivum* (F372A) was mutated to verify the mutation effect thereof.

### 1. Acquisition of HPPDs from different ecotypes of Triticum aestivum and mutant HPPDs (F372A)

The amino acid sequence (471 amino acids) of the native HPPD of *Triticum aestivum* ecotype 1 with the UniProt Accession Number A0A3B5YS13 (TaHPPD1) is set forth as SEQ ID NO: 177 in the SEQUENCE LISTING; the TaHPPD1-01 nucleotide sequence (1416 nucleotides) encoding the TaHPPD1 is set forth as SEQ ID NO: 178 in the SEQUENCE LISTING, and the TaHPPD1-02 nucleotide sequence encoding the TaHPPD1, which was obtained based on the *Arabidopsis thaliana* codon usage bias, is set forth as SEQ ID NO: 179 in the SEQUENCE LISTING. Position 372 of the TaHPPD1 amino acid sequence was mutated from the original phenylalanine to alanine to obtain a mutant TaHPPD1 (TaHPPD1m-F372A) amino acid sequence as set forth in SEQ ID NO: 180 in the SEQUENCE LISTING. The mutant TaHPPD1-01 (TaHPPD1m-F372A-01) nucleotide sequence is set forth as SEQ ID NO: 181 in the SEQUENCE LISTING. The TaHPPD1m-F372A-02 nucleotide sequence encoding the TaHPPD1m-F372A, which was obtained based on the *Arabidopsis thaliana* codon usage bias, is set forth as SEQ ID NO: 182 in the SEQUENCE LISTING.

The amino acid sequence (436 amino acids) of the native HPPD of *Triticum aestivum* ecotype 2 with the UniProt Accession Number Q45FE8 (TaHPPD2) is set forth as SEQ ID NO: 183 in the SEQUENCE LISTING; the TaHPPD2-01 nucleotide sequence (1311 nucleotides) encoding the TaHPPD2 is set forth as SEQ ID NO: 184 in the SEQUENCE LISTING, and the TaHPPD2-02 nucleotide sequence encoding the TaHPPD2, which was obtained based on the *Arabidopsis thaliana* codon usage bias, is set forth as SEQ ID NO: 185 in the SEQUENCE LISTING. Position 372 of the TaHPPD2 amino acid sequence was mutated from the original phenylalanine to alanine to obtain a mutant TaHPPD2 (TaHPPD2m-F372A) amino acid sequence as set forth in SEQ ID NO: 186 in the SEQUENCE LISTING. The mutant TaHPPD2-01 (TaHPPD2m-F372A-01) nucleotide sequence is set forth as SEQ ID NO: 187 in the SEQUENCE LISTING. The TaHPPD2m-F372A-02 nucleotide sequence encoding the TaHPPD2m-F372A, which was obtained based on the *Arabidopsis thaliana* codon usage bias, is set forth as SEQ ID NO: 188 in the SEQUENCE LISTING.

The amino acid sequence (436 amino acids) of the native HPPD of *Triticum aestivum* ecotype 3 with the UniProt Accession Number A0A3B6PKV0 (TaHPPD3) is set forth as SEQ ID NO: 189 in the SEQUENCE LISTING; the TaHPPD3-01 nucleotide sequence (1311 nucleotides) encoding the TaHPPD3 is set forth as SEQ ID NO: 190 in the SEQUENCE LISTING, and the TaHPPD3-02 nucleotide sequence encoding the TaHPPD3, which was obtained based on the *Arabidopsis thaliana* codon usage bias, is set forth as SEQ ID NO: 191 in the SEQUENCE LISTING. Position 372 of the TaHPPD3 amino acid sequence was mutated from the original phenylalanine to alanine to obtain a mutant TaHPPD3 (TaHPPD3m-F372A) amino acid sequence as set forth in SEQ ID NO: 192 in the SEQUENCE LISTING. The mutant TaHPPD3-01 (TaHPPD3m-F372A-01) nucleotide sequence is set forth as SEQ ID NO: 193 in the SEQUENCE LISTING. The TaHPPD3m-F372A-02 nucleotide sequence encoding the TaHPPD3m-F372A, which was obtained based on the *Arabidopsis thaliana* codon usage bias, is set forth as SEQ ID NO: 194 in the SEQUENCE LISTING.

The amino acid sequence (433 amino acids) of the native HPPD of *Triticum aestivum* ecotype 4 with the UniProt Accession Number A0A3B6NLC6 (TaHPPD4) is set forth as SEQ ID NO: 195 in the SEQUENCE LISTING; the TaHPPD4-01 nucleotide sequence (1302 nucleotides) encoding the TaHPPD4 is set forth as SEQ ID NO: 196 in the SEQUENCE LISTING, and the TaHPPD4-02 nucleotide sequence encoding the TaHPPD4, which was obtained based on the *Arabidopsis thaliana* codon usage bias, is set forth as SEQ ID NO: 197 in the SEQUENCE LISTING. Position 372 of the TaHPPD4 amino acid sequence was mutated from the original phenylalanine to alanine to obtain a mutant TaHPPD4 (TaHPPD4m-F372A) amino acid sequence as set forth in SEQ ID NO: 198 in the SEQUENCE LISTING. The mutant TaHPPD4-01 (TaHPPD4m-F372A-01) nucleotide sequence is set forth as SEQ ID NO: 199 in the SEQUENCE LISTING. The TaHPPD4m-F372A-02 nucleotide sequence encoding the TaHPPD4m-F372A, which was obtained based on the *Arabidopsis thaliana* codon usage bias, is set forth as SEQ ID NO: 200 in the SEQUENCE LISTING.

The amino acid sequence (413 amino acids) of the native HPPD of *Triticum aestivum* ecotype 5 with the UniProt Accession Number A0A3B6NNK0 (TaHPPD5) is set forth as SEQ ID NO: 201 in the SEQUENCE LISTING; the TaHPPD5-01 nucleotide sequence (1242 nucleotides) encoding the TaHPPD5 is set forth as SEQ ID NO: 202 in the SEQUENCE LISTING, and the TaHPPD5-02 nucleotide sequence encoding the TaHPPD5, which was obtained based on the *Arabidopsis thaliana* codon usage bias, is set forth as SEQ ID NO: 203 in the SEQUENCE LISTING. Position 372 of the TaHPPD5 amino acid sequence was mutated from the original phenylalanine to alanine to obtain a mutant TaHPPD5 (TaHPPD5m-F372A) amino acid sequence as set forth in SEQ ID NO: 204 in the SEQUENCE LISTING. The mutant TaHPPD5-01 (TaHPPD5m-F372A-01) nucleotide sequence is set forth as SEQ ID NO: 205 in the SEQUENCE LISTING. The TaHPPD5m-F372A-02 nucleotide sequence encoding the TaHPPD5m-F372A, which was obtained based on the *Arabidopsis thaliana* codon usage bias, is set forth as SEQ ID NO: 206 in the SEQUENCE LISTING.

The amino acid sequence (403 amino acids) of the native HPPD of *Triticum aestivum* ecotype 6 with the UniProt Accession Number A0A3B5YPS5 (TaHPPD6) is set forth as SEQ ID NO: 207 in the SEQUENCE LISTING; the TaHPPD6-01 nucleotide sequence (1212 nucleotides) encoding the TaHPPD6 is set forth as SEQ ID NO: 208 in the SEQUENCE LISTING, and the TaHPPD6-02 nucleotide sequence encoding the TaHPPD6, which was obtained based on the *Arabidopsis thaliana* codon usage bias, is set forth as SEQ ID NO: 209 in the SEQUENCE LISTING. Position 372 of the TaHPPD6 amino acid sequence was mutated from the original phenylalanine to alanine to obtain a mutant TaHPPD6 (TaHPPD6m-F372A) amino acid sequence as set forth in SEQ ID NO: 210 in the SEQUENCE LISTING. The mutant TaHPPD6-01 (TaHPPD6m-F372A-01) nucleotide sequence is set forth as SEQ ID NO: 211 in the SEQUENCE LISTING. The TaHPPD6m-F372A-02 nucleotide sequence encoding the TaHPPD6m-F372A, which was obtained based on the *Arabidopsis thaliana* codon usage bias, is set forth as SEQ ID NO: 212 in the SEQUENCE LISTING.

The amino acid sequence (381 amino acids) of the native HPPD of *Triticum aestivum* ecotype 7 with the UniProt Accession Number A7WK82 (TaHPPD7) is set forth as SEQ ID NO: 213 in the SEQUENCE LISTING; the TaHPPD7-01 nucleotide sequence (1146 nucleotides) encoding the TaHPPD7 is set forth as SEQ ID NO: 214 in the SEQUENCE LISTING, and the TaHPPD7-02 nucleotide sequence encoding the TaHPPD7, which was obtained based on the *Arabidopsis thaliana* codon usage bias, is set forth as SEQ ID NO: 215 in the SEQUENCE LISTING. Position 372 of the TaHPPD7 amino acid sequence was mutated from the original phenylalanine to alanine to obtain a mutant TaHPPD7 (TaHPPD7m-F372A) amino acid sequence as set forth in SEQ ID NO: 216 in the SEQUENCE LISTING. The mutant TaHPPD7-01 (TaHPPD7m-F372A-01) nucleotide sequence is set forth as SEQ ID NO: 217 in the SEQUENCE LISTING. The TaHPPD7m-F372A-02 nucleotide sequence encoding the TaHPPD7m-F372A, which was obtained based on the *Arabidopsis thaliana* codon usage bias, is set forth as SEQ ID NO: 218 in the SEQUENCE LISTING.

### 2. Construction of recombinant expression vectors containing HPPDs from different ecotypes of Triticum aestivum (F372A) for Arabidopsis thaliana

According to the method of constructing the recombinant expression vector DBN11375 containing the AsHPPDm-F372A-02 nucleotide sequence as described above in point 3 of Example 1, the TaHPPD1m-F372A-02 nucleotide sequence, TaHPPD2m-F372A-02 nucleotide sequence, TaHPPD3m-F372A-02 nucleotide sequence, TaHPPD4m-F372A-02 nucleotide sequence, TaHPPD5m-F372A-02 nucleotide sequence, TaHPPD6m-F372A-02 nucleotide sequence and TaHPPD7m-F372A-02 nucleotide sequence which were linked to the universal adapter primer 1 was respectively subjected to a recombination reaction with the linearized DBNBC-01 expression vector backbone to obtain the recombinant expression vectors DBN11403 to DBN11409 in sequence. Sequencing verified that the aforementioned nucleotide sequences were inserted correctly in the recombinant expression vectors DBN11403 to DBN11409.

According to the method of constructing the recombinant expression vector DBN11375 containing the AsHPPDm-F372A-02 nucleotide sequence as described above in point 3 of Example 1, the TaHPPD1-02 nucleotide sequence, TaHPPD2-02 nucleotide sequence, TaHPPD3-02 sequence, TaHPPD4-02 nucleotide sequence, TaHPPD5-02 nucleotide sequence, TaHPPD6-02 nucleotide sequence and TaHPPD7-02 nucleotide sequence which were linked to the universal adapter primer 1 was respectively subjected to a recombination reaction with the linearized DBNBC-01 expression vector backbone to obtain the recombinant expression vectors DBN11403N to DBN11409N in sequence. Sequencing verified that the aforementioned nucleotide sequences were inserted correctly in the recombinant expression vectors DBN11403N to DBN11409N.

### 3. Transformation of Agrobacterium with the recombinant expression vectors for Arabidopsis thaliana

According to the method of transforming *Agrobacterium* with the recombinant expression vectors for *Arabidopsis thaliana* as described above in point 4 of Example 1, the recombinant expression vectors DBN11403 to DBN11409 and DBN11403N to DBN11409N which had been constructed correctly were transformed into *Agrobacterium* GV3101 respectively using a liquid nitrogen method. The results were verified by sequencing, showing that the structures of the recombinant expression vectors DBN11403 to DBN11409 and DBN11403N to DBN11409N were completely correct.

### 4. Detection of the herbicide tolerance of the Arabidopsis thaliana plants into which HPPDs from different ecotypes of Triticum aestivum (F372A) were introduced

According to the method as described above in point 5 of Example 1, *Arabidopsis thaliana* inflorescences were immersed in the *Agrobacterium* solution as described in Example 3 so as to introduce the T-DNA in the recombinant expression vectors DBN11403 to DBN11409 and DBN11403N to DBN11409N constructed in Example 2 into the *Arabidopsis thaliana* chromosomes, thereby obtaining the corresponding transgenic *Arabidopsis thaliana* plants, i.e., *Arabidopsis thaliana* T₁ plants into which the TaHPPD1m-F372A-02 nucleotide sequence was introduced, *Arabidopsis thaliana* T₁ plants into which the TaHPPD2m-F372A-02 nucleotide sequence was introduced, *Arabidopsis thaliana* T₁ plants into which the TaHPPD3m-F372A-02 nucleotide sequence was introduced, *Arabidopsis thaliana* T₁ plants into which the TaHPPD4m-F372A-02 nucleotide sequence was introduced, *Arabidopsis thaliana* T₁ plants into which the TaHPPD5m-F372A-02 nucleotide sequence was introduced, *Arabidopsis thaliana* T₁ plants into which the TaHPPD6m-F372A-02 nucleotide sequence was introduced, *Arabidopsis thaliana* T₁ plants into which the TaHPPD7m-F372A-02 nucleotide sequence was introduced, and *Arabidopsis thaliana* T₁ plants into which the TaHPPD1-02 nucleotide sequence was introduced, *Arabidopsis thaliana* T₁ plants into which the TaHPPD2-02 nucleotide sequence was introduced, *Arabidopsis thaliana* T₁ plants into which the TaHPPD3-02 nucleotide sequence was introduced, *Arabidopsis thaliana* T₁ plants into which the TaHPPD4-02 nucleotide sequence was introduced, *Arabidopsis thaliana* T₁ plants into which the TaHPPD5-02 nucleotide sequence was introduced, *Arabidopsis thaliana* T₁ plants into which the TaHPPD6-02 nucleotide sequence was introduced and *Arabidopsis thaliana* T₁ plants into which the TaHPPD7-02 nucleotide sequence was introduced.

According to the method as described above in point 6 of Example 1, the aforementioned *Arabidopsis thaliana* T₁ plants and wild-type *Arabidopsis thaliana* plants (18 days after sowing) were sprayed with topramezone at three different concentrations respectively, i.e., 25 g ai/ha (one-fold field concentration, 1×), 100 g ai/ha (four-fold field concentration, 4×) and 0 g ai/ha (water, 0×) to detect the tolerance of *Arabidopsis thaliana* to the herbicide. The experimental results are shown in TABLE 3.

**TABLE 3 Topramezone tolerance of Arabidopsis thaliana T₁ plants into which HPPDs from different ecotypes of Triticum aestivum (F372A) were introduced**

| Source of the gene | *Arabidopsis thaliana* genotypes | Concentration ( g ai/ha ) | Classification and statistics of pesticide damage grade | | | | Scores | Resistance evaluation |
|---|---|---|---|---|---|---|---|---|
| | | | Grade 0 | Grade 1 | Grade 2 | Grade 3 | | |
| | Wild-type *Arabidopsis thaliana* | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| Ecotype 1 | TAHPPD1-02 | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | TaHPPD 1m-F372A-02 | 0 | 16 | 0 | 0 | 0 | 0 | |
| | | 25 | 16 | 0 | 0 | 0 | 0 | highly resistant |
| | | 100 | 15 | 1 | 0 | 0 | 2 | highly resistant |
| Ecotype 2 | TaHPPD2-02 | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | TaHPPD2m-F372A-02 | 0 | 16 | 0 | 0 | 0 | 0 | |
| | | 25 | 16 | 0 | 0 | 0 | 0 | highly resistant |
| | | 100 | 8 | 8 | 0 | 0 | 17 | moderately resistant |
| Ecotype 3 | TaHPPD3-02 | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | TaHPPD3m-F372A-02 | 0 | 16 | 0 | 0 | 0 | 0 | |
| | | 25 | 16 | 0 | 0 | 0 | 0 | highly resistant |
| | | 100 | 12 | 4 | 0 | 0 | 8 | highly resistant |
| Ecotype 4 | TaHPPD4-02 | 0 | 16 | 0 | 0 | 0 | 0 | |
| | | 25 | 0 | 0 | 4 | 12 | 92 | non-resistant |
| | | 100 | 0 | 0 | 0 | 16 | 100 | non-resistant |
| | TaHPPD4m-F372A-02 | 0 | 16 | 0 | 0 | 0 | 0 | |
| | | 25 | 16 | 0 | 0 | 0 | 0 | highly resistant |
| | | 100 | 11 | 5 | 0 | 0 | 10 | highly resistant |
| Ecotype 5 | TaHPPD5-02 | 0 | 16 | 0 | 0 | 0 | 0 | |
| | | 25 | 0 | 0 | 8 | 8 | 83 | non-resistant |
| | | 100 | 0 | 0 | 2 | 14 | 96 | non-resistant |
| | TaHPPD5m-F372A-02 | 0 | 16 | 0 | 0 | 0 | 0 | |
| | | 25 | 16 | 0 | 0 | 0 | 0 | highly resistant |
| | | 100 | 6 | 9 | 1 | 0 | 23 | moderately resistant |
| | TaHPPD6-02 | 0 | 16 | 0 | 0 | 0 | 0 | |

| Grade | Grade 1 | Grade 2 | Grade 3 | | | | | |
|---|---|---|---|---|---|---|---|---|
| Ecotype 6 | | 25 | 0 | 0 | 6 | 10 | 88 | non-resistant |
| | | 100 | 0 | 0 | 2 | 14 | 96 | non-resistant |
| | TaHPPD6m-F372A-02 | 0 | 16 | 0 | 0 | 0 | 0 | |
| | | 25 | 16 | 0 | 0 | 0 | 0 | highly resistant |
| | | 100 | 15 | 1 | 0 | 0 | 2 | highly resistant |
| Ecotype 7 | TaHPPD7-02 | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | TaHPPD7m-F372A-02 | 0 | 16 | 0 | 0 | 0 | 0 | |
| | | 25 | 12 | 4 | 0 | 0 | 8 | highly resistant |
| | | 100 | 6 | 10 | 0 | 0 | 21 | moderately resistant |

The results of TABLE 3 show that all the *Arabidopsis thaliana* plants into which HPPD genes with a mutation at position 372 from different ecotypes of *Triticum aestivum* were introduced had good tolerance to topramezone (with high or moderate resistance) while the *Arabidopsis thaliana* plants into which unmutated HPPD genes were introduced and the wild-type *Arabidopsis thaliana* plants showed no tolerance to topramezone. This indicates that mutation of position 372 of HPPD amino acid sequences from different ecotypes within the same species (F372A) can impart tolerance to topramezone upone plants; i.e., the effects of mutation are identical for the HPPD amino acid sequences from different ecotypes within the same species.

### Example 4: Different forms of mutation at the 372 position of HPPD amino acid sequence from different species and verification of the mutation effects

In order to further verify the effects of different forms of the mutation at the 372 position of HPPD amino acid sequence, saturation mutations of all amino acids were performed at the 372 position of HPPD from the plant source (*Avena sativa*) and the microorganism source (*Pseudomonas fluorescens*), both of which showed better tolerance to topramezone.

### 1. Acquisition of saturation mutation of HPPD from Avena sativa and Pseudomonas fluorescens sources

The 372 position of the AsHPPD amino acid sequence was subjected to saturation mutation, that is, the original phenylalanine was mutated into other 18 amino acids respectively (except phenylalanine and alanine) deletion mutation, to obtain the AsHPPDm-F372G amino acid sequence as set forth in SEQ ID NO:219 in the SEQUENCE LISTING, the AsHPPDm-F372V amino acid sequence as set forth in SEQ ID NO:220 in the SEQUENCE LISTING, the AsHPPDm-F372L amino acid sequence as set forth in SEQ ID NO:221 in the SEQUENCE LISTING, the AsHPPDm-F372I amino acid sequence as set forth in SEQ ID NO:222 in the SEQUENCE LISTING, the AsHPPDm-F372P amino acid sequence as set forth in SEQ ID NO:223 in the SEQUENCE LISTING, the AsHPPDm-F372Y amino acid sequence as set forth in SEQ ID NO:224 in the SEQUENCE LISTING, the AsHPPDm-F372W amino acid sequence as set forth in SEQ ID NO:225 in the SEQUENCE LISTING, the AsHPPDm-F372S amino acid sequence as set forth in SEQ ID NO:226 in the SEQUENCE LISTING, the AsHPPDm-F372T amino acid sequence as set forth in SEQ ID NO:227 in the SEQUENCE LISTING, the AsHPPDm-F372C amino acid sequence as set forth in SEQ ID NO:228 in the SEQUENCE LISTING, the AsHPPDm-F372M amino acid sequence as set forth in SEQ ID NO:229 in the SEQUENCE LISTING, the AsHPPDm-F372N amino acid sequence as set forth in SEQ ID NO:230 in the SEQUENCE LISTING, the AsHPPDm-F372Q amino acid sequence as set forth in SEQ ID NO:231 in the SEQUENCE LISTING, the AsHPPDm-F372D amino acid sequence as set forth in SEQ ID NO:232 in the SEQUENCE LISTING, the AsHPPDm-F372E amino acid sequence as set forth in SEQ ID NO:233 in the SEQUENCE LISTING, the AsHPPDm-F372K amino acid sequence as set forth in SEQ ID NO:234 in the SEQUENCE LISTING, the AsHPPDm-F372R amino acid sequence as set forth in SEQ ID NO:235 in the SEQUENCE LISTING, the AsHPPDm-F372H amino acid sequence as set forth in SEQ ID NO:236 in the SEQUENCE LISTING, and the AsHPPDm-F372 amino acid sequence (deletion) as set forth in SEQ ID NO:237 in the SEQUENCE LISTING; the nucleotide sequences encoding the above amino acid sequences were obtained based on the *Arabidopsis thaliana*/soybean/rice common codon usage bias, that is, the AsHPPDm-F372G nucleotide sequence as set forth in SEQ ID NO:238 in the SEQUENCE LISTING, the AsHPPDm-F372V nucleotide sequence as set forth in SEQ ID NO:239 in the SEQUENCE LISTING, the AsHPPDm-F372L nucleotide sequence as set forth in SEQ ID NO:240 in the SEQUENCE LISTING, the AsHPPDm-F372I nucleotide sequence as set forth in SEQ ID NO:241 in the SEQUENCE LISTING, the AsHPPDm-F372P nucleotide sequence as set forth in SEQ ID NO:242 in the SEQUENCE LISTING, the AsHPPDm-F372Y nucleotide sequence as set forth in SEQ ID NO:243 in the SEQUENCE LISTING, the AsHPPDm-F372W nucleotide sequence as set forth in SEQ ID NO:244 in the SEQUENCE LISTING, the AsHPPDm-F372S nucleotide sequence as set forth in SEQ ID NO:245 in the SEQUENCE LISTING, the AsHPPDm-F372T nucleotide sequence as set forth in SEQ ID NO:246 in the SEQUENCE LISTING, the AsHPPDm-F372C nucleotide sequence as set forth in SEQ ID NO:247 in the SEQUENCE LISTING, the AsHPPDm-F372M nucleotide sequence as set forth in SEQ ID NO:248 in the SEQUENCE LISTING, the AsHPPDm-F372N nucleotide sequence as set forth in SEQ ID NO:249 in the SEQUENCE LISTING, the AsHPPDm-F372Q nucleotide sequence as set forth in SEQ ID NO:250 in the SEQUENCE LISTING, the AsHPPDm-F372D nucleotide sequence as set forth in SEQ ID NO:251 in the SEQUENCE LISTING, the AsHPPDm-F372E nucleotide sequence as set forth in SEQ ID NO:252 in the SEQUENCE LISTING, the AsHPPDm-F372K nucleotide sequence as set forth in SEQ ID NO:253 in the SEQUENCE LISTING, the AsHPPDm-F372R nucleotide sequence as set forth in SEQ ID NO:254 in the SEQUENCE LISTING, the AsHPPDm-F372H nucleotide sequence as set forth in SEQ ID NO:255 in the SEQUENCE LISTING, and the AsHPPDm-F372 nucleotide sequence (deletion) as set forth in SEQ ID NO:256 in the SEQUENCE LISTING.

The 372 position of the PfHPPD amino acid sequence was subjected to saturation mutation, that is, the original phenylalanine was mutated into other 18 amino acids respectively (except phenylalanine and alanine) and deletion mutation, to obtain the PfHPPDm-F372G amino acid sequence as set forth in SEQ ID NO:257 in the SEQUENCE LISTING, the PfHPPDm-F372V amino acid sequence as set forth in SEQ ID NO:258 in the SEQUENCE LISTING, the PfHPPDm-F372L amino acid sequence as set forth in SEQ ID NO:259 in the SEQUENCE LISTING, the PfHPPDm-F372I amino acid sequence as set forth in SEQ ID NO:260 in the SEQUENCE LISTING, the PfHPPDm-F372P amino acid sequence as set forth in SEQ ID NO:261 in the SEQUENCE LISTING, the PfHPPDm-F372Y amino acid sequence as set forth in SEQ ID NO:262 in the SEQUENCE LISTING, the PfHPPDm-F372W amino acid sequence as set forth in SEQ ID NO:263 in the SEQUENCE LISTING, the PfHPPDm-F372S amino acid sequence as set forth in SEQ ID NO:264 in the SEQUENCE LISTING, the PfHPPDm-F372T amino acid sequence as set forth in SEQ ID NO:265 in the SEQUENCE LISTING, the PfHPPDm-F372C amino acid sequence as set forth in SEQ ID NO:266 in the SEQUENCE LISTING, the PfHPPDm-F372M amino acid sequence as set forth in SEQ ID NO:267 in the SEQUENCE LISTING, the PfHPPDm-F372N amino acid sequence as set forth in SEQ ID NO:268 in the SEQUENCE LISTING, the PfHPPDm-F372Q amino acid sequence as set forth in SEQ ID NO:269 in the SEQUENCE LISTING, the PfHPPDm-F372D amino acid sequence as set forth in SEQ ID NO:270 in the SEQUENCE LISTING, the PfHPPDm-F372E amino acid sequence as set forth in SEQ ID NO:271 in the SEQUENCE LISTING, the PfHPPDm-F372K amino acid sequence as set forth in SEQ ID NO:272 in the SEQUENCE LISTING, the PfHPPDm-F372R amino acid sequence as set forth in SEQ ID NO:273 in the SEQUENCE LISTING, the PfHPPDm-F372H amino acid sequence as set forth in SEQ ID NO:274 in the SEQUENCE LISTING, and the PfHPPDm-F372 amino acid sequence (deletion) as set forth in SEQ ID NO:275 in the SEQUENCE LISTING; the nucleotide sequences encoding the above amino acid sequences were obtained based on the *Arabidopsis thaliana*/soybean/rice common usage bias, that is, the PfHPPDm-F372G nucleotide sequence as set forth in SEQ ID NO:276 in the SEQUENCE LISTING, the PfHPPDm-F372V nucleotide sequence as set forth in SEQ ID NO:277 in the SEQUENCE LISTING, the PfHPPDm-F372L nucleotide sequence as set forth in SEQ ID NO:278 in the SEQUENCE LISTING, the PfHPPDm-F372I nucleotide sequence as set forth in SEQ ID NO:279 in the SEQUENCE LISTING, the PfHPPDm-F372P nucleotide sequence as set forth in SEQ ID NO:280 in the SEQUENCE LISTING, the PfHPPDm-F372Y nucleotide sequence as set forth in SEQ ID NO:281 in the SEQUENCE LISTING, the PfHPPDm-F372W nucleotide sequence as set forth in SEQ ID NO:282 in the SEQUENCE LISTING, the PfHPPDm-F372S nucleotide sequence as set forth in SEQ ID NO:283 in the SEQUENCE LISTING, the PfHPPDm-F372T nucleotide sequence as set forth in SEQ ID NO:284 in the SEQUENCE LISTING, the PfHPPDm-F372C nucleotide sequence as set forth in SEQ ID NO:285 in the SEQUENCE LISTING, the PfHPPDm-F372M nucleotide sequence as set forth in SEQ ID NO:286 in the SEQUENCE LISTING, the PfHPPDm-F372N nucleotide sequence as set forth in SEQ ID NO:287 in the SEQUENCE LISTING, the PfHPPDm-F372Q nucleotide sequence as set forth in SEQ ID NO:288 in the SEQUENCE LISTING, the PfHPPDm-F372D nucleotide sequence as set forth in SEQ ID NO:289 in the SEQUENCE LISTING, the PfHPPDm-F372E nucleotide sequence as set forth in SEQ ID NO:290 in the SEQUENCE LISTING, the PfHPPDm-F372K nucleotide sequence as set forth in SEQ ID NO:291 in the SEQUENCE LISTING, the PfHPPDm-F372R nucleotide sequence as set forth in SEQ ID NO:292 in the SEQUENCE LISTING, the PfHPPDm-F372H nucleotide sequence as set forth in SEQ ID NO:293 in the SEQUENCE LISTING, and the PfHPPDm-F372 nucleotide sequence (deletion) as set forth in SEQ ID NO:294 in the SEQUENCE LISTING.

### 2. Construction of recombinant expression vectors containing saturation mutation of HPPD for Arabidopsis thaliana

According to the method of constructing the recombinant expression vector DBN11375 containing AsHPPDm-F372A-02 nucleotide sequence as described in point 3 of Example 1, the AsHPPDm-F372G nucleotide sequence, AsHPPDm-F372V nucleotide sequence, AsHPPDm-F372L nucleotide sequence, AsHPPDm-F372I nucleotide sequence, AsHPPDm-F372P nucleotide sequence, AsHPPDm-F372Y nucleotide sequence, AsHPPDm-F372W nucleotide sequence, AsHPPDm-F372S nucleotide sequence, AsHPPDm-F372T nucleotide sequence, AsHPPDm-F372C nucleotide sequence, AsHPPDm-F372M nucleotide sequence, AsHPPDm-F372N nucleotide sequence, AsHPPDm-F372Q nucleotide sequence, AsHPPDm-F372D nucleotide sequence, AsHPPDm-F372E nucleotide sequence, AsHPPDm-F372K nucleotide sequence, AsHPPDm-F372R nucleotide sequence, AsHPPDm-F372H nucleotide sequence, AsHPPDm-F372 nucleotide sequence, and the PfHPPDm-F372G nucleotide sequence, PfHPPDm-F372V nucleotide sequence, PfHPPDm-F372L nucleotide sequence, PfHPPDm-F372I nucleotide sequence, PfHPPDm-F372P nucleotide sequence, PfHPPDm-F372Y nucleotide sequence, PfHPPDm-F372W nucleotide sequence, PfHPPDm-F372S nucleotide sequence, PfHPPDm-F372T nucleotide sequence, PfHPPDm-F372C nucleotide sequence, PfHPPDm-F372M nucleotide sequence, PfHPPDm-F372N nucleotide sequence, PfHPPDm-F372Q nucleotide sequence, PfHPPDm-F372D nucleotide sequence, PfHPPDm-F372E nucleotide sequence, PfHPPDm-F372K nucleotide sequence, PfHPPDm-F372R nucleotide sequence, PfHPPDm-F372H nucleotide sequence, and PfHPPDm-F372 nucleotide sequence which were linked with the universal adapter primer 1 were respectively subjected to a recombination reaction with the linearized DBNBC-01 expression vector skeleton to obtain the recombinant expression vectors DBN11410 to DBN11447 in sequence. Sequencing verified that the above nucleotide sequences were inserted correctly in the recombinant expression vectors DBN11410 to DBN11447.

### 3. Transformation of Agrobacterium with the recombinant expression vectors for Arabidopsis thaliana

According to the method of transformation of *Agrobacterium* with the recombinant expression vectors for *Arabidopsis thaliana* as described above in point 4 of Example 1, the recombinant expression vectors DBN11410 to DBN11447 which had been correctly constructed were transformed into the *Agrobacterium* GV3101 respectively using a liquid nitrogen method, and the results were verified by sequencing, showing that the structures of the recombinant expression vectors DBN11410 to DBN11447 were completely correct.

### 4. Detection of the herbicide tolerance of the Arabidopsis thaliana plants into which saturation mutation of HPPD was introduced

According to the method as described above in point 5 of Example 1, *Arabidopsis thaliana* inflorescences were immersed in the *Agrobacterium* solution as described in Example 3 so as to introduce the T-DNA of the recombinant expression vectors DBN11410 to DBN11447 constructed in Example 2 into the *Arabidopsis thaliana* chromosomes, thereby obtaining the corresponding transgenic *Arabidopsis thaliana* plants, that is, the *Arabidopsis thaliana* T₁ plants into which the AsHPPDm-F372G nucleotide sequence was introduced, *Arabidopsis thaliana* T₁ plants into which the AsHPPDm-F372V nucleotide sequence was introduced, *Arabidopsis thaliana* T₁ plants into which the AsHPPDm-F372L nucleotide sequence was introduced, *Arabidopsis thaliana* T₁ plants into which the AsHPPDm-F372I nucleotide sequence was introduced, *Arabidopsis thaliana* T₁ plants into which the AsHPPDm-F372P nucleotide sequence was introduced, *Arabidopsis thaliana* T₁ plants into which the AsHPPDm-F372Y nucleotide sequence was introduced, *Arabidopsis thaliana* T₁ plants into which the AsHPPDm-F372W nucleotide sequence was introduced, *Arabidopsis thaliana* T₁ plants into which the AsHPPDm-F372S nucleotide sequence was introduced, *Arabidopsis thaliana* T₁ plants into which the AsHPPDm-F372T nucleotide sequence was introduced, *Arabidopsis thaliana* T₁ plants into which the AsHPPDm-F372C nucleotide sequence was introduced, *Arabidopsis thaliana* T₁ plants into which the AsHPPDm-F372M nucleotide sequence was introduced, *Arabidopsis thaliana* T₁ plants into which the AsHPPDm-F372N nucleotide sequence was introduced, *Arabidopsis thaliana* T₁ plants into which the AsHPPDm-F372Q nucleotide sequence was introduced, *Arabidopsis thaliana* T₁ plants into which the AsHPPDm-F372D nucleotide sequence was introduced, *Arabidopsis thaliana* T₁ plants into which the AsHPPDm-F372E nucleotide sequence was introduced, *Arabidopsis thaliana* T₁ plants into which the AsHPPDm-F372K nucleotide sequence was introduced, *Arabidopsis thaliana* T₁ plants into which the AsHPPDm-F372R nucleotide sequence was introduced, *Arabidopsis thaliana* T₁ plants into which the AsHPPDm-F372H nucleotide sequence was introduced, *Arabidopsis thaliana* T₁ plants into which the AsHPPDm-F372 nucleotide sequence was introduced, and *Arabidopsis thaliana* T₁ plants into which the PfHPPDm-F372G nucleotide sequence was introduced, *Arabidopsis thaliana* T₁ plants into which the PfHPPDm-F372V nucleotide sequence was introduced, *Arabidopsis thaliana* T₁ plants into which the PfHPPDm-F372L nucleotide sequence was introduced, *Arabidopsis thaliana* T₁ plants into which the PfHPPDm-F372I nucleotide sequence was introduced, *Arabidopsis thaliana* T₁ plants into which the PfHPPDm-F372P nucleotide sequence was introduced, *Arabidopsis thaliana* T₁ plants into which the PfHPPDm-F372Y nucleotide sequence was introduced, *Arabidopsis thaliana* T₁ plants into which the PfHPPDm-F372W nucleotide sequence was introduced, *Arabidopsis thaliana* T₁ plants into which the PfHPPDm-F372S nucleotide sequence was introduced, *Arabidopsis thaliana* T₁ plants into which the PfHPPDm-F372T nucleotide sequence was introduced, *Arabidopsis thaliana* T₁ plants into which the PfHPPDm-F372C nucleotide sequence was introduced, *Arabidopsis thaliana* T₁ plants into which the PfHPPDm-F372M nucleotide sequence was introduced, *Arabidopsis thaliana* T₁ plants into which the PfHPPDm-F372N nucleotide sequence was introduced, *Arabidopsis thaliana* T₁ plants into which the PfHPPDm-F372Q nucleotide sequence was introduced, *Arabidopsis thaliana* T₁ plants into which the PfHPPDm-F372D nucleotide sequence was introduced, *Arabidopsis thaliana* T₁ plants into which the PfHPPDm-F372E nucleotide sequence was introduced, *Arabidopsis thaliana* T₁ plants into which the PfHPPDm-F372K nucleotide sequence was introduced, *Arabidopsis thaliana* T₁ plants into which the PfHPPDm-F372R nucleotide sequence was introduced, *Arabidopsis thaliana* T₁ plants into which the PfHPPDm-F372H nucleotide sequence was introduced, and *Arabidopsis thaliana* T₁ plants into which the PfHPPDm-F372 nucleotide sequence was introduced.

According to the method as described above in point 6 of Example 1, the above-mentioned *Arabidopsis thaliana* T₁ plants, *Arabidopsis thaliana* T₁ plants into which the AsHPPD-02 nucleotide sequence was introduced, *Arabidopsis thaliana* T₁ plants into which the PfHPPD-02 nucleotide sequence was introduced, and wild-type *Arabidopsis thaliana* plants (18 days after sowing) were sprayed with topramezone at four different concentrations respectively, that is, 25 g ai/ha (one-fold field concentration, 1×), 50 g ai/ha (two-fold field concentration, 2×), 100 g ai/ha (four-fold field concentration, 4×) and 0 g ai/ha (water, 0×) to detect the herbicide tolerance of *Arabidopsis thaliana.* The experimental results are shown in TABLEs 4 and 5.

**TABLE 4 Topramezone tolerance of Arabidopsis thaliana T₁ plants into which saturation mutation of HPPD from Avena sativa source was introduced**

| | Statistical data after evaluation of gene from *Avena sativa* source | | | | | | | |
|---|---|---|---|---|---|---|---|---|
| Mutation type of HPPD from *Avena sativa* source | | Treatment concentration ( g ai/ha ) | Grade 0 | Grade 1 | Grade 2 | Grade 3 | score | Resistance Grade |
| Wild-type *Arabidopsis thaliana* | | 0 | 20 | 0 | 0 | 0 | 0 | |
| | | 25 | 20 | 0 | 0 | 0 | 0 | Non-resistant |
| | | 50 | 20 | 0 | 0 | 0 | 0 | Non-resistant |
| | | 100 | 20 | 0 | 0 | 0 | 0 | Non-resistant |
| AsHPPD-02 | | 0 | 20 | 0 | 0 | 0 | 0 | |
| | | 25 | 2 | 2 | 16 | 0 | 57 | Poorly resistant |
| | | 50 | 0 | 2 | 4 | 14 | 87 | Non-resistant |
| | | 100 | 0 | 0 | 4 | 16 | 93 | Non-resistant |
| F372G | | 0 | 20 | 0 | 0 | 0 | 0 | |
| | | 25 | 20 | 0 | 0 | 0 | 0 | Highly resistant |
| | | 50 | 20 | 0 | 0 | 0 | 0 | Highly resistant |
| | | 100 | 20 | 0 | 0 | 0 | 0 | Highly resistant |
| F372V | | 0 | 20 | 0 | 0 | 0 | 0 | |
| | | 25 | 20 | 0 | 0 | 0 | 0 | Highly resistant |
| | | 50 | 20 | 0 | 0 | 0 | 0 | Highly resistant |
| | | 100 | 20 | 0 | 0 | 0 | 0 | Highly resistant |
| F372L | | 0 | 20 | 0 | 0 | 0 | 0 | |
| | | 25 | 20 | 0 | 0 | 0 | 0 | Highly resistant |
| | | 50 | 20 | 0 | 0 | 0 | 0 | Highly resistant |
| | | 100 | 6 | 10 | 4 | 0 | 30 | Moderately resistant |
| F372I | | 0 | 20 | 0 | 0 | 0 | 0 | |
| | | 25 | 20 | 0 | 0 | 0 | 0 | Highly resistant |
| | | 50 | 20 | 0 | 0 | 0 | 0 | Highly resistant |
| | | 100 | 20 | 0 | 0 | 0 | 0 | Highly resistant |
| F372P | | 0 | 20 | 0 | 0 | 0 | 0 | |
| | | 25 | 20 | 0 | 0 | 0 | 0 | Highly resistant |
| | | 50 | 20 | 0 | 0 | 0 | 0 | Highly resistant |
| | | 100 | 4 | 12 | 4 | 0 | 33 | Moderately resistant |
| F372Y | | 0 | 20 | 0 | 0 | 0 | 0 | |
| | | 25 | 0 | 0 | 0 | 20 | 100 | Non-resistant |
| | | 50 | 0 | 4 | 0 | 16 | 87 | Non-resistant |
| | | 100 | 0 | 0 | 0 | 20 | 100 | Non-resistant |
| F372W | | 0 | 20 | 0 | 0 | 0 | 0 | |
| | | 25 | 6 | 6 | 8 | 0 | 37 | Poorly resistant |
| | | 50 | 0 | 0 | 0 | 20 | 100 | Non-resistant |
| | | 100 | 0 | 0 | 0 | 20 | 100 | Non-resistant |
| F372S | | 0 | 20 | 0 | 0 | 0 | 0 | |
| | | 25 | 20 | 0 | 0 | 0 | 0 | Highly resistant |
| | | 50 | 20 | 0 | 0 | 0 | 0 | Highly resistant |
| | | 100 | 20 | 0 | 0 | 0 | 0 | Highly resistant |
| F372T | | 0 | 20 | 0 | 0 | 0 | 0 | |
| | | 25 | 20 | 0 | 0 | 0 | 0 | Highly resistant |
| | | 50 | 18 | 2 | 0 | 0 | 3 | Highly resistant |
| | | 100 | 10 | 10 | 0 | 0 | 17 | Moderately resistant |
| F372C | | 0 | 20 | 0 | 0 | 0 | 0 | |
| | | 25 | 20 | 0 | 0 | 0 | 0 | Highly resistant |
| | | 50 | 18 | 2 | 0 | 0 | 3 | Highly resistant |
| | | 100 | 8 | 8 | 4 | 0 | 27 | Moderately resistant |
| F372M | | 0 | 20 | 0 | 0 | 0 | 0 | |
| | | 25 | 20 | 0 | 0 | 0 | 0 | Highly resistant |
| | | 50 | 8 | 12 | 0 | 0 | 20 | Moderately resistant |
| | | 100 | 2 | 6 | 12 | 0 | 50 | Poorly resistant |
| F372N | | 0 | 20 | 0 | 0 | 0 | 0 | |
| | | 25 | 18 | 0 | 2 | 0 | 7 | Highly resistant |
| | | 50 | 20 | 0 | 0 | 0 | 0 | Highly resistant |
| | | 100 | 20 | 0 | 0 | 0 | 0 | Highly resistant |
| F372Q | | 0 | 20 | 0 | 0 | 0 | 0 | |
| | | 25 | 20 | 0 | 0 | 0 | 0 | Highly resistant |
| | | 50 | 20 | 0 | 0 | 0 | 0 | Highly resistant |
| | | 100 | 20 | 0 | 0 | 0 | 0 | Highly resistant |
| F372D | | 0 | 20 | 0 | 0 | 0 | 0 | |
| | | 25 | 16 | 4 | 0 | 0 | 7 | Highly resistant |
| | | 50 | 20 | 0 | 0 | 0 | 0 | Highly resistant |
| | | 100 | 20 | 0 | 0 | 0 | 0 | Highly resistant |
| F372E | | 0 | 20 | 0 | 0 | 0 | 0 | |
| | | 25 | 0 | 8 | 8 | 4 | 60 | Poorly resistant |
| | | 50 | 0 | 6 | 10 | 4 | 63 | Poorly resistant |
| | | 100 | 0 | 0 | 2 | 18 | 97 | Non-resistant |
| F372K | | 0 | 20 | 0 | 0 | 0 | 0 | |
| | | 25 | 0 | 20 | 0 | 0 | 33 | Moderately resistant |
| | | 50 | 2 | 8 | 10 | 0 | 47 | Poorly resistant |
| | | 100 | 0 | 0 | 6 | 14 | 90 | Non-resistant |
| F372R | | 0 | 20 | 0 | 0 | 0 | 0 | |
| | | 25 | 0 | 0 | 0 | 20 | 100 | Non-resistant |
| | | 50 | 0 | 0 | 0 | 20 | 100 | Non-resistant |
| | | 100 | 0 | 0 | 0 | 20 | 100 | Non-resistant |
| F372H | | 0 | 20 | 0 | 0 | 0 | 0 | |
| | | 25 | 0 | 6 | 2 | 12 | 77 | Non-resistant |
| | | 50 | 0 | 2 | 10 | 8 | 77 | Non-resistant |
| | | 100 | 0 | 0 | 0 | 20 | 100 | Non-resistant |
| F372 deletion | | 0 | 20 | 0 | 0 | 0 | 0 | |
| | | 25 | 20 | 0 | 0 | 0 | 0 | Highly resistant |
| | | 50 | 20 | 0 | 0 | 0 | 0 | Highly resistant |
| | | 100 | 18 | 2 | 0 | 0 | 3 | Highly resistant |

**TABLE 5 Topramezone tolerance of Arabidopsis thaliana T₁ plants into which saturation mutation of HPPD from Pseudomonas fluorescens source was introduced**

| Statistical data after evaluation of gene from *Pseudomonas fluorescens* source | | | | | | | |
|---|---|---|---|---|---|---|---|
| Mutation type of HPPD from *Pseudomonas fluorescens* source | Treatment concentration ( g ai/ha ) | Grade 0 | Grade 1 | Grade 2 | Grade 3 | score | Resistance Grade |
| PfHPPD-02 | 0 | 20 | 0 | 0 | 0 | 0 | |
| | 25 | 0 | 0 | 0 | 20 | 100 | Non-resistant |
| | 50 | 0 | 0 | 0 | 20 | 100 | Non-resistant |
| | 100 | 0 | 0 | 0 | 20 | 100 | Non-resistant |
| F372G | 0 | 20 | 0 | 0 | 0 | 0 | |
| | 25 | 4 | 14 | 2 | 0 | 30 | Moderately resistant |
| | 50 | 0 | 18 | 2 | 0 | 37 | Poorly resistant |
| | 100 | 0 | 12 | 8 | 0 | 47 | Poorly resistant |
| F372V | 0 | 20 | 0 | 0 | 0 | 0 | |
| | 25 | 2 | 10 | 8 | 0 | 43 | Poorly resistant |
| | 50 | 0 | 14 | 6 | 0 | 43 | Poorly resistant |
| | 100 | 0 | 4 | 12 | 4 | 67 | Poorly resistant |
| F372L | 0 | 20 | 0 | 0 | 0 | 0 | |
| | 25 | 0 | 0 | 0 | 20 | 100 | Non-resistant |
| | 50 | 0 | 0 | 0 | 20 | 100 | Non-resistant |
| | 100 | 0 | 0 | 0 | 20 | 100 | Non-resistant |
| F372I | 0 | 20 | 0 | 0 | 0 | 0 | |
| | 25 | 0 | 6 | 6 | 8 | 70 | Non-resistant |
| | 50 | 0 | 0 | 2 | 18 | 97 | Non-resistant |
| | 100 | 0 | 0 | 0 | 20 | 100 | Non-resistant |
| F372P | 0 | 20 | 0 | 0 | 0 | 0 | |
| | 25 | 10 | 8 | 2 | 0 | 20 | Moderately resistant |
| | 50 | 4 | 8 | 4 | 4 | 47 | Poorly resistant |
| | 100 | 0 | 0 | 5 | 15 | 92 | Non-resistant |
| F372Y | 0 | 20 | 0 | 0 | 0 | 0 | |
| | 25 | 0 | 0 | 0 | 20 | 100 | Non-resistant |
| | 50 | 0 | 0 | 0 | 20 | 100 | Non-resistant |
| | 100 | 0 | 0 | 0 | 20 | 100 | Non-resistant |
| F372W | 0 | 20 | 0 | 0 | 0 | 0 | |
| | 25 | 0 | 0 | 2 | 18 | 97 | Non-resistant |
| | 50 | 0 | 0 | 0 | 20 | 100 | Non-resistant |
| | 100 | 0 | 0 | 0 | 20 | 100 | Non-resistant |
| F372S | 0 | 20 | 0 | 0 | 0 | 0 | |
| | 25 | 6 | 10 | 4 | 0 | 30 | Moderately resistant |
| | 50 | 0 | 8 | 8 | 4 | 60 | Poorly resistant |
| | 100 | 0 | 0 | 2 | 18 | 97 | Non-resistant |
| F372T | 0 | 20 | 0 | 0 | 0 | 0 | |
| | 25 | 8 | 6 | 2 | 4 | 37 | Poorly resistant |
| | 50 | 0 | 8 | 4 | 8 | 67 | Poorly resistant |
| | 100 | 0 | 0 | 2 | 18 | 97 | Non-resistant |
| F372C | 0 | 20 | 0 | 0 | 0 | 0 | |
| | 25 | 0 | 12 | 6 | 2 | 50 | Poorly resistant |
| | 50 | 2 | 4 | 4 | 10 | 70 | Non-resistant |
| | 100 | 0 | 0 | 0 | 20 | 100 | Non-resistant |
| F372M | 0 | 20 | 0 | 0 | 0 | 0 | |
| | 25 | 4 | 6 | 4 | 6 | 53 | Poorly resistant |
| | 50 | 2 | 0 | 4 | 14 | 83 | Non-resistant |
| | 100 | 0 | 0 | 0 | 20 | 100 | Non-resistant |
| F372N | 0 | 20 | 0 | 0 | 0 | 0 | |
| | 25 | 0 | 2 | 6 | 12 | 83 | Non-resistant |
| | 50 | 0 | 0 | 0 | 20 | 100 | Non-resistant |
| | 100 | 0 | 0 | 0 | 20 | 100 | Non-resistant |
| F372Q | 0 | 20 | 0 | 0 | 0 | 0 | |
| | 25 | 2 | 4 | 10 | 4 | 60 | Poorly resistant |
| | 50 | 0 | 4 | 6 | 10 | 77 | Non-resistant |
| | 100 | 0 | 0 | 0 | 20 | 100 | Non-resistant |
| F372D | 0 | 20 | 0 | 0 | 0 | 0 | |
| | 25 | 0 | 8 | 4 | 8 | 67 | Poorly resistant |
| | 50 | 0 | 0 | 8 | 12 | 87 | Non-resistant |
| | 100 | 0 | 0 | 0 | 20 | 100 | Non-resistant |
| F372E | 0 | 20 | 0 | 0 | 0 | 0 | |
| | 25 | 0 | 0 | 4 | 16 | 93 | Non-resistant |
| | 50 | 0 | 0 | 0 | 20 | 100 | Non-resistant |
| | 100 | 0 | 0 | 0 | 20 | 100 | Non-resistant |
| F372K | 0 | 20 | 0 | 0 | 0 | 0 | |
| | 25 | 0 | 0 | 0 | 20 | 100 | Non-resistant |
| | 50 | 0 | 0 | 0 | 20 | 100 | Non-resistant |
| | 100 | 0 | 0 | 0 | 20 | 100 | Non-resistant |
| F372R | 0 | 20 | 0 | 0 | 0 | 0 | |
| | 25 | 0 | 0 | 0 | 20 | 100 | Non-resistant |
| | 50 | 0 | 0 | 0 | 20 | 100 | Non-resistant |
| | 100 | 0 | 0 | 0 | 20 | 100 | Non-resistant |
| F372H | 0 | 20 | 0 | 0 | 0 | 0 | |
| | 25 | 0 | 0 | 0 | 20 | 100 | Non-resistant |
| | 50 | 0 | 0 | 0 | 20 | 100 | Non-resistant |
| | 100 | 0 | 0 | 0 | 20 | 100 | Non-resistant |
| F372 deletion | 0 | 20 | 0 | 0 | 0 | 0 | |
| | 25 | 0 | 10 | 10 | 0 | 50 | Poorly resistant |
| | 50 | 0 | 6 | 11 | 3 | 62 | Poorly resistant |
| | 100 | 0 | 2 | 10 | 8 | 77 | Non-resistant |

The results in TABLEs 4 and 5 showed that: (1) after saturation mutation of HPPD from *Avena sativa* source, except that the *Arabidopsis thaliana* plants with mutated forms of F372Y, F372R and F372H had no tolerance to topramezone, the *Arabidopsis thaliana* plants with other mutated forms had different degrees of tolerance to topramezone, and especially those with mutated forms of F372G, F372V, F372I, F372N, F372Q, F372D, F372(deletion) had better tolerance to topramezone, while the wild-type *Arabidopsis thaliana* plants and the *Arabidopsis thaliana* plants into which unmutated HPPD gene (AsHPPD-02) was introduced had no or low tolerance to various concentrations of topramezone; therefore, the mutation at 372 position of HPPD amino acid sequence from *Avena sativa* source into F372A, F372G, F372V, F372L, F372I, F372P, F372W, F372S, F372T, F372C, F372M, F372N, F372Q, F372D, F372E, F372K and F372 (deletion) can confer tolerance to topramezone upon plants; (2) after saturation mutation of HPPD from *Pseudomonas fluorescens* source, the *Arabidopsis thaliana* plants with mutated forms of F372G, F372V, F372P, F372S, F372T, F372C, F372M, F372Q, F372D and F372 (deletion) had different degrees of tolerance to topramezone, the *Arabidopsis thaliana* plants with other mutated forms had no tolerance to topramezone, while the *Arabidopsis thaliana* plants into which unmutated HPPD gene (PfHPPD-02) was introduced and the wild-type *Arabidopsis thaliana* plants had no tolerance to topramezone; therefore, the mutation at 372 position of HPPD amino acid sequence from *Pseudomonas fluorescens* source into F372A, F372G, F372V, F372P, F372S, F372T, F372C, F372M, F372Q, F372D and F372 (deletion) can confer tolerance to topramezone upon plants. The results also showed that the mutation at 372 position of HPPD amino acid sequence from *Avena sativa* source and *Pseudomonas fluorescens* source into F372G and F372V can confer better tolerance to topramezone upon plants.

### Example 5: Mutation at 372 position of HPPD amino acid sequence from different species sources (F372G and F372V) and verification of the mutation effects

In order to further verify the effects of the F372G or F372V mutation at 372 position of HPPD amino acid sequence from different species sources, mutation (F372G or F372V) was performed at the 372 position of HPPD amino acid sequence from representative species sources selected in the Example 2.

### 1. Acquisition of mutant HPPD (F372G and F372V) from different species sources

The 372 position of the ZmHPPD amino acid sequence was mutated from the original phenylalanine to glycine, to obtain the mutated ZmHPPD (ZmHPPDm-F372G) amino acid sequence as set forth in SEQ ID NO:295 in the SEQUENCE LISTING, and the ZmHPPDm-F372G nucleotide sequence encoding the ZmHPPDm-F372G, which was obtained based on the *Arabidopsis thaliana* codon usage bias, is set forth as SEQ ID NO:296 in the SEQUENCE LISTING.

The 372 position of the AtHPPD amino acid sequence was mutated from the original phenylalanine to glycine, to obtain the mutated AtHPPD (AtHPPDm-F372G) amino acid sequence as set forth in SEQ ID NO:297 in the SEQUENCE LISTING, and the AtHPPDm-F372G nucleotide sequence encoding the AtHPPDm-F372G was set forth as SEQ ID NO:298 in the SEQUENCE LISTING.

The 372 position of the GsHPPD amino acid sequence was mutated from the original phenylalanine to glycine, to obtain the mutated GsHPPD (GsHPPDm-F372G) amino acid sequence as set forth in SEQ ID NO:299 in the SEQUENCE LISTING, and the GsHPPDm-F372G nucleotide sequence encoding the GsHPPDm-F372G, which was obtained based on the *Arabidopsis thaliana* codon usage bias, is set forth as SEQ ID NO:300 in the SEQUENCE LISTING.

The 372 position of the TaHPPD amino acid sequence was mutated from the original phenylalanine to glycine, to obtain the mutated TaHPPD (TaHPPDm-F372G) amino acid sequence as set forth in SEQ ID NO:301 in the SEQUENCE LISTING, and the TaHPPDm-F372G nucleotide sequence encoding the TaHPPDm-F372G, which was obtained based on the *Arabidopsis thaliana* codon usage bias, is set forth as SEQ ID NO:302 in the SEQUENCE LISTING.

The 372 position of the BdHPPD amino acid sequence was mutated from the original phenylalanine to glycine, to obtain the mutated BdHPPD (BdHPPDm-F372G) amino acid sequence as set forth in SEQ ID NO:303 in the SEQUENCE LISTING, and the BdHPPDm-F372G nucleotide sequence encoding the BdHPPDm-F372G, which was obtained based on the *Arabidopsis thaliana* codon usage bias, is set forth as SEQ ID NO:304 in the SEQUENCE LISTING.

The 372 position of the HvHPPD amino acid sequence was mutated from the original phenylalanine to glycine, to obtain the mutated HvHPPD (HvHPPDm-F372G) amino acid sequence as set forth in SEQ ID NO:305 in the SEQUENCE LISTING, and the HvHPPDm-F372G nucleotide sequence encoding the HvHPPDm-F372G, which was obtained based on the *Arabidopsis thaliana* codon usage bias, is set forth as SEQ ID NO:306 in the SEQUENCE LISTING.

The 372 position of the SiHPPD amino acid sequence was mutated from the original phenylalanine to glycine, to obtain the mutated SiHPPD (SiHPPDm-F372G) amino acid sequence as set forth in SEQ ID NO:307 in the SEQUENCE LISTING, and the SiHPPDm-F372G nucleotide sequence encoding the SiHPPDm-F372G, which was obtained based on the *Arabidopsis thaliana* codon usage bias, is set forth as SEQ ID NO:308 in the SEQUENCE LISTING.

The 372 position of the SbHPPD amino acid sequence was mutated from the original phenylalanine to glycine, to obtain the mutated SbHPPD (SbHPPDm-F372G) amino acid sequence as set forth in SEQ ID NO:309 in the SEQUENCE LISTING, and the SbHPPDm-F372G nucleotide sequence encoding the SbHPPDm-F372G, which was obtained based on the *Arabidopsis thaliana* codon usage bias, is set forth as SEQ ID NO:310 in the SEQUENCE LISTING.

The 372 position of the OsHPPD amino acid sequence was mutated from the original phenylalanine to glycine, to obtain the mutated OsHPPD (OsHPPDm-F372G) amino acid sequence as set forth in SEQ ID NO:311 in the SEQUENCE LISTING, and the OsHPPDm-F372G nucleotide sequence encoding the OsHPPDm-F372G, which was obtained based on the *Arabidopsis thaliana* codon usage bias, is set forth as SEQ ID NO:312 in the SEQUENCE LISTING.

The 372 position of the GmHPPD amino acid sequence was mutated from the original phenylalanine to glycine, to obtain the mutated GmHPPD (GmHPPDm-F372G) amino acid sequence as set forth in SEQ ID NO:313 in the SEQUENCE LISTING, and the GmHPPDm-F372G nucleotide sequence encoding the GmHPPDm-F372G, which was obtained based on the *Arabidopsis thaliana* codon usage bias, is set forth as SEQ ID NO:314 in the SEQUENCE LISTING.

The 372 position of the CaHPPD amino acid sequence was mutated from the original phenylalanine to glycine, to obtain the mutated CaHPPD (CaHPPDm-F372G) amino acid sequence as set forth in SEQ ID NO:315 in the SEQUENCE LISTING, and the CaHPPDm-F372G nucleotide sequence encoding the CaHPPDm-F372G, which was obtained based on the *Arabidopsis thaliana* codon usage bias, is set forth as SEQ ID NO:316 in the SEQUENCE LISTING.

The 372 position of the BnHPPD amino acid sequence was mutated from the original phenylalanine to glycine, to obtain the mutated BnHPPD (BnHPPDm-F372G) amino acid sequence as set forth in SEQ ID NO:317 in the SEQUENCE LISTING, and the BnHPPDm-F372G nucleotide sequence encoding the BnHPPDm-F372G, which was obtained based on the *Arabidopsis thaliana* codon usage bias, is set forth as SEQ ID NO:318 in the SEQUENCE LISTING.

The 372 position of the HaHPPD amino acid sequence was mutated from the original phenylalanine to glycine, to obtain the mutated HaHPPD (HaHPPDm-F372G) amino acid sequence as set forth in SEQ ID NO:319 in the SEQUENCE LISTING, and the HaHPPDm-F372G nucleotide sequence encoding the HaHPPDm-F372G, which was obtained based on the *Arabidopsis thaliana* codon usage bias, is set forth as SEQ ID NO:320 in the SEQUENCE LISTING.

The 372 position of the MsHPPD amino acid sequence was mutated from the original phenylalanine to glycine, to obtain the mutated MsHPPD (MsHPPDm-F372G) amino acid sequence as set forth in SEQ ID NO:321 in the SEQUENCE LISTING, and the MsHPPDm-F372G nucleotide sequence encoding the MsHPPDm-F372G, which was obtained based on the *Arabidopsis thaliana* codon usage bias, is set forth as SEQ ID NO:322 in the SEQUENCE LISTING.

The 372 position of the BvHPPD amino acid sequence was mutated from the original phenylalanine to glycine, to obtain the mutated BvHPPD (BvHPPDm-F372G) amino acid sequence as set forth in SEQ ID NO:323 in the SEQUENCE LISTING, and the BvHPPDm-F372G nucleotide sequence encoding the BvHPPDm-F372G, which was obtained based on the *Arabidopsis thaliana* codon usage bias, is set forth as SEQ ID NO:324 in the SEQUENCE LISTING.

The 372 position of the NtHPPD amino acid sequence was mutated from the original phenylalanine to glycine, to obtain the mutated NtHPPD (NtHPPDm-F372G) amino acid sequence as set forth in SEQ ID NO:325 in the SEQUENCE LISTING, and the NtHPPDm-F372G nucleotide sequence encoding the NtHPPDm-F372G, which was obtained based on the *Arabidopsis thaliana* codon usage bias, is set forth as SEQ ID NO:326 in the SEQUENCE LISTING.

The 372 position of the CsHPPD amino acid sequence was mutated from the original phenylalanine to glycine, to obtain the mutated CsHPPD (CsHPPDm-F372G) amino acid sequence as set forth in SEQ ID NO:327 in the SEQUENCE LISTING, and the CsHPPDm-F372G nucleotide sequence encoding the CsHPPDm-F372G, which was obtained based on the *Arabidopsis thaliana* codon usage bias, is set forth as SEQ ID NO:328 in the SEQUENCE LISTING.

The 372 position of the StHPPD amino acid sequence was mutated from the original phenylalanine to glycine, to obtain the mutated StHPPD (StHPPDm-F372G) amino acid sequence as set forth in SEQ ID NO:329 in the SEQUENCE LISTING, and the StHPPDm-F372G nucleotide sequence encoding the StHPPDm-F372G, which was obtained based on the *Arabidopsis thaliana* codon usage bias, is set forth as SEQ ID NO:330 in the SEQUENCE LISTING.

The 372 position of the SlHPPD amino acid sequence was mutated from the original phenylalanine to glycine, to obtain the mutated SlHPPD (SlHPPDm-F372G) amino acid sequence as set forth in SEQ ID NO:331 in the SEQUENCE LISTING, and the SlHPPDm-F372G nucleotide sequence encoding the SlHPPDm-F372G, which was obtained based on the *Arabidopsis thaliana* codon usage bias, is set forth as SEQ ID NO:332 in the SEQUENCE LISTING.

The 372 position of the AhHPPD amino acid sequence was mutated from the original phenylalanine to glycine, to obtain the mutated AhHPPD (AhHPPDm-F372G) amino acid sequence as set forth in SEQ ID NO:333 in the SEQUENCE LISTING, and the AhHPPDm-F372G nucleotide sequence encoding the AhHPPDm-F372G, which was obtained based on the *Arabidopsis thaliana* codon usage bias, is set forth as SEQ ID NO:334 in the SEQUENCE LISTING.

The 372 position of the CyHPPD amino acid sequence was mutated from the original phenylalanine to glycine, to obtain the mutated CyHPPD (CyHPPDm-F372G) amino acid sequence as set forth in SEQ ID NO:335 in the SEQUENCE LISTING, and the CyHPPDm-F372G nucleotide sequence encoding the CyHPPDm-F372G, which was obtained based on the *Arabidopsis thaliana* codon usage bias, is set forth as SEQ ID NO:336 in the SEQUENCE LISTING.

The 372 position of the N1HPPD amino acid sequence was mutated from the original phenylalanine to glycine, to obtain the mutated N1HPPD (N1HPPDm-F372G) amino acid sequence as set forth in SEQ ID NO:337 in the SEQUENCE LISTING, and the NlHPPDm-F372G nucleotide sequence encoding the N1HPPDm-F372G, which was obtained based on the *Arabidopsis thaliana* codon usage bias, is set forth as SEQ ID NO:338 in the SEQUENCE LISTING.

The 372 position of the N2HPPD amino acid sequence was mutated from the original phenylalanine to glycine, to obtain the mutated N2HPPD (N2HPPDm-F372G) amino acid sequence as set forth in SEQ ID NO:339 in the SEQUENCE LISTING, and the N2HPPDm-F372G nucleotide sequence encoding the N2HPPDm-F372G, which was obtained based on the *Arabidopsis thaliana* codon usage bias, is set forth as SEQ ID NO:340 in the SEQUENCE LISTING.

The 372 position of the N3HPPD amino acid sequence was mutated from the original phenylalanine to glycine, to obtain the mutated N3HPPD (N3HPPDm-F372G) amino acid sequence as set forth in SEQ ID NO:341 in the SEQUENCE LISTING, and the N3HPPDm-F372G nucleotide sequence encoding the N3HPPDm-F372G, which was obtained based on the *Arabidopsis thaliana* codon usage bias, is set forth as SEQ ID NO:342 in the SEQUENCE LISTING.

The 372 position of the N4HPPD amino acid sequence was mutated from the original phenylalanine to glycine, to obtain the mutated N4HPPD (N4HPPDm-F372G) amino acid sequence as set forth in SEQ ID NO:343 in the SEQUENCE LISTING, and the N4HPPDm-F372G nucleotide sequence encoding the N4HPPDm-F372G, which was obtained based on the *Arabidopsis thaliana* codon usage bias, is set forth as SEQ ID NO:344 in the SEQUENCE LISTING.

The 372 position of the N5HPPD amino acid sequence was mutated from the original phenylalanine to glycine, to obtain the mutated N5HPPD (N5HPPDm-F372G) amino acid sequence as set forth in SEQ ID NO:345 in the SEQUENCE LISTING, and the N5HPPDm-F372G nucleotide sequence encoding the N5HPPDm-F372G, which was obtained based on the *Arabidopsis thaliana* codon usage bias, is set forth as SEQ ID NO:346 in the SEQUENCE LISTING.

The 372 position of the ZmHPPD amino acid sequence was mutated from the original phenylalanine to valine, to obtain the mutated ZmHPPD (ZmHPPDm-F372V) amino acid sequence as set forth in SEQ ID NO:347 in the SEQUENCE LISTING, and the ZmHPPDm-F372V nucleotide sequence encoding the ZmHPPDm-F372V, which was obtained based on the *Arabidopsis thaliana* codon usage bias, is set forth as SEQ ID NO:348 in the SEQUENCE LISTING.

The 372 position of the AtHPPD amino acid sequence was mutated from the original phenylalanine to valine, to obtain the mutated AtHPPD (AtHPPDm-F372V) amino acid sequence as set forth in SEQ ID NO:349 in the SEQUENCE LISTING, and the AtHPPDm-F372V nucleotide sequence encoding the AtHPPDm-F372V is set forth as SEQ ID NO:350 in the SEQUENCE LISTING.

The 372 position of the GsHPPD amino acid sequence was mutated from the original phenylalanine to valine, to obtain the mutated GsHPPD (GsHPPDm-F372V) amino acid sequence as set forth in SEQ ID NO:351 in the SEQUENCE LISTING, and the GsHPPDm-F372V nucleotide sequence encoding the GsHPPDm-F372V, which was obtained based on the *Arabidopsis thaliana* codon usage bias, is set forth as SEQ ID NO:352 in the SEQUENCE LISTING.

The 372 position of the TaHPPD amino acid sequence was mutated from the original phenylalanine to valine, to obtain the mutated TaHPPD (TaHPPDm-F372V) amino acid sequence as set forth in SEQ ID NO:353 in the SEQUENCE LISTING, and the TaHPPDm-F372V nucleotide sequence encoding the TaHPPDm-F372V, which was obtained based on the *Arabidopsis thaliana* codon usage bias, is set forth as SEQ ID NO:354 in the SEQUENCE LISTING.

The 372 position of the BdHPPD amino acid sequence was mutated from the original phenylalanine to valine, to obtain the mutated BdHPPD (BdHPPDm-F372V) amino acid sequence as set forth in SEQ ID NO:355 in the SEQUENCE LISTING, and the BdHPPDm-F372V nucleotide sequence encoding the BdHPPDm-F372V, which was obtained based on the *Arabidopsis thaliana* codon usage bias, is set forth as SEQ ID NO:356 in the SEQUENCE LISTING.

The 372 position of the HvHPPD amino acid sequence was mutated from the original phenylalanine to valine, to obtain the mutated HvHPPD (HvHPPDm-F372V) amino acid sequence as set forth in SEQ ID NO:357 in the SEQUENCE LISTING, and the HvHPPDm-F372V nucleotide sequence encoding the HvHPPDm-F372V, which was obtained based on the *Arabidopsis thaliana* codon usage bias, is set forth as SEQ ID NO:358 in the SEQUENCE LISTING.

The 372 position of the SiHPPD amino acid sequence was mutated from the original phenylalanine to valine, to obtain the mutated SiHPPD (SiHPPDm-F372V) amino acid sequence as set forth in SEQ ID NO:359 in the SEQUENCE LISTING, and the SiHPPDm-F372V nucleotide sequence encoding the SiHPPDm-F372V, which was obtained based on the *Arabidopsis thaliana* codon usage bias, is set forth as SEQ ID NO:360 in the SEQUENCE LISTING.

The 372 position of the SbHPPD amino acid sequence was mutated from the original phenylalanine to valine, to obtain the mutated SbHPPD (SbHPPDm-F372V) amino acid sequence as set forth in SEQ ID NO:361 in the SEQUENCE LISTING, and the SbHPPDm-F372V nucleotide sequence encoding the SbHPPDm-F372V, which was obtained based on the *Arabidopsis thaliana* codon usage bias, is set forth as SEQ ID NO:362 in the SEQUENCE LISTING.

The 372 position of the OsHPPD amino acid sequence was mutated from the original phenylalanine to valine, to obtain the mutated OsHPPD (OsHPPDm-F372V) amino acid sequence as set forth in SEQ ID NO:363 in the SEQUENCE LISTING, and the OsHPPDm-F372V nucleotide sequence encoding the OsHPPDm-F372V, which was obtained based on the *Arabidopsis thaliana* codon usage bias, is set forth as SEQ ID NO:364 in the SEQUENCE LISTING.

The 372 position of the GmHPPD amino acid sequence was mutated from the original phenylalanine to valine, to obtain the mutated GmHPPD (GmHPPDm-F372V) amino acid sequence as set forth in SEQ ID NO:365 in the SEQUENCE LISTING, and the GmHPPDm-F372V nucleotide sequence encoding the GmHPPDm-F372V, which was obtained based on the *Arabidopsis thaliana* codon usage bias, is set forth as SEQ ID NO:366 in the SEQUENCE LISTING.

The 372 position of the CaHPPD amino acid sequence was mutated from the original phenylalanine to valine, to obtain the mutated CaHPPD (CaHPPDm-F372V) amino acid sequence as set forth in SEQ ID NO:367 in the SEQUENCE LISTING, and the CaHPPDm-F372V nucleotide sequence encoding the CaHPPDm-F372V, which was obtained based on the *Arabidopsis thaliana* codon usage bias, is set forth as SEQ ID NO:368 in the SEQUENCE LISTING.

The 372 position of the BnHPPD amino acid sequence was mutated from the original phenylalanine to valine, to obtain the mutated BnHPPD (BnHPPDm-F372V) amino acid sequence as set forth in SEQ ID NO:369 in the SEQUENCE LISTING, and the BnHPPDm-F372V nucleotide sequence encoding the BnHPPDm-F372V, which was obtained based on the *Arabidopsis thaliana* codon usage bias, is set forth as SEQ ID NO:370 in the SEQUENCE LISTING.

The 372 position of the HaHPPD amino acid sequence was mutated from the original phenylalanine to valine, to obtain the mutated HaHPPD (HaHPPDm-F372V) amino acid sequence as set forth in SEQ ID NO:371 in the SEQUENCE LISTING, and the HaHPPDm-F372V nucleotide sequence encoding the HaHPPDm-F372V, which was obtained based on the *Arabidopsis thaliana* codon usage bias, is set forth as SEQ ID NO:372 in the SEQUENCE LISTING.

The 372 position of the MsHPPD amino acid sequence was mutated from the original phenylalanine to valine, to obtain the mutated MsHPPD (MsHPPDm-F372V) amino acid sequence as set forth in SEQ ID NO:373 in the SEQUENCE LISTING, and the MsHPPDm-F372V nucleotide sequence encoding the MsHPPDm-F372V, which was obtained based on the *Arabidopsis thaliana* codon usage bias, is set forth as SEQ ID NO:374 in the SEQUENCE LISTING.

The 372 position of the BvHPPD amino acid sequence was mutated from the original phenylalanine to valine, to obtain the mutated BvHPPD (BvHPPDm-F372V) amino acid sequence as set forth in SEQ ID NO:375 in the SEQUENCE LISTING, and the BvHPPDm-F372V nucleotide sequence encoding the BvHPPDm-F372V, which was obtained based on the *Arabidopsis thaliana* codon usage bias, is set forth as SEQ ID NO:376 in the SEQUENCE LISTING.

The 372 position of the NtHPPD amino acid sequence was mutated from the original phenylalanine to valine, to obtain the mutated NtHPPD (NtHPPDm-F372V) amino acid sequence as set forth in SEQ ID NO:377 in the SEQUENCE LISTING, and the NtHPPDm-F372V nucleotide sequence encoding the NtHPPDm-F372V, which was obtained based on the *Arabidopsis thaliana* codon usage bias, is set forth as SEQ ID NO:378 in the SEQUENCE LISTING.

The 372 position of the CsHPPD amino acid sequence was mutated from the original phenylalanine to valine, to obtain the mutated CsHPPD (CsHPPDm-F372V) amino acid sequence as set forth in SEQ ID NO:379 in the SEQUENCE LISTING, and the CsHPPDm-F372V nucleotide sequence encoding the CsHPPDm-F372V, which was obtained based on the *Arabidopsis thaliana* codon usage bias, is set forth as SEQ ID NO:380 in the SEQUENCE LISTING.

The 372 position of the StHPPD amino acid sequence was mutated from the original phenylalanine to valine, to obtain the mutated StHPPD (StHPPDm-F372V) amino acid sequence as set forth in SEQ ID NO:381 in the SEQUENCE LISTING, and the StHPPDm-F372V nucleotide sequence encoding the StHPPDm-F372V, which was obtained based on the *Arabidopsis thaliana* codon usage bias, is set forth as SEQ ID NO:382 in the SEQUENCE LISTING.

The 372 position of the SlHPPD amino acid sequence was mutated from the original phenylalanine to valine, to obtain the mutated SlHPPD (SlHPPDm-F372V) amino acid sequence as set forth in SEQ ID NO:383 in the SEQUENCE LISTING, and the SlHPPDm-F372V nucleotide sequence encoding the SlHPPDm-F372V, which was obtained based on the *Arabidopsis thaliana* codon usage bias, is set forth as SEQ ID NO:384 in the SEQUENCE LISTING.

The 372 position of the AhHPPD amino acid sequence was mutated from the original phenylalanine to valine, to obtain the mutated AhHPPD (AhHPPDm-F372V) amino acid sequence as set forth in SEQ ID NO:385 in the SEQUENCE LISTING, and the AhHPPDm-F372V nucleotide sequence encoding the AhHPPDm-F372V, which was obtained based on the *Arabidopsis thaliana* codon usage bias, is set forth as SEQ ID NO:386 in the SEQUENCE LISTING.

The 372 position of the CyHPPD amino acid sequence was mutated from the original phenylalanine to valine, to obtain the mutated CyHPPD (CyHPPDm-F372V) amino acid sequence as set forth in SEQ ID NO:387 in the SEQUENCE LISTING, and the CyHPPDm-F372V nucleotide sequence encoding the CyHPPDm-F372V, which was obtained based on the *Arabidopsis thaliana* codon usage bias, is set forth as SEQ ID NO:388 in the SEQUENCE LISTING.

The 372 position of the N1HPPD amino acid sequence was mutated from the original phenylalanine to valine, to obtain the mutated N1HPPD (N1HPPDm-F372V) amino acid sequence as set forth in SEQ ID NO:389 in the SEQUENCE LISTING, and the NlHPPDm-F372V nucleotide sequence encoding the N1HPPDm-F372V, which was obtained based on the *Arabidopsis thaliana* codon usage bias, is set forth as SEQ ID NO:390 in the SEQUENCE LISTING.

The 372 position of the N2HPPD amino acid sequence was mutated from the original phenylalanine to valine, to obtain the mutated N2HPPD (N2HPPDm-F372V) amino acid sequence as set forth in SEQ ID NO:391 in the SEQUENCE LISTING, and the N2HPPDm-F372V nucleotide sequence encoding the N2HPPDm-F372V, which was obtained based on the *Arabidopsis thaliana* codon usage bias, is set forth as SEQ ID NO:392 in the SEQUENCE LISTING.

The 372 position of the N3HPPD amino acid sequence was mutated from the original phenylalanine to valine, to obtain the mutated N3HPPD (N3HPPDm-F372V) amino acid sequence as set forth in SEQ ID NO:393 in the SEQUENCE LISTING, and the N3HPPDm-F372V nucleotide sequence encoding the N3HPPDm-F372V, which was obtained based on the *Arabidopsis thaliana* codon usage bias, is set forth as SEQ ID NO:394 in the SEQUENCE LISTING.

The 372 position of the N4HPPD amino acid sequence was mutated from the original phenylalanine to valine, to obtain the mutated N4HPPD (N4HPPDm-F372V) amino acid sequence as set forth in SEQ ID NO:395 in the SEQUENCE LISTING, and the N4HPPDm-F372V nucleotide sequence encoding the N4HPPDm-F372V, which was obtained based on the *Arabidopsis thaliana* codon usage bias, is set forth as SEQ ID NO:396 in the SEQUENCE LISTING.

The 372 position of the N5HPPD amino acid sequence was mutated from the original phenylalanine to valine, to obtain the mutated N5HPPD (N5HPPDm-F372V) amino acid sequence as set forth in SEQ ID NO:397 in the SEQUENCE LISTING, and the N5HPPDm-F372V nucleotide sequence encoding the N5HPPDm-F372V, which was obtained based on the *Arabidopsis thaliana* codon usage bias, is set forth as SEQ ID NO:398 in the SEQUENCE LISTING.

### 2. Construction of the recombinant expression vectors containing mutated HPPD (F372G and F372V) from different species sources for Arabidopsis thaliana

According to the method of constructing the recombinant expression vector DBN11375 containing AsHPPDm-F372A-02 nucleotide sequence as described in point 3 of Example 1, the ZmHPPDm-F372G nucleotide sequence, AtHPPDm-F372G nucleotide sequence, GsHPPDm-F372G nucleotide sequence, TaHPPDm-F372G nucleotide sequence, BdHPPDm-F372G nucleotide sequence, HvHPPDm-F372G nucleotide sequence, SiHPPDm-F372G nucleotide sequence, SbHPPDm-F372G nucleotide sequence, OsHPPDm-F372G nucleotide sequence, GmHPPDm-F372G nucleotide sequence, CaHPPDm-F372G nucleotide sequence, BnHPPDm-F372G nucleotide sequence, HaHPPDm-F372G nucleotide sequence, MsHPPDm-F372G nucleotide sequence, BvHPPDm-F372G nucleotide sequence, NtHPPDm-F372G nucleotide sequence, CsHPPDm-F372G nucleotide sequence, StHPPDm-F372G nucleotide sequence, SlHPPDm-F372G nucleotide sequence, AhHPPDm-F372G nucleotide sequence, CyHPPDm-F372G nucleotide sequence, N1HPPDm-F372G nucleotide sequence, N2HPPDm-F372G nucleotide sequence, N3HPPDm-F372G nucleotide sequence, N4HPPDm-F372G nucleotide sequence and N5HPPDm-F372G nucleotide sequence which were linked with the universal adapter primer 1 were respectively subjected to a recombination reaction with the linearized DBNBC-01 expression vector skeleton to obtain the recombinant expression vectors DBN11448 to DBN11473 in sequence. Sequencing verified that the above nucleotide sequences were correctly inserted in the recombinant expression vectors DBN11448 to DBN11473.

According to the method of constructing the recombinant expression vector DBN11375 containing AsHPPDm-F372A-02 nucleotide sequence as described in point 3 of Example 1, the ZmHPPDm-F372V nucleotide sequence, AtHPPDm-F372V nucleotide sequence, GsHPPDm-F372V nucleotide sequence, TaHPPDm-F372V nucleotide sequence, BdHPPDm-F372V nucleotide sequence, HvHPPDm-F372V nucleotide sequence, SiHPPDm-F372V nucleotide sequence, SbHPPDm-F372V nucleotide sequence, OsHPPDm-F372V nucleotide sequence, GmHPPDm-F372V nucleotide sequence, CaHPPDm-F372V nucleotide sequence, BnHPPDm-F372V nucleotide sequence, HaHPPDm-F372V nucleotide sequence, MsHPPDm-F372V nucleotide sequence, BvHPPDm-F372V nucleotide sequence, NtHPPDm-F372V nucleotide sequence, CsHPPDm-F372V nucleotide sequence, StHPPDm-F372V nucleotide sequence, SlHPPDm-F372V nucleotide sequence, AhHPPDm-F372V nucleotide sequence, CyHPPDm-F372V nucleotide sequence, N1HPPDm-F372V nucleotide sequence, N2HPPDm-F372V nucleotide sequence, N3HPPDm-F372V nucleotide sequence, N4HPPDm-F372V nucleotide sequence and N5HPPDm-F372V nucleotide sequence which were linked with the universal adapter primer 1 were respectively subjected to a recombination reaction with the linearized DBNBC-01 expression vector skeleton to obtain the recombinant expression vectors DBN11474 to DBN11499 in sequence. Sequencing verified that the above nucleotide sequences were correctly inserted in the recombinant expression vectors DBN11474 to DBN11499.

### 3. Transformation of Agrobacterium with the recombinant expression vectors for Arabidopsis thaliana

According to the method of transformation of *Agrobacterium* with the recombinant expression vectors for *Arabidopsis thaliana* as described in point 4 of Example 1, the recombinant expression vectors DBN11448 to DBN11499 which had been correctly constructed were transformed into the *Agrobacterium* GV3101 respectively using a liquid nitrogen method, and the results were verified by sequencing, showing that the structures of the recombinant expression vectors DBN11448 to DBN11499 were completely correct.

### 4. Detection of the herbicide tolerance effects of Arabidopsis thaliana plants into which mutated HPPD (F372G and F372V) from different species sources was introduced

According to the method as described in point 5 of Example 1, the *Arabidopsis thaliana* inflorescences were immersed in the *Agrobacterium* solution as described in Example 3, so as to introduce the T-DNA of the recombinant expression vectors DBN11448 to DBN11499 constructed in Example 2 into the *Arabidopsis thaliana* chromosome, thereby obtaining the corresponding transgenic *Arabidopsis thaliana* plants, that is, the *Arabidopsis thaliana* T₁ plants into which the ZmHPPDm-F372G nucleotide sequence was introduced, *Arabidopsis thaliana* T₁ plants into which the AtHPPDm-F372G nucleotide sequence was introduced, *Arabidopsis thaliana* T₁ plants into which the GsHPPDm-F372G nucleotide sequence was introduced, *Arabidopsis thaliana* T₁ plants into which the TaHPPDm-F372G nucleotide sequence was introduced, *Arabidopsis thaliana* T₁ plants into which the BdHPPDm-F372G nucleotide sequence was introduced, *Arabidopsis thaliana* T₁ plants into which the HvHPPDm-F372G nucleotide sequence was introduced, *Arabidopsis thaliana* T₁ plants into which the SiHPPDm-F372G nucleotide sequence was introduced, *Arabidopsis thaliana* T₁ plants into which the SbHPPDm-F372G nucleotide sequence was introduced, *Arabidopsis thaliana* T₁ plants into which the OsHPPDm-F372G nucleotide sequence was introduced, *Arabidopsis thaliana* T₁ plants into which the GmHPPDm-F372G nucleotide sequence was introduced, *Arabidopsis thaliana* T₁ plants into which the CaHPPDm-F372G nucleotide sequence was introduced, *Arabidopsis thaliana* T₁ plants into which the BnHPPDm-F372G nucleotide sequence was introduced, *Arabidopsis thaliana* T₁ plants into which the HaHPPDm-F372G nucleotide sequence was introduced, *Arabidopsis thaliana* T₁ plants into which the MsHPPDm-F372G nucleotide sequence was introduced, *Arabidopsis thaliana* T₁ plants into which the BvHPPDm-F372G nucleotide sequence was introduced, *Arabidopsis thaliana* T₁ plants into which the NtHPPDm-F372G nucleotide sequence was introduced, *Arabidopsis thaliana* T₁ plants into which the CsHPPDm-F372G nucleotide sequence was introduced, *Arabidopsis thaliana* T₁ plants into which the StHPPDm-F372G nucleotide sequence was introduced, *Arabidopsis thaliana* T₁ plants into which the SlHPPDm-F372G nucleotide sequence was introduced, *Arabidopsis thaliana* T₁ plants into which the AhHPPDm-F372G nucleotide sequence was introduced, *Arabidopsis thaliana* T₁ plants into which the CyHPPDm-F372G nucleotide sequence was introduced, *Arabidopsis thaliana* T₁ plants into which the N1HPPDm-F372G nucleotide sequence was introduced, *Arabidopsis thaliana* T₁ plants into which the N2HPPDm-F372G nucleotide sequence was introduced, *Arabidopsis thaliana* T₁ plants into which the N3HPPDm-F372G nucleotide sequence was introduced, *Arabidopsis thaliana* T₁ plants into which the N4HPPDm-F372G nucleotide sequence was introduced, *Arabidopsis thaliana* T₁ plants into which the N5HPPDm-F372G nucleotide sequence was introduced, and *Arabidopsis thaliana* T₁ plants into which the ZmHPPDm-F372V nucleotide sequence was introduced, *Arabidopsis thaliana* T₁ plants into which the AtHPPDm-F372V nucleotide sequence was introduced, *Arabidopsis thaliana* T₁ plants into which the GsHPPDm-F372V nucleotide sequence was introduced, *Arabidopsis thaliana* T₁ plants into which the TaHPPDm-F372V nucleotide sequence was introduced, *Arabidopsis thaliana* T₁ plants into which the BdHPPDm-F372V nucleotide sequence was introduced, *Arabidopsis thaliana* T₁ plants into which the HvHPPDm-F372V nucleotide sequence was introduced, *Arabidopsis thaliana* T₁ plants into which the SiHPPDm-F372V nucleotide sequence was introduced, *Arabidopsis thaliana* T₁ plants into which the SbHPPDm-F372V nucleotide sequence was introduced, *Arabidopsis thaliana* T₁ plants into which the OsHPPDm-F372V nucleotide sequence was introduced, *Arabidopsis thaliana* T₁ plants into which the GmHPPDm-F372V nucleotide sequence was introduced, *Arabidopsis thaliana* T₁ plants into which the CaHPPDm-F372V nucleotide sequence was introduced, *Arabidopsis thaliana* T₁ plants into which the BnHPPDm-F372V nucleotide sequence was introduced, *Arabidopsis thaliana* T₁ plants into which the HaHPPDm-F372V nucleotide sequence was introduced, *Arabidopsis thaliana* T₁ plants into which the MsHPPDm-F372V nucleotide sequence was introduced, *Arabidopsis thaliana* T₁ plants into which the BvHPPDm-F372V nucleotide sequence was introduced, *Arabidopsis thaliana* T₁ plants into which the NtHPPDm-F372V nucleotide sequence was introduced, *Arabidopsis thaliana* T₁ plants into which the CsHPPDm-F372V nucleotide sequence was introduced, *Arabidopsis thaliana* T₁ plants into which the StHPPDm-F372V nucleotide sequence was introduced, *Arabidopsis thaliana* T₁ plants into which the SlHPPDm-F372V nucleotide sequence was introduced, *Arabidopsis thaliana* T₁ plants into which the AhHPPDm-F372V nucleotide sequence was introduced, *Arabidopsis thaliana* T₁ plants into which the CyHPPDm-F372V nucleotide sequence was introduced, *Arabidopsis thaliana* T₁ plants into which the N1HPPDm-F372V nucleotide sequence was introduced, *Arabidopsis thaliana* T₁ plants into which the N2HPPDm-F372V nucleotide sequence was introduced, *Arabidopsis thaliana* T₁ plants into which the N3HPPDm-F372V nucleotide sequence was introduced, *Arabidopsis thaliana* T₁ plants into which the N4HPPDm-F372V nucleotide sequence was introduced, and *Arabidopsis thaliana* T₁ plants into which the N5HPPDm-F372V nucleotide sequence was introduced.

According to the method as described in point 6 of Example 1, the above-mentioned *Arabidopsis thaliana* T₁ plants and wild-type *Arabidopsis thaliana* plants (18 days after sowing) were sprayed with topramezone at two different concentrations respectively, that is, 25 g ai/ha (one-fold field concentration, 1×) and 100 g ai/ha (four-fold field concentration, 4×) to detect the herbicide tolerance of *Arabidopsis thaliana,.* The experimental results are shown in TABLEs 6 and 7.

**TABLE 6 Topramezone tolerance of Arabidopsis thaliana T₁ plants into which mutated HPPD (F372G) from different species sources was introduced**

| Source of the gene | *Arabidopsis thaliana* genotype | Concentration (g ai/ha) | Resistance evaluation |
|---|---|---|---|
| | Wild-type *Arabidopsis thaliana* | 25 | Non-resistant |
| | | 100 | Non-resistant |
| *Zea mays* | ZmHPPDm-F372G | 25 | Highly resistant |
| | | 100 | Moderately resistant |
| *Gossypium hirsutum* | GsHPPDm-F372G | 25 | Moderately resistant |
| | | 100 | Non-resistant |
| *Triticum aestivum* | TaHPPDm-F372G | 25 | Highly resistant |
| | | 100 | Moderately resistant |
| *Brachypodium distachyon* | BdHPPDm-F372G | 25 | Highly resistant |
| | | 100 | Moderately resistant |
| *Hordeum vulgare* | HvHPPDm-F372G | 25 | Highly resistant |
| | | 100 | Poorly resistant |
| *Setaria italica* | SiHPPDm-F372G | 25 | Moderately resistant |
| | | 100 | Poorly resistant |
| *Sorghum bicolor* | SbHPPDm-F372G | 25 | Moderately resistant |
| | | 100 | Non-resistant |
| *Oryza sativa* | OsHPPDm-F372G | 25 | Moderately resistant |
| | | 100 | Non-resistant |
| *Arabidopsis thaliana* | AtHPPDm-F372G | 25 | Poorly resistant |
| | | 100 | Non-resistant |
| *Glycine max* | GmHPPDm-F372G | 25 | Poorly resistant |
| | | 100 | Non-resistant |
| *Cicer arietinum* | CaHPPDm-F372G | 25 | Poorly resistant |
| | | 100 | Non-resistant |
| *Brassica napus* | BnHPPDm-F372G | 25 | Poorly resistant |
| | | 100 | Non-resistant |
| *Helianthus annuus* | HaHPPDm-F372G | 25 | Poorly resistant |
| | | 100 | Non-resistant |
| *Medicago sativa* | MsHPPDm-F372G | 25 | Moderately resistant |
| | | 100 | Non-resistant |
| *Beta vulgaris* | BvHPPDm-F372G | 25 | Moderately resistant |
| | | 100 | Non-resistant |
| *Nicotiana tabacum* | NtHPPDm-F372G | 25 | Poorly resistant |
| | | 100 | Non-resistant |
| *Cucumis sativus* | CsHPPDm-F372G | 25 | Poorly resistant |
| | | 100 | Non-resistant |
| *Solanum tuberosum* | StHPPDm-F372G | 25 | Poorly resistant |
| | | 100 | Non-resistant |
| *Solanum lycopersicum* | SlHPPDm-F372G | 25 | Poorly resistant |
| | | 100 | Non-resistant |
| *Arachis hypogaea* | AhHPPDm-F372G | 25 | Poorly resistant |
| | | 100 | Non-resistant |
| *Cyanobacteria* | CyHPPDm-F372G | 25 | Moderately resistant |
| | | 100 | Non-resistant |
| N1 | N1HPPDm-F372G | 25 | Highly resistant |
| | | 100 | Highly resistant |
| N2 | N2HPPDm-F372G | 25 | Highly resistant |
| | | 100 | Highly resistant |
| N3 | N3HPPDm-F372G | 25 | Highly resistant |
| | | 100 | Highly resistant |
| N4 | N4HPPDm-F372G | 25 | Highly resistant |
| | | 100 | Highly resistant |
| N5 | N4HPPDm-F372G | 25 | Highly resistant |
| | | 100 | Highly resistant |

**TABLE 7 Topramezone tolerance of Arabidopsis thaliana T₁ plants into which mutated**

| HPPD (F372V) from different species sources was introduced | | | |
|---|---|---|---|
| Source of the gene | *Arabidopsis thaliana* genotype | Concentration (g ai/ha) | Resistance evaluation |
| | Wild-type *Arabidopsis thaliana* | 25 | Non-resistant |
| | | 100 | Non-resistant |
| *Zea mays* | ZmHPPDm-F372V | 25 | Highly resistant |
| | | 100 | Poorly resistant |
| *Gossypium hirsutum* | GsHPPDm-F372V | 25 | Moderately resistant |
| | | 100 | Non-resistant |
| *Triticum aestivum* | TaHPPDm-F372V | 25 | Highly resistant |
| | | 100 | Moderately resistant |
| *Brachypodium distachyon* | BdHPPDm-F372V | 25 | Highly resistant |
| | | 100 | Poorly resistant |
| *Hordeum vulgare* | HvHPPDm-F372V | 25 | Highly resistant |
| | | 100 | Poorly resistant |
| *Setaria italica* | SiHPPDm-F372V | 25 | Moderately resistant |
| | | 100 | Poorly resistant |
| *Sorghum bicolor* | SbHPPDm-F372V | 25 | Poorly resistant |
| | | 100 | Non-resistant |
| *Oryza sativa* | OsHPPDm-F372V | 25 | Moderately resistant |
| | | 100 | Non-resistant |
| *Arabidopsis thaliana* | AtHPPDm-F372V | 25 | Poorly resistant |
| | | 100 | Non-resistant |
| *Glycine max* | GmHPPDm-F372V | 25 | Poorly resistant |
| | | 100 | Non-resistant |
| *Cicer artietinum* | CaHPPDm-F372V | 25 | Poorly resistant |
| | | 100 | Non-resistant |
| *Brassica napus* | BnHPPDm-F372V | 25 | Poorly resistant |
| | | 100 | Non-resistant |
| *Helianthus annuus* | HaHPPDm-F372V | 25 | Poorly resistant |
| | | 100 | Non-resistant |
| *Medicago sativa* | MsHPPDm-F372V | 25 | Poorly resistant |
| | | 100 | Non-resistant |
| *Beta vulgaris* | BvHPPDm-F372V | 25 | Poorly resistant |
| | | 100 | Non-resistant |
| *Nicotiana tabacum* | NtHPPDm-F372V | 25 | Poorly resistant |
| | | 100 | Non-resistant |
| *Cucumis sativus* | CsHPPDm-F372V | 25 | Poorly resistant |
| | | 100 | Non-resistant |
| *Solanum tuberosum* | StHPPDm-F372V | 25 | Poorly resistant |
| | | 100 | Non-resistant |
| *Solanum lycopersicum* | SlHPPDm-F372V | 25 | Poorly resistant |
| | | 100 | Non-resistant |
| *Arachis hypogaea* | AhHPPDm-F372V | 25 | Poorly resistant |
| | | 100 | Non-resistant |
| *Cyanobacteria* | CyHPPDm-F372V | 25 | Poorly resistant |
| | | 100 | Non-resistant |
| N1 | N1HPPDm-F372V | 25 | Highly resistant |
| | | 100 | Highly resistant |
| N2 | N2HPPDm-F372V | 25 | Highly resistant |
| | | 100 | Highly resistant |
| N3 | N3HPPDm-F372V | 25 | Highly resistant |
| | | 100 | Highly resistant |
| N4 | N4HPPDm-F372V | 25 | Highly resistant |
| | | 100 | Highly resistant |
| N5 | N5HPPDm-F372V | 25 | Highly resistant |
| | | 100 | Highly resistant |

The results in TABLEs 6 and 7 showed that: as compared with the *Arabidopsis thaliana* plants into which unmutated HPPD gene was introduced in Example 2 and the wild-type *Arabidopsis thaliana* plants, the *Arabidopsis thaliana* plants into which the mutation (F372G or F372V) at 372 position of HPPD gene from different species sources was introduced had different degrees of tolerance to topramezone, and especially the *Arabidopsis thaliana* plants into which the mutation at 372 position of HPPD gene from the microorganism source was introduced had better tolerance to topramezone. Therefore, the mutation (F372G or F372V) at 372 position of HPPD amino acid sequence from different species sources can confer tolerance to topramezone upon plants.

### Example 6: Combination of mutation of the 372 position with that of other position in the HPPD amino acid sequence and the mutation effect thereof

### 1. Acquisition of the sequence with mutation combination

The 372 position of the AsHPPD amino acid sequence was mutated from the original phenylalanine (F) to alanine (A), and deletion mutation was performed upon the alanine (A) at the 110 position, to obtain the mutated AsHPPD (AsHPPDm-F372A-A110) amino acid sequence as set forth in SEQ ID NO:412 in the SEQUENCE LISTING; the AsHPPDm-F372A-A110 nucleotide sequence encoding the above amino acid sequence, which was obtained based on the *Arabidopsis thaliana* /soybean/rice common codon usage bias, is set forth in SEQ ID NO:413 in the SEQUENCE LISTING.

Deletion mutation was performed upon the alanine (A) at the position 110 of the AsHPPD amino acid sequence, to obtain the mutated AsHPPD (AsHPPDm-A110) amino acid sequence as set forth in SEQ ID NO:414 in the SEQUENCE LISTING; the AsHPPDm-A110 nucleotide sequence encoding the above amino acid sequence, which was obtained based on the *Arabidopsis thaliana*/*soybean*/*rice* common codon usage bias, is set forth in SEQ ID NO:415 in the SEQUENCE LISTING.

The 372 position of the PfHPPD amino acid sequence was mutated from the original phenylalanine (F) to alanine (A), and the 336 position (corresponding to the 413 position of the amino acid sequence as set forth in SEQ ID NO:1, that is, 413 position) was mutated from the original glycine (G) to tryptophan (W), to obtain the mutated PfHPPD (PfHPPDm-F372A-G413W) amino acid sequence as set forth in SEQ ID NO:416 in the SEQUENCE LISTING; the PfHPPDm-F372A-G413W nucleotide sequence encoding the above amino acid sequence, which was obtained based on the *Arabidopsis thaliana*/*soybean*/*rice* common codon usage bias, is set forth in SEQ ID NO:417 in the SEQUENCE LISTING.

The 336 position of the PfHPPD amino acid sequence (corresponding to the 413 position of the amino acid sequence as set forth in SEQ ID NO:1, that is, 413 position) was mutated from the original glycine (G) to tryptophan (W), to obtain the mutated PfHPPD (PfHPPDm-G413W) amino acid sequence as set forth in SEQ ID NO:418 in the SEQUENCE LISTING; the PfHPPDm-G413W nucleotide sequence encoding the above amino acid sequence, which was obtained based on the *Arabidopsis thaliana*/*soybean*/*rice* common codon usage bias, is set forth in SEQ ID NO:419 in the SEQUENCE LISTING.

### 2. Construction of the recombinant expression vectors containing the mutation combination of HPPD for Arabidopsis thaliana

According to the method of constructing the recombinant expression vector DBN11375 containing AsHPPDm-F372A-02 nucleotide sequence as described in point 3 of Example 1, the AsHPPDm-F372A-A110 nucleotide sequence, AsHPPDm-A110 nucleotide sequence, PfHPPDm-F372A-G413W nucleotide sequence and PfHPPDm-G413W nucleotide sequence which were linked with the universal adapter primer 1 were respectively subjected to a recombination reaction with the linearized DBNBC-01 expression vector skeleton, to obtain the recombinant expression vectors DBN11500 to DBN11503 in sequence. Sequencing verified that the above nucleotide sequences were correctly inserted in the recombinant expression vectors DBN11500 to DBN11503.

### 3. Transformation of Agrobacterium with the recombinant expression vectors for Arabidopsis thaliana

According to the method of transformation of *Agrobacterium* with the recombinant expression vectors for *Arabidopsis thaliana* as described in point 4 of Example 1, the recombinant expression vectors DBN11500 to DBN11503 which had been correctly constructed were transformed into the *Agrobacterium* GV3101 respectively using a liquid nitrogen method. The results were verified by sequencing, showing that the structures of the recombinant expression vectors DBN11500 to DBN11503 were completely correct.

### 4. Detection of the herbicide tolerance of the Arabidopsis thaliana plants into which mutation combination of HPPD was introduced

According to the method as described in point 5 of Example 1, the *Arabidopsis thaliana* inflorescences were immersed in the *Agrobacterium* solution as described in Example 3, so as to introduce the T-DNA of the recombinant expression vectors DBN11500 to DBN11503 constructed in Example 2, the recombinant expression vectors DBN11375 to DBN11375N constructed in point 3 of Example 1, and the recombinant expression vectors DBN11378 to DBN11378N constructed in point 2 of Example 2, into *Arabidopsis thaliana* chromosome, thereby obtaining the corresponding transgenic *Arabidopsis thaliana* plants, that is, the *Arabidopsis thaliana* T₁ plants into which the AsHPPDm-F372A-A110 nucleotide sequence was introduced, *Arabidopsis thaliana* T₁ plants into which the AsHPPDm-A110 nucleotide sequence was introduced, *Arabidopsis thaliana* T₁ plants into which the PfHPPDm-F372A-G413W nucleotide sequence was introduced, *Arabidopsis thaliana* T₁ plants into which the PfHPPDm-G413W nucleotide sequence was introduced, *Arabidopsis thaliana* T₁ plants into which the AsHPPDm-F372A-02 nucleotide sequence was introduced, *Arabidopsis thaliana* T₁ plants into which the PfHPPDm-F372A-02 nucleotide sequence was introduced, *Arabidopsis thaliana* T₁ plants into which the AsHPPDm-02 nucleotide sequence was introduced and *Arabidopsis thaliana* T₁ plants into which the PfHPPDm-02 nucleotide sequence was introduced.

According to the method as described in point 6 of Example 1, the *Arabidopsis thaliana* T₁ plants into which the AsFPPDm-F372A-A110 nucleotide sequence was introduced, *Arabidopsis thaliana* T₁ plants into which the PfHPPDm-F372A-G413W nucleotide sequence was introduced, *Arabidopsis thaliana* T₁ plants into which the PfHPPDm-G413W nucleotide sequence was introduced, *Arabidopsis thaliana* T₁ plants into which the AsHPPDm-F372A-02 nucleotide sequence was introduced, *Arabidopsis thaliana* T₁ plants into which the PfHPPDm-F372A-02 nucleotide sequence was introduced, *Arabidopsis thaliana* T₁ plants into which the AsHPPDm-02 nucleotide sequence was introduced, *Arabidopsis thaliana* T₁ plants into which the PfHPPDm-02 nucleotide sequence was introduced and wild-type *Arabidopsis thaliana* plants (18 days after sowing) were sprayed with topramezone at three different concentrations respectively, that is, 25 g ai/ha (one-fold field concentration, 1×), 100 g ai/ha (four-fold field concentration, 4×) and 0 g ai/ha (water, 0×) to detect the herbicide tolerance of *Arabidopsis thaliana.* The experimental results are shown in TABLE 8.

**TABLE 8 Topramezone tolerance of Arabidopsis thaliana T₁ plants into which the mutation combination of HPPD was introduced**

| HPPD mutation type | Treatment concentration ( g ai/ha ) | Grade 0 | Grade 1 | Grade 2 | Grade 3 | Score | Resistance Grade |
|---|---|---|---|---|---|---|---|
| AsHPPDm-F372A-A110 | 0 | 20 | 0 | 0 | 0 | 0 | |
| | 25 | 20 | 0 | 0 | 0 | 0 | Highly resistant |
| | 100 | 20 | 0 | 0 | 0 | 0 | Highly resistant |
| AsHPPDm-F372A-02 | 0 | 20 | 0 | 0 | 0 | 0 | |
| | 25 | 20 | 0 | 0 | 0 | 0 | Highly resistant |
| | 100 | 20 | 0 | 0 | 0 | 0 | Highly resistant |
| AsHPPDm-A110 | 0 | 20 | 0 | 0 | 0 | 0 | |
| | 25 | 4 | 16 | 0 | 0 | 27 | Moderately resistant |
| | 100 | 0 | 0 | 14 | 6 | 77 | Non-resistant |
| AsHPPD-02 | 0 | 20 | 0 | 0 | 0 | 0 | |
| | 25 | 2 | 2 | 16 | 0 | 57 | Poorly resistant |
| | 100 | 0 | 0 | 4 | 16 | 93 | Non-resistant |
| PfHPPDm-F372A-G413W | 0 | 20 | 0 | 0 | 0 | 0 | |
| | 25 | 20 | 0 | 0 | 0 | 0 | Highly resistant |
| | 100 | 20 | 0 | 0 | 0 | 0 | Highly resistant |
| PfHPPDm-F372A-02 | 0 | 20 | 0 | 0 | 0 | 0 | |
| | 25 | 20 | 0 | 0 | 0 | 0 | Highly resistant |
| | 100 | 16 | 4 | 0 | 0 | 7 | Highly resistant |
| PfHPPDm-G413W | 0 | 20 | 0 | 0 | 0 | 0 | |
| | 25 | 2 | 18 | 0 | 0 | 30 | Moderately resistant |
| | 100 | 0 | 0 | 10 | 10 | 83 | Non-resistant |
| PfHPPD-02 | 0 | 20 | 0 | 0 | 0 | 0 | |
| | 25 | 0 | 0 | 0 | 20 | 100 | Non-resistant |
| | 100 | 0 | 0 | 0 | 20 | 100 | Non-resistant |
| Wild-type *Arabidopsis thaliana* | 0 | 20 | 0 | 0 | 0 | | |
| | 25 | 0 | 0 | 0 | 20 | 100 | Non-resistant |
| | 100 | 0 | 0 | 0 | 20 | 100 | Non-resistant |

The results in TABLE 8 showed that: (1) compared with wild-type *Arabidopsis thaliana* plants and the *Arabidopsis thaliana* T₁ plants into which AsHPPDm-02 nucleotide sequence was introduced, the *Arabidopsis thaliana* T₁ plants into which AsHPPDm-F372A-A110 nucleotide sequence was introduced, *Arabidopsis thaliana* T₁ plants into which PfHPPDm-F372A-G413W nucleotide sequence was introduced, *Arabidopsis thaliana* T₁ plants into which AsHPPDm-F372A-02 nucleotide sequence was introduced and *Arabidopsis thaliana* T₁ plants into which PfHPPDm-F372A-02 nucleotide sequence was introduced had better tolerance to topramezone (highly resistant); (2) compared with the *Arabidopsis thaliana* T₁ plants into which the AsHPPDm-02 nucleotide sequence was introduced, the *Arabidopsis thaliana* T₁ plants into which the AsPPDm-A110 nucleotide sequence was introduced had a certain tolerance to low concentration of topramezone, but the tolerance was significantly lower than that of the *Arabidopsis thaliana* T₁ plants into which the AsHPPDm-F372A-A110 nucleotide sequence was introduced and the *Arabidopsis thaliana* T₁ plants into which the AsHPPDm-F372A-02 nucleotide sequence was introduced; (3) compared with the *Arabidopsis thaliana* T₁ plants into which the PfHPPDm-02 nucleotide sequence was introduced, the *Arabidopsis thaliana* T₁ plants into which the PfHPPDm-G413W nucleotide sequence was introduced had a certain tolerance to low concentration of topramezone, but the tolerance was significantly lower than that of the *Arabidopsis thaliana* T₁ plants into which the PfHPPDm-F372A-G413W nucleotide sequence was introduced and the *Arabidopsis thaliana* T₁ plants into which the PfHPPDm-F372A-02 nucleotide sequence was introduced. It can be seen that the combination of the mutations at 372 position and at other positions of the HPPD amino acid sequence did not affect the tolerance of the single mutation at 372 position to topramezone, indicating that the mutation at 372 position of the HPPD amino acid sequence conferred the plants with the importance and stability of the tolerance to topramezone.

### Example 7: Mutation of other positions (non-372 positions) of AsHPPD amino acid sequence and verification of the mutation effect

### 1. Acquisition of mutant genes at other positions of AsHPPD amino acid sequence (non-position 372)

The 284 position (corresponding to the 284 position of the amino acid sequence as set forth in SEQ ID NO: 1, i.e. the 284 position) of the AsHPPD amino acid sequence was mutated from the original glutamine (Q) to alanine (A), to obtain the mutant AsHPPD (AsHPPDm-Q284A) amino acid sequence as set forth in SEQ ID NO: 420 in the SEQUENCE LISTING. The AsHPPDm-Q284A nucleotide sequence encoding the above amino acid sequence, which was obtained based on the *Arabidopsis thaliana*/*soybean*/*rice* common codon usage bias, is set forth in SEQ ID NO: 421 in the SEQUENCE LISTING.

The 359 position (corresponding to the 359 position of the amino acid sequence as set forth in SEQ ID NO: 1, i.e. the 359 position) of the AsHPPD amino acid sequence was mutated from the original leucine (L) to tryptophan (W), to obtain the mutant AsHPPD (AsHPPDm-L359W) amino acid sequence as set forth in SEQ ID NO: 422 in the SEQUENCE LISTING. The AsHPPDm-L359W nucleotide sequence encoding the above amino acid sequence, which was obtained based on the *Arabidopsis thaliana*/*soybean*/*rice* common codon usage bias, is set forth in SEQ ID NO: 423 in the SEQUENCE LISTING.

The 415 position (corresponding to the 415 position of the amino acid sequence as set forth in SEQ ID NO: 1, i.e. the 415 position) of the AsHPPD amino acid sequence was mutated from the original phenylalanine (F) to alanine (A), to obtain the mutant AsHPPD (AsHPPDm-F415A) amino acid sequence as set forth in SEQ ID NO: 424 in the SEQUENCE LISTING. The AsHPPDm-F415A nucleotide sequence encoding the above amino acid sequence, which was obtained based on the *Arabidopsis thaliana*/*soybean*/*rice* common codon usage bias, is set forth in SEQ ID NO: 425 in the SEQUENCE LISTING.

### 2. Construction of the recombinant expression vectors containing HPPD gene mutated at other positions (non-position 372) for Arabidopsis thaliana

According to the method of constructing the recombinant expression vector DBN11375 containing the AsHPPDm-F372A-02 nucleotide sequence as described in point 3 of Example 1, the AsHPPDm-Q284A nucleotide sequence, AsHPPDm-L359W nucleotide sequence and AsHPPDm-F415A nucleotide sequence which were linked to the universal adapter primer 1 were respectively subjected to a recombination reaction with the linearized DBNBC-01 expression vector backbone, to obtain the recombinant expression vectors DBN11504, DBN11505 and DBN11506 in sequence. Sequencing verified that the above nucleotide sequences were correctly inserted in the recombinant expression vectors DBN11504, DBN11505 and DBN11506.

### 3. Transformation of Agrobacterium with the recombinant expression vectors for Arabidopsis thaliana

According to the method of transformation of *Agrobacterium* with the recombinant expression vectors for *Arabidopsis thaliana* as described in point 4 of Example 1, the recombinant expression vectors DBN11504, DBN11505 and DBN11506 which had been correctly constructed were transformed into the *Agrobacterium* GV3101 respectively using a liquid nitrogen method. The results were verified by sequencing, showing that the structures of the recombinant expression vectors DBN11504, DBN11505 and DBN11506 were completely correct.

### 4. Detection of the herbicide tolerance of the Arabidopsis thaliana plants into which the mutation at other positions (non-position 372) of the HPPD gene was introduced

According to the method as described in point 5 of Example 1, the *Arabidopsis thaliana* inflorescences were soaked in the *Agrobacterium* solution of Example 3, so as to transform the T-DNA of the recombinant expression vectors DBN11504, DBN11505 and DBN11506 into the *Arabidopsis thaliana* chromosome, thereby obtaining the corresponding transgenic *Arabidopsis thaliana* plants, i.e. *Arabidopsis thaliana* T₁ plants into which the AsHPPDm-Q284A nucleotide sequence was introduced, *Arabidopsis thaliana* T₁ plants into which the AsHPPDm-L359W was introduced and *Arabidopsis thaliana* T₁ plants into which the AsHPPDm-F415A nucleotide sequence was introduced.

According to the method as described in point 6 of Example 1,the *Arabidopsis thaliana* T₁ plants into which the AsHPPDm-Q284A nucleotide sequence was introduced, *Arabidopsis thaliana* T₁ plants into which the AsHPPDm-L359W nucleotide sequence was introduced, *Arabidopsis thaliana* T₁ plants into which the AsHPPDm-F415A nucleotide sequence was introduced and the wild-type *Arabidopsis thaliana* plants (18 days after sowing) were sprayed with topramezone at three different concentrations respectively, i.e. 25 g ai/ha (one-fold field concentrations, 1 ×), 100 g ai/ha (four-fold field concentrations, 4 ×) and 0 g ai/ha (water, 0 ×). The experimental results were shown in TABLE 9.

**TABLE 9 Topramezone tolerance of Arabidopsis thaliana T₁ plants into which HPPD gene mutated at other positions (non-372 positions) was introduced**

| HPPD mutation type | Treatment concentration (g ai/ha) | Grade 0 | Grade 1 | Grade 2 | Grade 3 | Score | Resistance Grade |
|---|---|---|---|---|---|---|---|
| wild-type | 0 | 20 | 0 | 0 | 0 | | |
| | 25 | 0 | 0 | 0 | 20 | 100 | non-resistant |
| | 100 | 0 | 0 | 0 | 20 | 100 | non-resistant |
| AsHPPD-02 | 0 | 20 | 0 | 0 | 0 | | |
| | 25 | 0 | 5 | 13 | 2 | 62 | poorly resistant |
| | 100 | 0 | 0 | 4 | 16 | 93 | non-resistant |
| AsHPPDm-Q284A | 0 | 20 | 0 | 0 | 0 | | |
| | 25 | 0 | 2 | 16 | 2 | 67 | poorly resistant |
| | 100 | 0 | 0 | 5 | 15 | 92 | non-resistant |
| AsHPPDm-L359W | 0 | 20 | 0 | 0 | 0 | | |
| | 25 | 0 | 0 | 2 | 18 | 97 | non-resistant |
| | 100 | 0 | 0 | 0 | 20 | 100 | non-resistant |
| AsHPPDm-F415A | 0 | 20 | 0 | 0 | 0 | | |
| | 25 | 0 | 6 | 12 | 2 | 60 | poorly resistant |
| | 100 | 0 | 0 | 0 | 20 | 100 | non-resistant |

The results in TABLE 9 showed that: as compared with the *Arabidopsis thaliana* T₁ plant into which the AsHPPD-02 nucleotide was introduced, the *Arabidopsis thaliana* T₁ plants into which the AsHPPDm-Q284A nucleotide sequence was introduced, the *Arabidopsis thaliana* T₁ plants into which the AsHPPDm-L359W nucleotide sequence was introduced, and the *Arabidopsis thaliana* T₁ plants into which the AsHPPDm-F415A nucleotide sequence was introduced showed no significant difference in terms of the tolerance to topramezone. It can be seen that not all the mutations of any positions in the HPPD amino acid sequence (such as the three positions described in this example or the adjacent positions of other known effective mutation positions) can confer topramezone tolerance to the plants, and it also indicates that the mutation at position 372 of the HPPD amino acid sequence of the present invention is of unpredictable technical effect.

### Example 8: Acquisition and verification of transgenic soybean plants

### 1. Transformation of Agrobacterium with the recombinant expression vectors

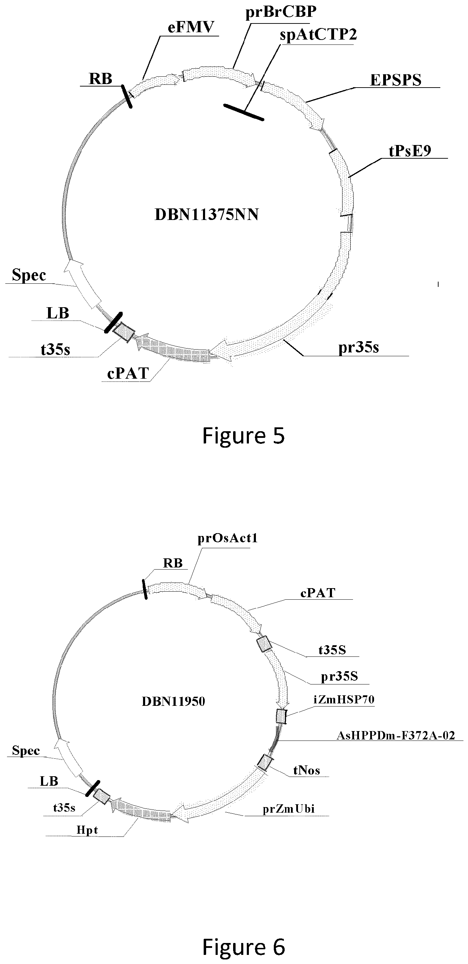

According to the method for constructing the recombinant expression vector DBN11375 containing AsHPPDm-F372A-02 nucleotide sequence as described in point 3 of Example 1, the recombinant expression vector DBN11375NN was constructed as a control, and its structure was shown in FIG. 5 (Spec: the spectinomycin gene; RB: right border; eFMV: 34S enhancer of figwort mosaic virus (SEQ ID NO:7); prBrCBP: promoter of oilseed rape eukaryotic elongation factor gene 1α (Tsf1) (SEQ ID NO:8); spAtCTP2: *Arabidopsis thaliana* chloroplast transit peptide (SEQ ID NO:9); EPSPS: 5-enol-pyruvylshikimate-3-phosphate synthase gene (SEQ ID NO:10); tPsE9: terminator of pea RbcS gene (SEQ ID NO: 11); pr35S: cauliflower mosaic virus 35S promoter (SEQ ID NO: 14); PAT: phosphinothricin-N-acetyl-transferase gene (SEQ ID NO:15); t35S: cauliflower mosaic virus 35S terminator (SEQ ID NO: 16); LB: left border).

The recombinant expression vectors DBN11375 described in point 3 of Example 1, DBN11376 and DBN11378 described in point 2 of Example 2, DBN11500 and DBN11502 described in Example 6, and the above-mentioned control recombinant expression vectors DBN11375NN were transformed into the *Agrobacterium* LBA4404 (Invitrogen, Chicago, USA, CAT: 18313-015) respectively using a liquid nitrogen method, under the following transformation conditions: 100 µL of *Agrobacterium* LBA4404, and 3 µL of plasmid DNA (recombinant expression vector) were placed in liquid nitrogen for 10 minutes, and bathed in warm water at 37°C for 10 minutes; the transformed *Agrobacterium* LBA4404 were inoculated into an LB tube, cultured under the conditions of a temperature of 28°C and a rotation speed of 200 rpm for 2 hours, and then spread on the LB plate containing 50 mg/L of rifampicin and 50 mg/L of spectinomycin until positive single clones were grown, and single clones were picked out for culturing and the plasmids thereof were extracted. The extracted plasmids were identified by sequencing. The results showed that the structures of the recombinant expression vectors DBN11375, DBN11376, DBN11378, DBN11500, DBN11502 and DBN11375NN were completely correct.

### 2. Acquisition of transgenic soybean plants

According to the conventional *Agrobacterium* infection method, the cotyledonary node tissue of a sterilely cultured soybean variety Zhonghuang13 was co-cultured with the *Agrobacterium* as described in point 1 of this Example, so as to introduce the T-DNA (including the figwort mosaic virus 34S enhancer sequence, the oilseed rape eukaryotic elongation factor gene 1α (Tsf1) promoter sequence, the *Arabidopsis thaliana* chloroplast transit peptide sequence, a 5-enolpyruvylshikimate-3-phosphate synthase gene, the pea RbcS gene terminator sequence, the *Arabidopsis thaliana* Ubiquitin10 gene promoter sequence, AsHPPDm-F372A-02 nucleotide sequence, ZmHPPDm-F372A-02 nucleotide sequence, PfHPPDm-F372A-02 nucleotide sequence, AsHPPDm-F372A-A110 nucleotide sequence, PfHPPDm-F372A-G413W nucleotide sequence, the nopaline synthetase gene terminator sequence, the cauliflower mosaic virus 35S promoter sequence, phosphinothricin-N-acetyl-transferase gene, and the cauliflower mosaic virus 35S terminator sequence) of the recombinant expression vectors DBN11375, DBN11376, DBN11378, DBN11500, DBN11502 and DBN11375NN into the soybean chromosomes, thereby obtaining soybean plants into which the AsHPPDm-F372A-02 nucleotide sequence was introduced, soybean plants into which the ZmHPPDm-F372A-02 nucleotide sequence was introduced, soybean plants into which the PfHPPDm-F372A-02 nucleotide sequence was introduced, soybean plants into which the AsHPPDm-F372A-A110 nucleotide sequence was introduced, soybean plants into which the PfHPPDm-F372A-G413W nucleotide sequence was introduced, and soybean plants into which the control vector DBN11375NN was introduced.

For the *Agrobacterium-mediated* soybean transformation, briefly, mature soybean seeds were germinated in a soybean germination culture medium (3.1 g/L of B5 salt, B5 vitamin, 20 g/L of sucrose, and 8 g/L of agar, pH 5.6), and then cultured under the conditions of a temperature of 25 ± 1°C; and a photoperiod (light/dark) of 16 h/8 h. After 4-6 days of germination, soybean sterile seedlings swelling at bright green cotyledonary nodes were taken, hypocotyledonary axes were cut off 3-4 millimeters below the cotyledonary nodes, the cotyledons were cut longitudinally, and apical buds, lateral buds and seminal roots were removed. A wound was created at a cotyledonary node using the knife back of a scalpel, and the wounded cotyledonary node tissues were contacted with an *Agrobacterium* suspension, wherein the *Agrobacterium* can transfer the AsHPPDm-F372A-02 nucleotide sequence, ZmHPPDm-F372A-02 nucleotide sequence, PfHPPDm-F372A-02 nucleotide sequence, AsHPPDm-F372A-A110 nucleotide sequence, or PfHPPDm-F372A-G413W nucleotide sequence to the wounded cotyledonary node tissues (step 1: the infection step). In this step, the cotyledonary node tissues were preferably immersed in the *Agrobacterium* suspension (OD₆₆₀ = 0.5-0.8, an infection culture medium (2.15 g/L of MS salt, B5 vitamin, 20 g/L of sucrose, 10 g/L of glucose, 40 mg/L of acetosyringone (AS), 4 g/L of 2-morpholine ethanesulfonic acid (MES), and 2 mg/L of zeatin (ZT), pH 5.3)) to initiate the inoculation. The cotyledonary node tissues were co-cultured with *Agrobacterium* for a period of time (3 days) (step 2: the co-culturing step). Preferably, after the infection step, the cotyledonary node tissues were cultured in a solid culture medium (4.3 g/L of MS salt, B5 vitamin, 20 g/L of sucrose, 10 g/L of glucose, 4 g/L of MES, 2 mg/L of ZT, and 8 g/L of agar, pH 5.6). After this co-culturing stage, there can be an optional "recovery" step in which a recovery culture medium (3.1 g/L of B5 salt, B5 vitamin, 1 g/L of MES, 30 g/L of sucrose, 2 mg/L of ZT, 8 g/L of agar, 150 mg/L of cephalosporin, 100 mg/L of glutamic acid, and 100 mg/L of aspartic acid, pH 5.6) with the addition of at least one antibiotic (150-250 mg/L of cephalosporin) for inhibiting the growth of *Agrobacterium,* and without the addition of a selective agent for a plant transformant, was used (step 3: the recovery step). Preferably, tissue blocks regenerated from the cotyledonary nodes were cultured in a solid culture medium containing the antibiotic and no selective agent, to eliminate *Agrobacterium* and provide a recovery stage for the infected cells. Subsequently, the tissue blocks regenerated from the cotyledonary nodes were cultured in a culture medium containing a selective agent (glyphosate), and on-growing transformed calli were selected (step 4: the selection step). Preferably, the tissue blocks regenerated from the cotyledonary nodes were cultured in a screening solid culture medium (3.1 g/L of B5 salt, B5 vitamin, 1 g/L of MES, 30 g/L of sucrose, 1 mg/L of 6-benzyladenine (6-BAP), 8 g/L of agar, 150 mg/L of cephalosporin, 100 mg/L of glutamic acid, 100 mg/L of aspartic acid, and 0.25 mol/L of N-(phosphonomethyl)glycine, pH 5.6) containing a selective agent, thus resulting in selective growth of the transformed cells. Then, plants were regenerated from the transformed cells (step 5: the regeneration step). Preferably, the tissue blocks regenerated from the cotyledonary nodes grown in a culture medium containing a selective agent were cultured in solid culture media (a B5 differentiation culture medium and B5 rooting culture medium) to regenerate plants.

The screened out resistant tissues were transferred onto the B5 differentiation culture medium (3.1 g/L of B5 salt, B5 vitamin, 1 g/L of MES, 30 g/L of sucrose, 1 mg/L of ZT, 8 g/L of agar, 150 mg/L of cephalosporin, 50 mg/L of glutamic acid, 50 mg/L of aspartic acid, 1 mg/L of gibberellin, 1 mg/L of auxin, and 0.25 mol/L of N-(phosphonomethyl)glycine, pH 5.6), and cultured at 25°C for differentiation. The differentiated seedlings were transferred onto the B5 rooting culture medium (3.1 g/L of B5 salt, B5 vitamin, 1 g/L of MES, 30 g/L of sucrose, 8 g/L of agar, 150 mg/L of cephalosporin, and 1 mg/L of indole-3-butyric acid (IBA)), cultured in the rooting culture medium at 25°C until a height of about 10 cm, and then transferred to a glasshouse until fruiting. In the greenhouse, the plants were cultured at 26°C for 16 hours, and then cultured at 20°C for 8 hours per day.

The soybean T₀ plants into which the AsHPPDm-F372A-02 nucleotide sequence was introduced, soybean T₀ plants into which the ZmHPPDm-F372A-02 nucleotide sequence was introduced, soybean T₀ plants into which the PfHPPDm-F372A-02 nucleotide sequence was introduced, soybean T₀ plants into which the AsHPPDm-F372A-A110 nucleotide sequence was introduced, and soybean T₀ plants into which the PfHPPDm-F372A-G413W nucleotide sequence was introduced were transplanted into the greenhouse for cultivation and propagation to obtain corresponding transgenic T₁ plants.

### 3. Verification of the transgenic soybean plants using TaqMan

About 100 mg of leaves from the soybean T₁ plants into which the AsHPPDm-F372A-02 nucleotide sequence was introduced, soybean T₁ plants into which the ZmHPPDm-F372A-02 nucleotide sequence was introduced, soybean T₁ plants into which the PfHPPDm-F372A-02 nucleotide sequence was introduced, soybean T₁ plants into which the AsHPPDm-F372A-A110 nucleotide sequence was introduced, soybean T₁ plants into which the PfHPPDm-F372A-G413W nucleotide sequence was introduced, and soybean T₁ plants into which the control vector DBN11375NN was introduced were taken as samples, and the genomic DNA thereof was extracted with a DNeasy Plant Maxi Kit of Qiagen, and copy numbers of an *EPSPS* gene were detected by the Taqman probe fluorescence quantitative PCR method so as to determine the copy numbers of the mutant HPPD gene. At the same time, wild-type soybean plants were used as controls, and detected and analyzed according to the above-mentioned method. Triple repeats were set for the experiments, and were averaged.

The specific method for detecting the copy number of the *EPSPS* gene was as follows:
Step 11. 100 mg of leaves of the soybean T₁ plants into which the AsHPPDm-F372A-02 nucleotide sequence was introduced, soybean T₁ plants into which the ZmHPPDm-F372A-02 nucleotide sequence was introduced, soybean T₁ plants into which the PfHPPDm-F372A-02 nucleotide sequence was introduced, soybean T₁ plants into which the AsFPPDm-F372A-A110 nucleotide sequence was introduced, soybean T₁ plants into which the PfHPPDm-F372A-G413W nucleotide sequence was introduced, soybean T₁ plants into which the control vector DBN11375NN was introduced, and wild-type soybean plants were taken, and ground into a homogenate using liquid nitrogen in a mortar, and triple repeats were taken for each sample;
Step 12. The genomic DNA of the above-mentioned samples was extracted using a DNeasy Plant Mini Kit of Qiagen, with the particular method as described in the product manual;
Step 13. The concentrations of the genomic DNA of the above-mentioned samples were detected using NanoDrop 2000 (Thermo Scientific);
Step 14. The concentrations of the genomic DNA of the above-mentioned samples were adjusted to a same value in the range of from 80 to 100 ng/µL;
Step 15. The copy numbers of the samples were identified using the Taqman probe fluorescence quantitative PCR method, wherein samples for which the copy numbers were known and had been identified were taken as standards, the samples of the wild-type soybean plants were taken as the control, and triple repeats were taken for each sample, and were averaged; the sequences of fluorescence quantitative PCR primers and a probe were as follows:
   the following primers and probe were used to detect the *EPSPS* gene sequence:
   primer 1:ctggaaggcgaggacgtcatcaata, as set forth in SEQ ID NO: 401 in the SEQUENCE LISTING;
   primer 2: tggcggcattgccgaaatcgag, as set forth in SEQ ID NO: 402 in the SEQUENCE LISTING;
   probe 1: atgcaggcgatgggcgcccgcatccgta, as set forth in SEQ ID NO: 403 in the SEQUENCE LISTING;

### PCR reaction system:

| | |
|---|---|
| JumpStart^{™} Taq ReadyMix^{™} (Sigma) | 10 µL |
| 50× primer/probe mixture | 1 µL |
| genomic DNA | 3 µL |
| water (ddH₂O) | 6 µL |

The 50× primer/probe mixture comprises 45 µL of each primer at a concentration of 1 mM, 50 µL of the probe at a concentration of 100 µM, and 860 µL of 1× TE buffer, and was stored at 4°C in an amber tube.

### PCR reaction conditions:

| Step | Temperature | Time |
|---|---|---|
| 21 | 95°C | 5 min |
| 22 | 95°C | 30 s |
| 23 | 60°C | 1 min |
| 24 | go back to step 22, and repeat 40 times | |

Data was analyzed using software SDS2.3 (Applied Biosystems).

By analyzing the experimental results of the copy number of the *EPSPS* gene, it was further demonstrated that the AsHPPDm-F372A-02 nucleotide sequence, ZmHPPDm-F372A-02 nucleotide sequence, PfHPPDm-F372A-02 nucleotide sequence, AsHPPDm-F372A-A110 nucleotide sequence, PfHPPDm-F372A-G413W nucleotide sequence had all been incorporated into the chromosome of the detected soybean plants, and all of the soybean T₁ plants into which the AsHPPDm-F372A-02 nucleotide sequence was introduced, soybean T₁ plants into which the ZmHPPDm-F372A-02 nucleotide sequence was introduced, soybean T₁ plants into which the PfHPPDm-F372A-02 nucleotide sequence was introduced, soybean T₁ plants into which the AsHPPDm-F372A-A110 nucleotide sequence was introduced, soybean T₁ plants into which the PfHPPDm-F372A-G413W nucleotide sequence was introduced, and the soybean T₁ plants into which the control vector DBN11375NN was introduced resulted in single-copy transgenic soybean plants.

### 4. Detection of the herbicide tolerance of the transgenic soybean plants to topramezone

The soybean T₁ plants into which the AsHPPDm-F372A-02 nucleotide sequence was introduced, soybean T₁ plants into which the ZmHPPDm-F372A-02 nucleotide sequence was introduced, soybean T₁ plants into which the PfHPPDm-F372A-02 nucleotide sequence was introduced, soybean T₁ plants into which the AsHPPDm-F372A-A110 nucleotide sequence was introduced, soybean T₁ plants into which the PfHPPDm-F372A-G413W nucleotide sequence was introduced, soybean T₁ plants into which the control vector DBN11375NN was introduced and wild-type soybean plants (V3-V4 at seedling stage) were sprayed with topramezone at two different concentrations respectively, i.e. 50 g ai/ha (2-fold field concentration, 2 ×) and 100 g ai/ha (4-fold field concentration, 4 ×) to detect the herbicide tolerance of soybean plants. According to the method in point 6 of Example 1, after 7 days of spraying (7 DAT), the damage degree of each plant by the herbicide was statistically analyzed, and the scoring and resistance evaluation were carried out accordingly. The soybean plants into which the AsHPPDm-F372A-02 nucleotide sequence was introduced were of two strains in total (S1 and S2), the soybean plants into which the ZmHPPDm-F372A-02 nucleotide sequence was introduced were of two strains in total (S3 and S4), the soybean plants into which the PfHPPDm-F372A-02 nucleotide sequence was introduced were of two strains in total (S5 and S6), the soybean plants into which the AsHPPDm-F372A-A110 nucleotide sequence was introduced were of two strains in total (S7 and S8), the soybean plants into which the PfHPPDm-F372A-G413W nucleotide sequence was introduced were of two strains in total (S9 and S10), the soybean plants into which the control vector DBN11375NN nucleotide sequence was introduced were of one strain in total (S11), and the wild-type soybean plants were of one strain in total (CK1); and 8 plants were selected from each strain and detected. The results were shown in TABLE 10 and FIG. 7.

**TABLE 10 Herbicide tolerance of transgenic soybean T₁ plants**

| Source of the gene | Strain | Treatment concentration (g ai/ha) | Classification and statistics of phytotoxicity | | | | Score | Resistance evaluation |
|---|---|---|---|---|---|---|---|---|
| | | | Grade 0 | Grade 1 | Grade 2 | Grade 3 | | |
| | CK1 | 50 | 0 | 0 | 0 | 8 | 100 | non-resistant |
| | | 100 | 0 | 0 | 0 | 8 | 100 | non-resistant |
| | S11 | 50 | 0 | 0 | 0 | 8 | 100 | non-resistant |
| | | 100 | 0 | 0 | 0 | 8 | 100 | non-resistant |
| *Avena sativa* | S1 | 50 | 8 | 0 | 0 | 0 | 0 | Highly resistant |
| | | 100 | 8 | 0 | 0 | 0 | 0 | Highly resistant |
| | S2 | 50 | 8 | 0 | 0 | 0 | 0 | Highly resistant |
| | | 100 | 8 | 0 | 0 | 0 | 0 | Highly resistant |
| | S7 | 50 | 8 | 0 | 0 | 0 | 0 | Highly resistant |
| | | 100 | 8 | 0 | 0 | 0 | 0 | Highly resistant |
| | S8 | 50 | 8 | 0 | 0 | 0 | 0 | Highly resistant |
| | | 100 | 8 | 0 | 0 | 0 | 0 | Highly resistant |
| *Zea mays* | S3 | 50 | 7 | 1 | 0 | 0 | 4 | Highly resistant |
| | | 100 | 2 | 5 | 1 | 0 | 29 | Moderately resistant |
| | S4 | 50 | 6 | 2 | 0 | 0 | 8 | Highly resistant |
| | | 100 | 2 | 6 | 0 | 0 | 25 | Moderately resistant |
| *Pseudomonas fluorescens* | S5 | 50 | 5 | 3 | 0 | 0 | 13 | Moderately resistant |
| | | 100 | 1 | 3 | 4 | 0 | 46 | Poorly resistant |
| | S6 | 50 | 4 | 3 | 1 | 0 | 21 | Moderately resistant |
| | | 100 | 0 | 5 | 3 | 0 | 46 | Poorly resistant |
| | S9 | 50 | 4 | 4 | 0 | 0 | 17 | Moderately resistant |
| | | 100 | 2 | 2 | 4 | 0 | 42 | Poorly resistant |
| | S10 | 50 | 5 | 2 | 1 | 0 | 17 | Moderately resistant |
| | | 100 | 0 | 4 | 4 | 0 | 50 | Poorly resistant |

For soybeans, 4-fold field concentration of topramezone is an effective dose for high-pressure treatment. The results in TABLE 10 and FIG. 7 showed: (1) as compared to the soybean T₁ plants into which the control vector DBN11375NN was introduced and the wild-type soybean plants, the soybean T₁ plants into which the AsHPPDm-F372A-02 nucleotide sequence was introduced, soybean T₁ plants into which the ZmHPPDm-F372A-02 nucleotide sequence was introduced, soybean T₁ plants into which the PfHPPDm-F372A-02 nucleotide sequence was introduced, soybean T₁ plants into which the AsHPPDm-F372A-A110 nucleotide sequence was introduced and soybean T₁ plants into which the PfHPPDm-F372A-G413W nucleotide sequence was introduced were able to produce higher tolerance to topramezone herbicides, indicating that the mutant HPPD (F372A) can confer the transgenic soybean plants a high level of tolerance to topramezone; (2) as compared to the soybean T₁ plants into which the AsHPPDm-F372A-02 nucleotide sequence was introduced and soybean T₁ plants into which the PfHPPDm-F372A-02 nucleotide sequence was introduced, there was no significant difference in the topramezone tolerance of soybean T₁ plants into which the AsHPPDm-F372A-A110 nucleotide sequence was introduced and the soybean T₁ plants into which the PfHPPDm-F372A-G413W nucleotide sequence was introduced, further indicating that the mutation at position 372 of the HPPD amino acid sequence confers importance and stability of the tolerance to topramezone upon the plants.

### Example 9: Acquisition and verification of transgenic rice plants

### 1. Construction of the recombinant expression vectors of rice containing HPPD gene

The 5' and 3' ends of the AsHPPDm-F372A-02 nucleotide sequence as described in point 1 of Example 1, the ZmHPPDm-F372A-02 nucleotide sequence and the PfHPPDm-F372A-02 nucleotide sequence as described in point 1 of Example 2, and the AsHPPDm-F372A-A110 nucleotide sequence as described in Example 6 were respectively linked to the following universal adapter primer 2:
Universal adapter primer 2 for the 5' end: 5'- tgcagataccaagcggccactagt -3', as set forth in SEQ ID NO: 404 in the SEQUENCE LISTING;
Universal adapter primer 2 for the 3' end: 5'- caaatgtttgaacgatcggcgcgcc -3', as set forth in SEQ ID NO: 400 in the SEQUENCE LISTING;

A plant expression vector DBNBC-02 was subjected to double digestion using restriction enzymes *Spe* I and *Asc* I to linearize the plant expression vector. The digestion product was purified to obtain the linearized DBNBC-02 expression vector backbone (vector backbone: pCAMBIA2301 (which is available from CAMBIA)), which then underwent a recombination reaction with the AsHPPDm-F372A-02 nucleotide sequence linked to the universal adapter primer 2, according to the procedure of Takara In-Fusion products seamless connection kit (Clontech, CA, USA, CAT: 121416) instructions, to construct a recombinant expression vector DBN11950 with the vector structure as shown in FIG. 6. (Spec: spectinomycin gene; RB: right border; prOsAct1: rice actin 1 promoter (SEQ ID NO:405); PAT: phosphinothricin-N-acetyl-transferase gene (SEQ ID NO:15); t35S: cauliflower mosaic virus 35S terminator (SEQ ID NO: 16); pr35S: cauliflower mosaic virus 35S promoter (SEQ ID NO:14); iZmHSP70: *Zea mays* heat shock 70 kDa protein intron (SEQ ID NO:406); AsHPPDm-F372A-02: AsHPPDm-F372A-02 nucleotide sequence (SEQ ID NO:6); tNos: nopaline synthetase gene terminator (SEQ ID NO:13); prZmUbi: *Zea mays* ubiquitin 1 gene promoter (SEQ ID NO:407); Hpt: hygromycin phosphotransferase gene (SEQ ID NO:408); t35S: cauliflower mosaic virus 35S terminator (SEQ ID NO:16); LB: left border).

*Escherichia coli* T₁ competent cells were transformed according to the heat shock method described in point 3 of Example 1, and the plasmids in the cells were extracted through the alkaline method. The extracted plasmid was identified by sequencing. The results indicated that the recombinant expression vector DBN11950 contained the nucleotide sequence set forth in SEQ ID NO: 6 in the SEQUENCE LISTING, i.e. the AsHPPDm-F372A-02 nucleotide sequence.

According to the above method for constructing recombinant expression vector DBN11950, the ZmHPPDm-F372A-02 nucleotide sequence, the PfHPPDm-F372A-02 nucleotide sequence and the AsHPPDm-F372A-A110 nucleotide sequence which were linked to the universal adapter primer 2 were respectively subjected to a recombination reaction with the linearized DBNBC-02 expression vector backbone, to construct the recombinant expression vectors DBN11951 to DBN11953 in sequence. Sequencing verified that the ZmHPPDm-F372A-02 nucleotide sequence, the PfHPPDm-F372A-02 nucleotide sequence and the AsHPPDm-F372A-A110 nucleotide sequence were correctly inserted in the recombinant expression vectors DBN11951 to DBN11953.

### 2. Transformation of Agrobacterium with the recombinant expression vectors

The recombinant expression vectors DBN11950 to DBN11953 which had been constructed correctly were respectively transformed into *Agrobacterium* EHA105α using a liquid nitrogen method, with the following transformation conditions: 100 µL of *Agrobacterium* EHA105α, and 3 µL of plasmid DNA (recombinant expression vector) were placed in liquid nitrogen for 10 minutes, and bathed in warm water at 37°C for 10 minutes; the transformed *Agrobacterium* EHA105α was inoculated into an LB tube, cultured under the conditions of a temperature of 28°C and a rotation speed of 200 rpm for 2 hours, and spread on the LB plate containing 50 mg/L of rifampicin and 50 mg/L of spectinomycin until positive single clones were grown, and single clones were picked out for culturing and the plasmids thereof were extracted. The extracted plasmids were identified by sequencing. The results showed that the structures of the recombinant expression vectors DBN11950 to DBN11953 were completely correct.

### 3. Acquisition of transgenic rice plants

For the *Agrobacterium-mediated* rice transformation, briefly, the rice cultivar Nipponbare were germinated in the induction culture medium (3.1 g/L N6 salts, N6 vitamins, 300 mg/L casein, 30 g/L sucrose, 2 mg/L dichlorophenoxyacetic acid (2,4-D), 3 g/L plant gel; pH 5.8), and the calli were induced from mature rice embryos (Step 1: calli induction step). After that, calli were selected and then were contacted with an *Agrobacterium* suspension, wherein *Agrobacterium* can transfer the nucleotide sequences of AsHPPDm-F372A-02 nucleotide sequence, AsHPPDm-F372A-A110 nucleotide sequence, ZmHPPDm-F372A-02 nucleotide sequence or PfHPPDm-F372A-02 nucleotide sequence into at least one cell in one of the calli (step 2: infection step). In this step, the calli were preferably immersed in the *Agrobacterium* suspension (OD₆₆₀ = 0.3, infection medium (3.1 g/L N6 salts, N6 vitamins, 300 mg/L casein, 30 g/L sucrose, 10 g/L glucose, 40 mg/L AS, 2 mg/L 2,4-D; pH 5.4)) to start the inoculation. The calli were co-cultured with *Agrobacterium* for a period of time (3 days) (step 3: co-culturing step). Preferably, after the infection step, the calli were cultured in a solid medium (3.1 g/L N6 salts, N6 vitamins, 300 mg/L casein, 30 g/L sucrose, 10 g/L glucose, 40 mg/L AS, 2 mg/L 2,4-D, 3 g/L plant gel; pH 5.8). After the co-culturing step, there may be a "recovery" step in which a recovery medium (3.1 g/L N6 salts, N6 vitamins, 300 mg/L casein, 30 g/L sucrose, 2 mg/L 2,4-D, 3 g/L plant gel; pH 5.8) with the addition of at least one antibiotic (150-250 mg/L of cephamycin) for inhibiting the growth of *Agrobacterium,* and without the addition of any selective agent for plant transformants, was used (step 4: recovery step). Preferably, the calli were cultured in a solid medium comprising antibiotic but no selective agent, so as to eliminate *Agrobacterium* and provide a recovery period for the infected cells. Then, the inoculated calli were cultured on a medium containing a selective agent (hygromycin) and the on-growing transformed calli were selected (step 5: selection step). Preferably, the calli were cultured in a solid selective medium (3.1 g/L N6 salts, N6 vitamins, 300 mg/L casein, 30 g/L sucrose, 50 mg/L of hygromycin, 2 mg/L 2,4-D, 3 g/L plant gel; pH 5.8) comprising a selective agent, which resulted in the selective growth of the transformed cells. Then, the calli was regenerated into plants (step 6: regeneration step). Preferably, the calli growing on the medium containing a selective agent were cultured in a solid medium (N6 differential medium and MS rooting medium) to regenerate plants.

The resistant calli obtained from screening were transferred to N6 differential medium (3.1 g/L N6 salts, N6 vitamins, 300 mg/L casein, 30 g/L sucrose, 150 mg/L of cephamycin, 20 mg/L of hygromycin, 2 mg/L 6-benzyladenine, 1 mg/L napthalene acetic acid, 3 g/L plant gel; pH 5.8), and cultured for differentiation at 25°C. The differentiated plantlets were transferred to MS rooting medium (2.15 g/L MS salts, MS vitamins, 300 mg/L casein, 15 g/L sucrose, 3 g/L plant gel; pH 5.8), and cultured at 25°C. When the plantlets reached about 10 cm in height, they were moved to greenhouse and cultured until fruiting. In the greenhouse, they were cultured at 30°C to obtain transformed T₀ rice plants.

The rice T₀ plants into which the AsHPPDm-F372A-02 nucleotide sequence was introduced, rice T₀ plants into which the AsHPPDm-F372A-A110 nucleotide sequence was introduced, rice T₀ plants into which the ZmHPPDm-F372A-02 nucleotide sequence was introduced and rice T₀ plants into which the PfHPPDm-F372A-02 nucleotide sequence was introduced were transplanted into the greenhouse for cultivation and propagation to obtain corresponding transgenic T₁ plants.

### 4. Verification of the transgenic rice plants using TaqMan

About 100 mg of leaves from the rice T₁ plants into which the AsHPPDm-F372A-02 nucleotide sequence was introduced, rice T₁ plants into which the AsFPPDm-F372A-A110 nucleotide sequence was introduced, rice T₁ plants into which the ZmHPPDm-F372A-02 nucleotide sequence was introduced, and rice T₁ plants into which the PfHPPDm-F372A-02 nucleotide sequence was introduced were taken as samples, and the genomic DNA thereof was extracted with a DNeasy Plant Maxi Kit of Qiagen, and copy numbers of an *Hpt* gene were detected by the Taqman probe fluorescence quantitative PCR method so as to determine the copy numbers of the mutant HPPD gene. At the same time, wild-type rice plants were used as controls, and detected and analyzed according to the above-mentioned method. Triple repeats were set for the experiments, and were averaged.

The specific method for detecting the copy number of the *Hpt* gene was as follows:
Step 31. 100 mg of leaves of the rice plants into which the AsHPPDm-F372A-02 nucleotide sequence was introduced, rice plants into which the AsHPPDm-F372A-A110 nucleotide sequence was introduced, rice plants into which the ZmHPPDm-F372A-02 nucleotide sequence was introduced, rice plants into which the PfHPPDm-F372A-02 nucleotide sequence was introduced, and wild-type rice plants were taken, and ground into a homogenate using liquid nitrogen in a mortar, and triple repeats were taken for each sample;
Step 32. The genomic DNA of the above-mentioned samples was extracted using a DNeasy Plant Mini Kit of Qiagen, with the particular method as described in the product manual;
Step 33. The concentrations of the genomic DNA of the above-mentioned samples were detected using NanoDrop 2000 (Thermo Scientific);
Step 34. The concentrations of the genomic DNA of the above-mentioned samples were adjusted to a consistent value in the range of from 80 to 100 ng/µL;
Step 35. The copy numbers of the samples were identified using the Taqman probe fluorescence quantitative PCR method, wherein samples for which the copy numbers were known and had been identified were taken as standards, the samples of the wild-type rice plants were taken as the control, and triple repeats were taken for each sample, and were averaged; the sequences of fluorescence quantitative PCR primers and a probe were as follows:
   the following primers and probe were used to detect the *Hpt* gene sequence:
   primer 3: gcataacagcggtcattgactg, as set forth in SEQ ID NO: 409 in SEQUENCE LISTING;
   primer 4: agaagatgttggcgacctcg, as set forth in SEQ ID NO: 410 in SEQUENCE LISTING;
   probe 2: agcgaggcgatgttcggggattc, as set forth in SEQ ID NO: 411 in the SEQUENCE LISTING;

### PCR reaction system:

| | |
|---|---|
| JumpStart^{™} Taq ReadyMix^{™} (Sigma) | 10 µL |
| 50× primer/probe mixture | 1 µL |
| genomic DNA | 3 µL |
| water (ddH₂O) | 6 µL |

The 50× primer/probe mixture comprises 45 µL of each primer at a concentration of 1 mM, 50 µL of the probe at a concentration of 100 µM, and 860 µL of 1× TE buffer, and was stored at 4°C in an amber tube.

### PCR reaction conditions:

| Step | Temperature | Time |
|---|---|---|
| 41 | 95°C | 5 min |
| 42 | 95°C | 30 s |
| 43 | 60°C | 1 min |
| 44 | go back to step 42, and repeat 40 times | |

Data was analyzed using software SDS2.3 (Applied Biosystems).

By analyzing the experimental results of the copy number of the *EPSPS* gene, it was further demonstrated that the AsHPPDm-F372A-02 nucleotide sequence, AsHPPDm-F372A-A110 nucleotide sequence, ZmHPPDm-F372A-02 nucleotide sequence and PfHPPDm-F372A-02 nucleotide sequence had all been incorporated into the chromosome of the detected rice plants, and all of the rice T₁ plants into which the AsHPPDm-F372A-02 nucleotide sequence was introduced, rice T₁ plants into which the AsHPPDm-F372A-A110 nucleotide sequence was introduced, rice T₁ plants into which the ZmHPPDm-F372A-02 nucleotide sequence was introduced and rice T₁ plants into which the PfHPPDm-F372A-02 nucleotide sequence was introduced resulted in single-copy transgenic soybean plants.

### 5. Detection of the herbicide tolerance of the transgenic rice plants

The rice T₁ plants into which the AsHPPDm-F372A-02 nucleotide sequence was introduced, rice T₁ plants into which the AsHPPDm-F372A-A110 nucleotide sequence was introduced, rice T₁ plants into which the ZmHPPDm-F372A-02 nucleotide sequence was introduced, rice T₁ plants into which the PfHPPDm-F372A-02 nucleotide sequence was introduced and wild-type rice plants (V3-V4 at seedling stage) were sprayed with topramezone at two different concentrations respectively, i.e. 50 g ai/ha (2-fold field concentration, 2 ×) and 100 g ai/ha (4-fold field concentration, 4 ×) to detect the herbicide tolerance of rice plants. According to the method in point 6 of Example 1, after 7 days of spraying (7 DAT), the damage degree of each plant by the herbicide was statistically analyzed, and the scoring and resistance evaluation were carried out accordingly. The rice T₁ plants into which AsHPPDm-F372A-02 nucleotide sequence was introduced were of two strains in total (S12 and S13), rice T₁ plants into which AsHPPDm-F372A-A110 nucleotide sequence was introduced were of two strains in total (S14 and S15), rice T₁ plants into which ZmHPPDm-F372A-02 nucleotide sequence was introduced were of two strains in total (S16 and S17), rice T₁ plants into which PfHPPDm-F372A-02 nucleotide sequence was introduced were of two strains in total (S18 and S19), and the wild-type rice plants were of one strain in total (CK2); and 8 plants were selected from each strain and tested. The results were shown in TABLE 11.

**TABLE 11 Herbicide tolerance of transgenic rice T₁ plants**

| Source of the gene | Strain | Treatment concentration (g ai/ha) | Classification and statistics of phytotoxicity | | | | Score | Resistance evaluation |
|---|---|---|---|---|---|---|---|---|
| | | | Grade 0 | Grade 1 | Grade 2 | Grade 3 | | |
| | CK2 | 50 | 0 | 0 | 0 | 8 | 100 | non-resistant |
| | | 100 | 0 | 0 | 0 | 8 | 100 | non-resistant |
| *Avena sativa* | S12 | 50 | 8 | 0 | 0 | 0 | 0 | Highly resistant |
| | | 100 | 8 | 0 | 0 | 0 | 0 | Highly resistant |
| | S13 | 50 | 8 | 0 | 0 | 0 | 0 | Highly resistant |
| | | 100 | 85 | 0 | 0 | 0 | 0 | Highly resistant |
| | S14 | 50 | 8 | 0 | 0 | 0 | 0 | Highly resistant |
| | | 100 | 8 | 0 | 0 | 0 | 0 | Highly resistant |
| | S15 | 50 | 8 | 0 | 0 | 0 | 0 | Highly resistant |
| | | 100 | 7 | 1 | 0 | 0 | 4 | Highly resistant |
| *Zea mays* | S16 | 50 | 8 | 0 | 0 | 0 | 0 | Highly resistant |
| | | 100 | 3 | 3 | 2 | 0 | 29 | Moderately resistant |
| | S17 | 50 | 6 | 2 | 0 | 0 | 8 | Highly resistant |
| | | 100 | 5 | 2 | 1 | 0 | 17 | Moderately resistant |
| *Pseudomonas fluorescens* | S18 | 50 | 4 | 3 | 1 | 0 | 21 | Moderately resistant |
| | | 100 | 1 | 4 | 3 | 0 | 42 | Poorly resistant |
| | S19 | 50 | 4 | 4 | 0 | 0 | 17 | Moderately resistant |
| | | 100 | 0 | 3 | 5 | 0 | 54 | Poorly resistant |

For rice, 4-fold field concentration of topramezone is an effective dose for high-pressure treatment. The results in TABLE 11 showed that as compared to the wild-type rice plants, the rice T₁ plants into which the AsHPPDm-F372A-02 nucleotide sequence was introduced, rice T₁ plants into which the AsHPPDm-F372A-A110 nucleotide sequence was introduced, rice T₁ plants into which the ZmHPPDm-F372A-02 nucleotide sequence was introduced, rice T₁ plants into which the PfHPPDm-F372A-02 nucleotide sequence was introduced were able to produce higher tolerance to topramezone herbicides. Meanwhile, there was no significant difference between the topramezone tolerance of the rice T₁ plants into which the AsHPPDm-F372A-02 nucleotide sequence was introduced and that of the rice T₁ plants into which the AsHPPDm-F372A-A110 nucleotide sequence was introduced, indicating that the mutation at position 372 of the HPPD amino acid sequence is sufficient to provide the transgenic rice plants with a high level of tolerance to topramezone herbicides.

### Example 10: Verification of the tolerance of transgenic soybean plants to other HPPD inhibitors

In order to further verify the tolerance effect of HPPD (F372A) on other HPPD inhibitors, the soybean T₁ plants into which the AsHPPDm-F372A-02 nucleotide sequence was introduced, soybean T₁ plants into which the ZmHPPDm-F372A-02 nucleotide sequence was introduced, soybean T₁ plants into which the PfHPPDm-F372A-02 nucleotide sequence was introduced, soybean T₁ plants into which the AsHPPDm-F372A-A110 nucleotide sequence was introduced, soybean T₁ plants into which the control vector DBN11375NN was introduced in Example 8, and the wild-type soybean plants (V3-V4 at seedling stage) were sprayed as follows to detect the herbicide tolerance of each soybean plants, (1) 140 g ai/ha (2-fold field concentration, 2 ×) isoxaflutole; (2) 280 g ai/ha (4-fold field concentration, 4 ×) isoxaflutole; (3) 210 g ai/ha (2-fold field concentration, 2 ×) mesotrione; (4) 420 g ai/ha (4-fold field concentration, 4 ×) mesotrione. According to the method in point 6 of Example 1, after 7 days of spraying (7 DAT), the damage degree of each plant by the herbicide was statistically analyzed, and the scoring and resistance evaluation were carried out accordingly. The soybean T₁ plants into which AsHPPDm-F372A-02 nucleotide sequence was introduced were of two strains in total (S1 and S2), the soybean T₁ plants into which the ZmHPPDm-F372A-02 nucleotide sequence was introduced were of two strains in total (S3 and S4), the soybean T₁ plants into which the PfHPPDm-F372A-02 nucleotide sequence was introduced were of two strains in total (S5 and S6), the soybean T₁ plants into which the AsHPPDm-F372A-A110 nucleotide sequence was introduced were of two strains in total (S7 and S8), the soybean T₁ plants into which the control vector DBN11375NN nucleotide sequence was introduced were of one strain in total (S11), and the wild-type soybean plants were of one strain in total (CK1); and 8 plants were selected from each strain and tested. The results were shown in TABLE 12.

**TABLE 12 Herbicide tolerance of transgenic rice T₁ plants**

| Source of the gene | Strain | Treatment concentration of isoxaflutole (g ai/ha) | Resistance evaluation | Treatment concentration of mesotrione (g ai/ha) | Resistance evaluation |
|---|---|---|---|---|---|
| | CK1 | 140 | non-resistant | 210 | non-resistant |
| | | 280 | non-resistant | 420 | non-resistant |
| | S11 | 140 | non-resistant | 210 | non-resistant |
| | | 280 | non-resistant | 420 | non-resistant |
| *Avena sativa* | S1 | 140 | Highly resistant | 210 | Highly resistant |
| | | 280 | Moderately resistant | 420 | Highly resistant |
| | S2 | 140 | Highly resistant | 210 | Highly resistant |
| | | 280 | Moderately resistant | 420 | Highly resistant |
| | S7 | 140 | Highly resistant | 210 | Highly resistant |
| | | 280 | Moderately resistant | 420 | Highly resistant |
| | S8 | 140 | Highly resistant | 210 | Highly resistant |
| | | 280 | Moderately resistant | 420 | Highly resistant |
| *Zea mays* | S3 | 140 | Moderately resistant | 210 | Moderately resistant |
| | | 280 | Poorly resistant | 420 | Poorly resistant |
| | S4 | 140 | Moderately resistant | 210 | Poorly resistant |
| | | 280 | Poorly resistant | 420 | Poorly resistant |
| *Pseudomonas fluorescens* | S5 | 140 | Highly resistant | 210 | Moderately resistant |
| | | 280 | Moderately resistant | 420 | Poorly resistant |
| | S6 | 140 | Highly resistant | 210 | Moderately resistant |
| | | 280 | Moderately resistant | 420 | Poorly resistant |

For soybeans, 4-fold field concentration of isoxaflutole and 4-fold field concentration of mesotrione are effective doses for high-pressure treatment. The results in TABLE 12 showed: (1) as compared to the soybean T₁ plants into which the control vector DBN11375NN was introduced and the wild-type soybean plants, the soybean T₁ plants into which the AsHPPDm-F372A-02 nucleotide sequence was introduced, soybean T₁ plants into which the ZmHPPDm-F372A-02 nucleotide sequence was introduced, soybean T₁ plants into which the PfHPPDm-F372A-02 nucleotide sequence was introduced and soybean T₁ plants into which the AsHPPDm-F372A-A110 nucleotide sequence was introduced were able to produce different degrees of tolerance to isoxaflutole and mesotrione herbicides, and especially soybean T₁ plants with HPPD gene at position 372 mutated from *Avena sativa* origin have better herbicide tolerance to isoxaflutole and mesotrione, indicating that the mutant HPPD (F372A) can confer the tolerance to these two HPPD inhibitor herbicides upon the transgenic soybean plants; (2) as compared to the soybean T₁ plants into which the AsHPPDm-F372A-02 nucleotide sequence was introduced, there was no significant difference in the isoxaflutole and mesotrione herbicides tolerance of soybean T₁ plants into which the AsHPPDm-F372A-A110 nucleotide sequence was introduced, indicating that the mutation at position 372 of the HPPD amino acid sequence is sufficient to provide the plants with a high level of tolerance to isoxaflutole and mesotrione herbicides.

In conclusion, the present invention discloses for the first time that mutation at position 372 of hydroxyphenyl pyruvate dioxygenase polypeptides from different species can confer a higher tolerance to pyrazolinates, isoxazoles and triketones HPPD inhibitor herbicides upon plants, to such an extent that the plants can tolerate at least one-fold field concentration of topramezone, isoxaflutole or mesotrione. Therefore, the present invention has a broad application prospect in plants.

At last, it should be noted that all the above Examples are only used to illustrate the embodiments of the present invention rather than to limit the present invention. Although the present invention is described in detail with reference to the preferred Examples, those skilled in the art should understand that the embodiments of the present invention could be modified or substituted equivalently without departing from the spirit and scope of the technical solutions of the present invention.

## Claims

1. A mutant hydroxyphenylpyruvate dioxygenase polypeptide, which retains the activity of catalyzing the reaction of transforming 4-hydroxyphenylpyruvic acid into homogentisic acid or homogentisate and is less sensitive to an HPPD-inhibitor herbicide than the native unmutated HPPD, **characterized by** comprising the following mutation at a position corresponding to position 372 of the amino acid sequence as set forth in SEQ ID NO:1: F372A, F372G, F372V, F372P, F372S, F372T, F372C, F372M, F372Q, F372D, or F372 deletion;
Preferably, the mutant hydroxyphenylpyruvate dioxygenase polypeptide comprises the following mutation at a position corresponding to position 372 of the amino acid sequence as set forth in SEQ ID NO:1: F372A, F372G or F372V.
More preferably, the mutant hydroxyphenylpyruvate dioxygenase polypeptide comprises the following mutation at a position corresponding to position 372 of the amino acid sequence as set forth in SEQ ID NO:1: F372A.

2. The mutant hydroxyphenylpyruvate dioxygenase polypeptide according to claim 1, **characterized in that** the mutant hydroxyphenylpyruvate dioxygenase polypeptide is derived from HPPDs in plants or microorganisms.
Preferably, the plant comprises monocotyledonous plants and dicotyledonous plants; more preferably, the plant is oats, wheat, barley, millet, corn, sorghum, *Brachypodium distachyon,* rice, tobacco, sunflower, alfalfa, soybean, *cicer arietinum,* peanut, sugar beet, cucumber, cotton, oilseed rape, potato, tomato or *Arabidopsis thaliana;*
Preferably, the microorganism is Cyanophyta, Pseudomonas fluorescens, or bacteria from the genus Sphingobium or Burkholderia.

3. The mutant hydroxyphenylpyruvate dioxygenase polypeptide according to claim 2, **characterized in that**, when the native unmutated HPPD has an amino acid sequence as set forth in SEQ ID NO: 1, the polypeptide further comprises the following mutation at position 372 of the amino acid sequence as set forth in SEQ ID NO:1: F372L, F372I, F372W, F372N, F372E or F372K.

4. The mutant hydroxyphenylpyruvate dioxygenase polypeptide according to any of claims 1-3, **characterized in that** the mutant hydroxyphenylpyruvate dioxygenase polypeptide comprises a second mutation;
Preferably, the second mutation at a position corresponding to position 413 of the amino acid sequence as set forth in SEQ ID NO:1 comprises the following mutation: G413W, G413H, G413M, G413F or G413C; more preferably, the second mutation at a position corresponding to position 413 of the amino acid sequence as set forth in SEQ ID NO:1 is G413W mutation;
Optionally, when the native unmutated HPPD has an amino acid sequence as set forth in SEQ ID NO:1, the second mutation at position 110 of the amino acid sequence as set forth in SEQ ID NO:1 is deletion mutation.

5. A polynucleotide encoding the mutant hydroxyphenylpyruvate dioxygenase polypeptide according to any of claims 1-4.

6. An expression cassette or a recombinant vector, **characterized by** comprising the polynucleotide according to claim 5 under the regulation of effectively-linked regulatory sequences.

7. A method for expanding the scope of herbicides to which the plants are tolerant, comprising expressing the mutant hydroxyphenylpyruvate dioxygenase polypeptide according to any of claims 1-4 together with at least one herbicide-tolerant protein other than the mutant hydroxyphenylpyruvate dioxygenase polypeptide according to any of claims 1-4.

8. The method for expanding the scope of herbicides to which the plants are tolerant according to claim 7, **characterized in that** the herbicide-tolerant protein is 5-enolpyruvylshikimate-3-phosphate synthase (EPSPS), glyphosate oxidoreductase, glyphosate-N-acetyltransferase, glyphosate decarboxylase, glufosinate acetyltransferase, alpha-ketoglutarate-dependent dioxygenase, dicamba monooxygenase, acetolactate synthase, cytochrome-like protein and/or protoporphyrinogen oxidase.

9. A method for selecting transformed plant cells, **characterized by** comprising transforming a plurality of plant cells with the polynucleotide according to claim 5, and cultivating the cells under a concentration of the HPPD-inhibitor herbicide that allows the growth of the transformed cells expressing the polynucleotide, while killing the untransformed cells or inhibiting the growth of the untransformed cells;
Preferably, the plant comprises monocotyledonous plants and dicotyledonous plants; more preferably, the plant is oats, wheat, barley, millet, corn, sorghum, *Brachypodium distachyon,* rice, tobacco, sunflower, alfalfa, soybean, *Cicer arietinum,* peanuts, sugar beet, cucumber, cotton, oilseed rape, potato, tomato or *Arabidopsis thaliana;*
Preferably, the HPPD-inhibitor herbicide comprises an HPPD-inhibitor herbicide from the class of pyrazolinates, triketones and/or isoxazoles; more preferably, the HPPD-inhibitor herbicide of pyrazolinates is topramezone, the HPPD-inhibitor herbicide of triketones is mesotrione, and the HPPD-inhibitor herbicide of isoxazoles is isoxaflutole.

10. A method for controlling weeds, **characterized by** comprising applying an effective dose of the HPPD-inhibitor herbicide to a field planting with a target plant, wherein the target plant contains the polynucleotide according to claim 5;
Preferably, the target plant comprises monocotyledonous plants and dicotyledonous plants;
more preferably, the target plant is oats, wheat, barley, millet, corn, sorghum, *Brachypodium distachyon,* rice, tobacco, sunflower, alfalfa, soybean, *Cicer arietinum,* peanut, sugar beet, cucumber, cotton, oilseed rape, potato, tomato or *Arabidopsis thaliana;* further preferably, the target plant is glyphosate-tolerant plant, and the weeds are glyphosate-resistant weeds;
Preferably, the HPPD-inhibitor herbicide comprises an HPPD-inhibitor herbicide from the class of pyrazolinates, triketones and/or isoxazoles; more preferably, the HPPD-inhibitor herbicide of pyrazolinates is topramezone, the HPPD-inhibitor herbicide of triketones is mesotrione, and the HPPD-inhibitor herbicide of isoxazoles is isoxaflutole.

11. A method for protecting a plant from damages caused by an HPPD-inhibitor herbicide or for conferring tolerance to HPPD-inhibitor herbicide upon a plant, **characterized by** comprising introducing the polynucleotide according to claim 5 or the expression cassette or the recombinant vector according to claim 6 into the plant, resulting in an amount of the mutant hydroxyphenylpyruvate dioxygenase polypeptide that is sufficient to protect the plant into which the polynucleotide or the expression cassette or the recombinant vector has been introduced from damages caused by the HPPD-inhibitor herbicide.
Preferably, the plant comprises monocotyledonous plants and dicotyledonous plants; more preferably, the plant is oats, wheat, barley, millet, corn, sorghum, *Brachypodium distachyon,* rice, tobacco, sunflower, alfalfa, soybean, *Cicer arietinum,* peanut, sugar beet, cucumber, cotton, oilseed rape, potato, tomato or *Arabidopsis thaliana;*
Preferably, the HPPD-inhibitor herbicide comprises an HPPD-inhibitor herbicide from the class of pyrazolinates, triketones and/or isoxazoles; more preferably, the HPPD-inhibitor herbicide of pyrazolinates is topramezone, the HPPD-inhibitor herbicide of triketones is mesotrione, and the HPPD-inhibitor herbicide of isoxazoles is isoxaflutole.

12. A method for generating a plant which is tolerant to an HPPD-inhibitor herbicide, **characterized by** comprising introducing the polynucleotide according to claim 5 into the genome of the plant;
Preferably, the introduction method comprises genetic transformation, genome editing or gene mutation methods.
Preferably, the plant comprises monocotyledonous plants and dicotyledonous plants; more preferably, the plant is oats, wheat, barley, millet, corn, sorghum, *Brachypodium distachyon,* rice, tobacco, sunflower, alfalfa, soybean, *Cicer arietinum,* peanut, sugar beet, cucumber, cotton, oilseed rape, potato, tomato or *Arabidopsis thaliana;*
Preferably, the HPPD-inhibitor herbicide comprises an HPPD-inhibitor herbicide from the class of pyrazolinates, triketones and/or isoxazoles; more preferably, the HPPD-inhibitor herbicide of pyrazolinates is topramezone, the HPPD-inhibitor herbicide of triketones is mesotrione, and the HPPD-inhibitor herbicide of isoxazoles is isoxaflutole.

13. A method for cultivating a plant which is tolerant to an HPPD-inhibitor herbicide, **characterized by** comprising:
planting at least one plant propagule, wherein the plant propagule comprises in its genome the polynucleotide according to claim 5;
allowing the plant propagule to grow into a plant; and
applying an effective dose of the HPPD-inhibitor herbicide to a plant growing environment comprising at least the plant, and harvesting the plant having a reduced plant damage and/or increased plant yield compared to other plants without the polynucleotide according to claim 5;
Preferably, the plant comprises monocotyledonous plants and dicotyledonous plants; more preferably, the plant is oats, wheat, barley, millet, corn, sorghum, *Brachypodium distachyon,* rice, tobacco, sunflower, alfalfa, soybean, *Cicer arietinum,* peanut, sugar beet, cucumber, cotton, oilseed rape, potato, tomato or *Arabidopsis thaliana;*
Preferably, the HPPD-inhibitor herbicide comprises an HPPD-inhibitor herbicide from the class of pyrazolinates, triketones and/or isoxazoles; more preferably, the HPPD-inhibitor herbicide of pyrazolinates is topramezone, the HPPD-inhibitor herbicide of triketones is mesotrione, and the HPPD-inhibitor herbicide of isoxazoles is isoxaflutole.

14. A method for obtaining a processed agricultural product, **characterized by** comprising treating the harvested product obtained by the method according to claim 13 from the plant which is tolerant to the HPPD-inhibitor herbicide to obtain a processed agricultural product.

15. A planting system for controlling the growth of weeds, **characterized by** comprising an HPPD-inhibitor herbicide and a plant growing environment in which at least one target plant is present, wherein the target plant contains the polynucleotide according to claim 5;
Preferably, the target plant comprises monocotyledonous plants and dicotyledonous plants; more preferably, the target plant is oats, wheat, barley, millet, corn, sorghum, *Brachypodium distachyon,* rice, tobacco, sunflower, alfalfa, soybean, *Cicer arietinum,* peanut, sugar beet, cucumber, cotton, oilseed rape, potato, tomato or *Arabidopsis thaliana;* further preferably, the target plant is a glyphosate-tolerant plant, and the weeds are glyphosate-resistant weeds.
Preferably, the HPPD-inhibitor herbicide comprises an HPPD-inhibitor herbicide from the class of pyrazolinates, triketones and/or isoxazoles; more preferably, the HPPD-inhibitor herbicide of pyrazolinates is topramezone, the HPPD-inhibitor herbicide of triketones is mesotrione, and the HPPD-inhibitor herbicide of isoxazoles is isoxaflutole.

16. Use of the mutant hydroxyphenylpyruvate dioxygenase polypeptide according to any of claims 1-4 for conferring tolerance to an HPPD-inhibitor herbicide upon a plant;
Preferably, the plant comprises monocotyledonous plants and dicotyledonous plants; more preferably, the plant is oats, wheat, barley, millet, corn, sorghum, *Brachypodium distachyon,* rice, tobacco, sunflower, alfalfa, soybean, *Cicer arietinum,* peanut, sugar beet, cucumber, cotton, oilseed rape, potato, tomato or *Arabidopsis thaliana*;
Preferably, the HPPD-inhibitor herbicide comprises an HPPD-inhibitor herbicide from the class of pyrazolinates, triketones and/or isoxazoles; more preferably, the HPPD-inhibitor herbicide of pyrazolinates is topramezone, the HPPD-inhibitor herbicide of triketones is mesotrione, and the HPPD-inhibitor herbicide of isoxazoles is isoxaflutole.
